(19) Europäisches Patentamt
European Patent Office
Office européen des brevets

(11) EP 3 572 468 B1

(12) # EUROPEAN PATENT SPECIFICATION

(45) Date of publication and mention of the grant of the patent:
**14.02.2024 Bulletin 2024/07**

(21) Application number: **18174086.1**

(22) Date of filing: **24.05.2018**

(51) International Patent Classification (IPC):
***C09B 23/04*** (2006.01) ***C09B 23/06*** (2006.01)
***C09B 23/08*** (2006.01) ***G01N 33/58*** (2006.01)
***C12Q 1/689*** (2018.01)

(52) Cooperative Patent Classification (CPC):
(C-Sets available)
**C09B 23/06; C09B 23/04; C09B 23/083; G01N 33/569; G01N 33/582;** C12Q 1/68; C12Q 1/689 (Cont.)

(54) **CYANINE DYES AND THEIR USAGE FOR IN VIVO STAINING OF MICROORGANISMS**

CYANINFARBSTOFFE UND DEREN VERWENDUNG ZUR IN VIVO-FÄRBUNG VON MIKROORGANISMEN

COLORANTS DE CYANINE ET LEUR UTILISATION POUR LA COLORATION IN VIVO DE MICRO-ORGANISMES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**27.11.2019 Bulletin 2019/48**

(73) Proprietor: **PHILIPPS-UNIVERSITÄT MARBURG**
**35037 Marburg (DE)**

(72) Inventors:
- **Vázquez Vázquez, Olalla**
  **35037 Marburg (ES)**
- **Schulte, Leon N.**
  **35037 Marburg (DE)**
- **Plocher, Benedikt**
  **61118 Bad Vilbel (DE)**
- **Schmeck, Bernd T.**
  **35037 Marburg (DE)**

(74) Representative: **Stumpf, Peter**
**c/o TransMIT GmbH**
**Kerkrader Strasse 3**
**35394 Gießen (DE)**

(56) References cited:
**WO-A1-2013/189043** **US-A1- 2010 151 509**
**US-A1- 2011 262 904** **US-A1- 2015 185 182**
**US-A1- 2016 340 717**

- **JIEQIONG QIU ET AL: "Combination probes with intercalating anchors and proximal fluorophores for DNA and RNA detection", NUCLEIC ACIDS RESEARCH, vol. 44, no. 17, e138, 1 July 2016 (2016-07-01), pages 1-12, XP55523033, ISSN: 0305-1048, DOI: 10.1093/nar/gkw579**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
C12Q 1/68, C12Q 2563/173

## Description

The invention relates to the production of cyanine dyes and their use for in vivo-staining of microorganisms, especially pathogens.

## Description

### Field of the invention

The discovery, characterization and implementation of the green fluorescent protein (GFP) has revolutionized modern molecular cell biology. GFP derivatives are widely used as fluorescent reporters to study biological processes at the cellular and molecular level. In the case of bacterial infection, it can be used for fluorescent labeling of bacteria to study host-pathogen interactions. These approaches currently rely on genetic manipulation. Unfortunately, several important organisms have remained difficult to modify for GFP expression. This is particularly true for the growing number of multi-drug resistant bacteria, posing an emerging threat to patients in clinics worldwide. Specifically, these strains may acquire resistances to the antibiotics used to select transformants carrying the GFP transgene. Thus, the investigation of host cell interplay with drug-resistant bacteria by means of fluorescent labeling has lagged behind.

### Background of the technology

Excessive use of antibiotics has propelled the spread of multidrug-resistant bacteria at alarming rates. As a consequence, incremental failure of anti-microbial treatment in patients leads to increased fatality rates due to systematic spread of bacteria and organ failure. Among a number of major disease causing agents Klebsiella pneumoniae has received highest research priority by the world health organization due to rapid acquisition of novel resistances. New research tools compatible with resistant bacteria such as fluorescent labeling approaches are urgently required. Detailed investigation of the disease-causing strategies of drugresistant bacteria interacting with their hosts will assist rational drug design and may eventually reduce fatality rates of infected patients.

Document US 2016/340717 A1 reveals a method of staining which requires an oligonucleotide, a fluorophore operably linked to the oligonucleotide, and a fluorescence quencher, also operably linked to the oligonucleotide. Upon specific cleavage of the oligonucleotide, fluorescence is exerted because of separation of the quencher from the fluorophore; the fluorophore may be a cyanine dye.

Jieqiong Qui et al. describe (cf. "Combination probes with intercalating anchors and proximal fluorophores for DNA and RNA detection", Nucleic Acid Research, vol. 44, no. 17, 1 July 2016, pages 1-12) the usage of an anchor-dye and a reporter dye, i. e. two dyes, intercalating into DNA/RNA for achieving the desired staining. No viability of stained cells is reported.

US 2011/262904 A1 reveals dyes having a good solubility in water and a good live cell permeability, but nowhere reveals any hint that staining with these dyes is not negatively interfering with the viability of the stained cells.

WO 2013/189043 A1 reveals that living cells are stained, but provides no hint that the staining is not negatively interfering with the viability of the stained cells. After being stained, the cells are directly investigated via confokal laser microscopy and viability of the stained cells is no subject within WO 2013/189043 A1 at all.

US 2015/185182 A1 reveals staining of living cells, but also provides no hint at all that the staining is not negatively interfering with the viability of the stained cells. Investigation of the stained cells is performed at latest about 30 minutes after staining (microscopic inspection), but no measurement, not even a faint hint is given that the cells are still alive at that time.

US 2010/151509 A1 reveals staining and counting of leucocytes. Because leucocytes are completely different from microbial cells and cannot grow/multiply, the aspect of viability is of no relevance within US 2010/151509 A1.

### Content of the invention

An objective of the present invention is to provide substances that can be used for in vivo-staining of microorganisms without reducing the viability of the stained microbes. The term "microorganism" (and all of its grammatical forms) as used with regard to the present invention comprehends not only all types of bacteria, fungi etc., but also pathogenic structures of all types. The terms "microorganism", "pathogen", "bacteria" etc. are therefore synonymously used throughout this whole revelation of the current invention.

In order to achieve the above object, the present invention provides new types of cyanine dyes of formula (I),

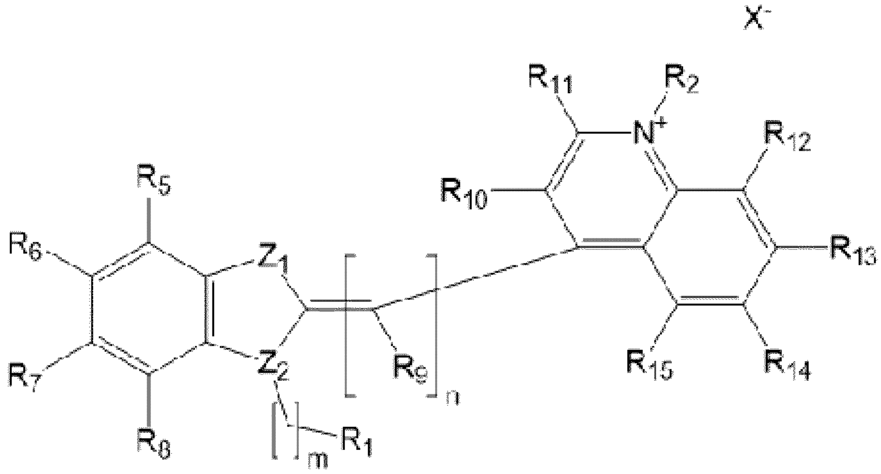

(I)

,

allowing in vivo staining of pathogens without negatively interfering with the viability of the pathogens. The above objection is achieved through the independent claims 1, 6, 7 and 8. The dependent claims reveal additional advantageous embodiments of the invention. The most preferred examples of the invention comprise 5/6-membered rings, especially heterocyclic 5/6-membered rings, within $R_1$.

Thus, herein the synthesis and applicability of novel near-infrared (NIR) DNA cyanine dyes for straightforward in vivo bacterial labeling is revealed, allowing subsequent infection studies. **6-TramTO-3** is identified as a vital dye, conferring bacteria fluorescent staining without compromising microbial growth. The inventive approach is exemplified with Escherichia coli (E. coli) and antibiotic-resistant and - sensitive Klebsiella pneumoniae (K. pneumoniae) strains. Importantly, the results reveal differential strategies of the examined strains interacting with primary human host cells.

In the past decades, chemical biology has made progress towards the development of fluorescent small molecules as alternatives to genetically encoded fluorescent reporters. In the context of inflammation fluorophores have recently been shown to selectively track or report the activity of phagocytic immune cells such as macrophages. These cells play a major role in the clearance of bacteria in a process referred to as phagocytosis. To study bacteria-phagocyte interactions fluorescein derivatives have been used in bacteria uptake assays, though they tend to suffer from quenching in low-pH environments. Nanospheres may fluorescently label living bacteria; however, with unclear consequences for bacterial viability. BODIPY derivatives were used to stain obligate intracellular bacteria, however with high background due to hostcell co-staining and continuous fluorescence emission independent of target structure binding. Light-up DNA binders have been used instead, but at the expense of compromising bacteria viability. For instance, in vivo labeling of bacteria for macrophage uptake assays has been performed using the nucleic acid stain SyTO-9™. However, reduced colony formation of the stained bacteria was observed, indicative of toxicity, which compromises the use of SyTO-9™ as a vital dye for pathogen-host investigations. Taking SyTO-9™ as a starting point, and due to the excellent spectroscopic properties of such cyanine compounds, we exemplary aimed at the synthesis of a versatile azidefunctionalized unsymmetrical NIR cyanine dye (**6-AzTO-3**) based on the classical thiazol orange (TO) and related compounds. To increase both solubility and affinity, exemplary two amino derivatives were also prepared: **6-NH2TO-3** and **6-TramTO-3** (cf. Fig. 1).

Initially, it was evaluated whether common fluorophores for biological research such as ethidium bromide (EtBr), carboxyfluorescein succinimidyl ester (CFSE) or SyTO-9™ may confer fluorescent labeling of live E. coli without affecting viability. To this end, candidate compounds were supplemented directly into bacterial culture medium and E. coli growth was monitored over time (Fig. 2 and Fig. 28A). At mid-exponential growth phase, fluorescent labeling was determined by fluorescence-activated cell sorting (FACS) analysis. Importantly, none of the standard fluorophores (EtBr and CFSE) was found eligible for bacterial vital staining at 2.5 μM: despite being compatible with bacterial growth, CFSE hardly labeled bacteria while EtBr did not confer a pronounced fluorescence-shift in the red/green/yellow emission bands commonly used in FACS (Fig. 28B). As a possible explanation, EtBr displays intrinsic membrane- impermeability in living cells at low concentrations. Interestingly, SyTO-9™, which conferred efficient green-fluorescent labeling, delayed bacterial growth substantially. Moreover, cross-emission into the yellow and red spectrum was observed, rendering combination of SyTO-9™ with other fluorophores difficult. Surprisingly, all inventive cyanine dyes conferred a highly specific red-fluorescence shift of live bacteria in the 652-671 nm red2 channel, as determined by FACS (Fig. 2 and Fig. 28B) and microscopy (Fig. 27A, Fig. 27B and Fig. 27C). Especially, the compound **6-TramTO-3** is fully compatible with normal bacterial growth in liquid medium (Fig. 2) and on solid medium (Fig. 29A, Fig. 29B, Fig. 29C), proving that there are no adverse effects on viability. Consequently, we exemplarily selected **6-TramTO-3** as the most promising candidate for further spectroscopic characterization, DNA-binding affinity, sequence selectivity and activity studies with antibiotic-sensitive and -resistant bacteria.

In aqueous solution, **6-TramTO-3** shows long wavelength maxima of excitation and emission (633 nm and 645 nm, respectively; Fig. 13, part b), and low intrinsic fluorescence ($\Phi F$ = 0.005). Remarkably, upon the addition of double-stranded calf thymus DNA ($dsDNA_{CT}$) the fluorescence emission intensity increased dramatically (>25-fold; $\Phi F$ = 0.27). Interestingly, this outcompetes the fluorescence increase observed for EtBr under the same conditions (Figure 3A). Furthermore, **6-TramTO-3** emission maximum was found to be shifted to the near-infrared part of the spectrum, which enabled **6-TramTO-3** detection in the FACS red2 fluorescence channel. The results also show an implementation of **6-TramTO-3** as a replacement for EtBr to post-stain DNA in gel electrophoresis (Figure 3A and Figure 25).

In order to obtain more information on the affinity and selectivity of **6-TramTO-3** DNA-binding, we performed fluorescence titrations with $dsDNA_{CT}$ and hairpin oligonucleotides, which contain the sequence either ATTA ($dsDNA_{hAT}$) or GGCCC ($dsDNA_{hGC}$). In all cases, addition of DNA resulted in a progressive increase in fluorescence emission (Figure 3B). The rise in the emission at 647 nm could be fitted to a simplified 1:1 binding model resulting in the corresponding apparent dissociation constants. Without being confined to a certain theory this simplified 1:1 binding model may be further described as follows: The calculated affinity of **6-TramTO-3** to $dsDNA_{CT}$ (KD = 19.5 $\pm$ 2.6 $\mu$M) is reminiscent of previously obtained results for DNA intercalators like EtBr (KD = 15 $\mu$M), methylene blue (KD = 46 $\mu$M) and acridine orange (KD = 36 $\mu$M) with the same DNA. Furthermore, thermal melting experiments showed significant $dsDNA_{CT}$ stabilization upon **6-TramTO-3** binding ($\Delta$Tm = 5 °C). **6-TramTO-3** displays a higher affinity for both hairpin oligonucleotides than for $dsDNA_{CT}$. Concerning sequence selectivity, the analysis of the apparent dissociation constants of **6-TramTO-3** for $dsDNAh_{AT}$ and $dsDNAh_{GC}$ (KD = 0.114 $\pm$ 0.03 $\mu$M, KD = 0.104 $\pm$ 0.054 $\mu$M, respectively) indicates non-sequence-specific binding, further supporting the intercalation binding mode. The slightly lower fluorescence intensity observed with $dsDNAh_{GC}$ might be explained by photo-induced electron transfer (PET) in GCrich sequences. Next, to better understand the binding mode of **6-TramTO-3** we recorded absorbance titrations. Addition of $dsDNA_{CT}$ (< 2.5 eq of DNA) caused a dramatic change in the absorption spectra: a 102 nm blue shift of the absorption maximum of **6-TramTO-3** was observed, which was also temperature-dependent (Figure 23). Consecutive increment of DNA equivalents promoted a new response: a red-shifted transition with a maximum intensity at 631 nm. This points to a half intercalating binding mode. Circular dichroism (CD) experiments corroborated the suggested binding modes. Taken together, our findings introduce **6-TramTO-3** as an example of a novel type of DNA intercalating dye compatible with bacterial cell growth, conferring fluorescent labeling at an emission wavelength suitable for routine FACS analysis.

Thus the interaction mode between the inventive cyanine dye and the DNA is quite complex and several binding modes are observed. The observed 102 nm blue shift of the absorption maximum for low DNA concentrations (<2.5 eq. of calf thymus DNA) at the absorbance titrations with calf thymus DNA, which is also temperature dependent is in agreement with an exterior binding to the DNA, also observed for other cyanine dyes according to the state of the art. Increasing the amount of DNA leads to a red shifted signal, showing that a half intercalation mode of binding is now taking place. The fluorescence titration experiments with hairpin oligonucleotides, which contain either the sequence ATTA or GGCCC and the analysis of the apparent dissociation constant show a non-sequence-specific binding mode (KD for ATTA 0.114 $\pm$ 0.03 $\mu$M, KD for GGCCC 0.104 $\pm$ 0.054 $\mu$M), which further supports the intercalation binding mode.

It is obvious to the person skilled in the art that the inventive method of DNA staining (the usage of the new cyanine dyes) can be universally applied, i.e. not only to bacteria DNA, but also to any other type of DNA, may it be viral, cancerogenic or even not pathogenic at all. The high stability of the DNA-dye-complex resulting from the binding of the dye to the DNA minimizes bleaching out and thus allows effective staining of the desired living organism and then let it interact with non-stained organisms/cells observing the process of interaction.

The inventive method can not only be applied for scientific studies but also for extracorporal medical investigations, similar to blood diagnostics: A mixture of bacteria from the patient is isolated, singularized and selected on different media to split up the mixture into individual, well determined species and these are then used for the inventive extracorporal analysis. The inventive dye can then report about how the identified species interact with host cells, which provides information about their pathogenic potential (cf. Fig. 30).

Having established an assay for in vivo fluorescence labeling of E. coli we exemplary tested the applicability of **6-TramTO-3** for staining of clinically relevant bacteria hard to modify genetically due to resistance to selection antibiotics. Lack of genetic tools has hampered studies on the interaction of such strains with host cells using standard tools such as fluorescence microscopy or FACS. To test whether the dyes may overcome this current limitation, we focused on the major human pathogen Klebsiella pneumoniae. To this end, antibiotic-resistant and -sensitive K. pneumoniae were cultured and compared with E. coli. Resistance to standard antibiotics was confirmed by spotting bacterial dilutions onto solid medium (Figure 5A). While multiresistant K. pneumoniae were insensitive to kanamycin, ampicillin and chloramphenicol both E. coli and the other K. pneumoniae strain were sensitive to all three antibiotics. All bacterial strains were successfully fluorescently labeled (Figure 5B, Figure 29A) and grew normally in presence of **6-TramTO-3** (Figure 29B and Fig. 29C). Thus, **6-TramTO-3** and derivatives thereof do not disturb bacterial growth and overcome the current lack of a universal fluorescent-labeling approach for multi-drug resistant pathogenic bacteria.

After demonstrating successful fluorescent labeling of multi-resistant microbes, we sought to evaluate the use of **6-TramTO-3** and its derivatives in studies addressing the interaction of these bacteria with host cells. **6-TramTO-3**

is used exemplarily within these studies. Bacterial infections typically occur through ingestion or inhalation and subsequent colonization of the mucosal surfaces of the lung or gastro-intestinal tract. Resident immune cells such as macrophages may phagocytose and neutralize bacteria or signal to the environment to prevent them from causing disease. In a proof-of-principle experiment we exemplarily examined the interaction of E. coli and both Klebsiella strains with primary human macrophages through bacterial labeling with **6-TramTO-3.** Non-pathogenic E. coli have previously been shown to be readily phagocytosed by macrophages. Consequently, the E. coli strain used here was readily taken up in co-culture experiments, indicated by a red-shift of ~ 26 % of the macrophages (Figure 6A and B). Separation of this population from unstained macrophages by FACS and subsequent quantitative real-time PCR (qRT-PCR) (Figure 6D) confirmed that the red-shifted population represented macrophages that had taken up bacteria. Interestingly, in Klebsiella exposure experiments ~ 4 % of the macrophages took up the antibiotic-resistant strain while the antibiotic sensitive strain was merely taken up (~ 0.2 % Figure 6A and B), which was again confirmed by FACS and qRT-PCR. Costaining with the yellow-fluorescent cell-death stain propidium iodide furthermore indicated that macrophages with intracellular E. coli or sensitive and multiresistant K. pneumoniae preferentially enter cell death (Figure 6C). This is in line with the reported role of macrophages in digestion and subsequent self-degradation as part of the bacterial clearance.

Together these observations show that the examined Klebsiella strains adopt different virulence strategies: while the multidrug-resistant strain evades from clearance by anti-microbial compounds the antibiotic sensitive strain has learned to cope with clearance by immune cells such as macrophages. This is the first report providing a direct comparison of the interaction of antibiotic-resistant and sensitive pathogens with human immune cells by means of fluorescent labeling.

Thus herein the synthesis and efficacy study of novel NIR cyanine dyes for in vivo bacterial tracking is revealed by exemplary use of **6-TramTO-3. 6-TramTO-3** is an exemplary sample of a new type of fluorescent light-up probes with up to 27-fold signal increase in presence of dsDNA and no apparent effects on bacteria viability. We demonstrated efficient labeling of multi-drug resistant bacteria, where fluorescent tagging by genetic manipulation is not feasible. Thereby, our exemplary investigations with pathogenic microbes revealed divergent interaction modes of multi-resistant and antibioticsensitive Klebsiella bacteria with human macrophages. This invention opens the doors for further advanced applications such as Dual RNA-Seq or Single Cell RNA-Seq to understand the disease-strategies of antibiotic-resistant bacteria, an emerging world-wide threat.

**6-TramTO-3** and its derivatives constitute off-the-shelf-reagents for real-time analysis of bacterial infection, including strains where the use of genetically-encoded reporters is not feasible. The invention may therefore be summarized as follows.

The invention comprises a cyanine dye binding to DNA, which is selected from the group of cyanine dyes being characterized by the general formula (**I**):

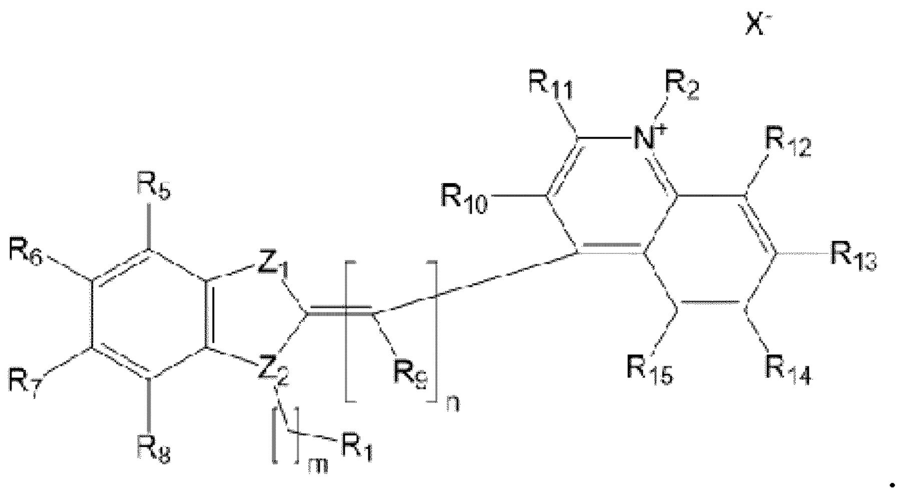

**(I)**

In formula (I) the substituents, symbols and indexes represent the following values: $Z_1$ is S and $Z_2$ is N. $X^-$ is selected from the list comprising halogenate, sulfate, triflate, trifluoro acetate, difluoro acetate or fluoro acetate. Index n is 3. Index m is 1.

$R_1$ is selected from the list comprising 5-azidopentyl, 5-(4-(aminomethyl)-1H-1,2,3-triazol-1-yl)pentyl or 5-ammoniopentyl, $R_2$ is ethyl, and $R_5$ to $R_{15}$ are each a hydrogen atom.

The invention further comprises a cyanine dye as described above, which is binding to oligomeric DNA or primer-DNA or DNA-aptameres.

The invention further comprises a cyanine dye as described above in its protonated or deprotonated form; the

counterion, resp. counterions, of the protonated or deprotonated form is, resp. are, independently selected from the list comprising halogenate, sulfate, triflate, trifluoro acetate, difluoro acetate or fluoro acetate.

The invention further comprises a method for in vivo staining of organisms using a cyanine dye as described above. The method comprises the following steps: a) isolation of the organisms that are to be stained, whereat this isolation-step optionally includes one or more steps for concentrating and/or breeding of the organisms; b) staining the organisms with the cyanine dye by adding a solution of the cyanine dye to the organisms.

The invention further comprises a kit for carrying out the method as described above, whereat the kit comprises the cyanine dye and optionally supportive substances.

**List of abbreviations/reference signs**

| | |
|---|---|
| A | absorbance |
| CD | circular dicroismn |
| CFSE | carboxyfluorescein N-succinimidyl ester |
| COSY | correlation spectroscopy |
| $dsDNA_{CT}$ | double-stranded calf thymus DNA |
| $dsDNAh_{AT}$ | double-stranded DNA hairpin with the consensus sequence ATTA |
| $dsDNA_{hGC}$ | double-stranded DNA hairpin with the consensus sequence GGCCC |
| d | doublett |
| DMSO | dimethylsulfoxide |
| ECL | enhanced chemiluminescence |
| EI | electron ionisation, emission intensity |
| Eq. | equivalent |
| ESI | electrospray ionisation |
| EtBr | Ethidium bromide |
| G | gravitational acceleration |
| 9 | gram |
| HPLC | high performance liquid chromatography |
| HSQC | heteronuclear single quantum correlation spectroscopy |
| Hz | hertz |
| KD | apparent dissociation constant |
| m | multiplett |
| m | mass |
| MeCN | acetonitrile |
| MS | mass spectrometry |
| NIR | Near infrared |
| NMR | nuclear magnetic resonance |
| PBS | phosphate buffered saline |
| PMT | photomultiplier tube |
| ppm | part per million |
| q | quartett |
| r.t. | room temperature |
| s | singulett |
| t | triplett |
| TCEP | tris(2-carboxyethyl)phosphine |
| TAE | tris-acetate-EDTA |
| TFA | trifluoroacetic acid |
| $T_m$ | melting temperature |
| Tris | tris(hydroxymethyl)aminomethane |
| UV | ultraviolet |
| Vis | visible |
| z | charge |
| $\varepsilon$ | molar extinction coefficient |
| $\eta$ | refractive index |
| $\lambda$ | wavelength |
| $\Phi F$ | quantum yield |

## Detailed embodiments of the invention

### Materials and Experimental Procedures

All commercially purchased reagents were used without further purification as delivered from the corresponding company. The respective reagents were purchased from the following companies: lepidine, N,N'-diphenylformamidine, 2-methylbenzothiazole, ethyliodide, copper powder, triphenylphosphine, DMSO for molecular biology, ethidium bromide, potassium chloride, 2,6-lutidine and DNA hairpins from Sigma Aldrich (USA), tris(2-carboxyethyl)phosphine (TCEP), acetic anhydride, copper(II) sulfate, Tris(hydroxymethyl)aminomethane (Tris) and sodium chloride from Carl Roth (Germany), sodium azide, and propargylamine from Acros Organics (Belgium), triethylamine and acetonitrile (MeCN) from VWR (USA), dibromohexane from Alfa Aesar (USA), copper(I) bromide from Santa Cruz Biotechnology (USA), trifluoroacetic acid (TFA) from abcr (Germany), Atto 655 NHS-ester from Atto-tec (Germany), SYTO-9™ nucleic acid stain from Life Technologies (USA), carboxyfluorescein N-succinimidyl ester (CFSE) from Thermo Fisher (Germany), CSL 1kb DNA ladder from Cleaver Scientific (UK) and calf thymus DNA (dsDNACT) and Alexa Fluor® 647 $C_2$-maleimide was purchased from Invitrogen (USA). Water was purified with a milli-Q Ultra Pure Water Purification System (TKA, Germany).

### High-Performance Liquid Chromatography (HPLC)

For preparative purposes, a PLC 2020 Personal Purification System (Gilson) with a preparative Nucleodur C18 HTec-column (5 $\mu$m, 250 $\times$ 16 mm; Macherey & Nagel) using an isocratic regime during the first five minutes for column equilibration, followed by a linear gradient 5% to 75% B in 30 min, for both compounds: **6-TramTO-3 (2)** and **6-NH2TO-3** (**3**), with a flow rate of 10 mL/min at 25°C was used. The detection was carried out by measuring the absorption at wavelengths: 220 nm and 300 nm. Milli-Q water (A) and MeCN (B) were employed as eluents with an addition of 0.1% of TFA for both solvents.

For analytical purposes, an Agilent 1260 Infinity II HPLC-System (Agilent Technologies) with an EC 125/2 Nucleodur 100-C18 ec column (Macherey & Nagel) using an isocratic regime during the first five minutes for column equilibration, followed by a linear gradient 5% to 75% B in 30 min with a flow rate of 0.2 mL/min at 55°C was used. The detection was carried out by measuring the absorbance at a wavelength of 660 nm for the NIR cyanine dye derivatives **1**, **2** and **3**. In all cases, milli-Q water (A) and MeCN (B) were employed as eluents with an addition of 0.05% of TFA for A and 0.03% for B.

Figs. 8A-C show the HPLC chromatograms and structures of the synthesized NIR cyanine dyes used in the bacteria cell-based experiments.

### Nuclear Magnetic Resonance Spectroscopy (NMR)

All NMR spectra were automatically measured at 300 K either in a Bruker AV III HD 300 MHz at a frequency of 300 MHz ($^1$H) or 75 MHz ($^{13}$C) or in a Bruker AV III HD 500 MHz at a frequency of 500 MHz ($^1$H) or 125 MHz ($^{13}$C). All chemical shifts ($\delta$) are relative to tetramethylsilane (TMS), i.e. $\delta$(TMS) = 0 ppm. As internal standards, deuterated chloroform ($CDCl_3$), dimethyl sulfoxide (DMSO) with TMS were used. Solvent shifts (ppm): $\delta$($CHCl_3$) = 7.26 ppm ($^1$H), 5($CHCl_3$) = 77.16 ppm ($^{13}$C), $\delta$(DMSO) = 2.50 ppm ($^1$H), $\delta$(DMSO) =39.52 ppm ($^{13}$C). The assignment of each signal was based on two-dimensional nuclear magnetic resonance spectroscopy (2D NMR) ,i.e. heteronuclear single quantum coherence (HSQC), heteronuclear multiple bond correlation spectroscopy (HMBC) and correlation spectroscopy (COSY).

### Mass Spectrometry

Electrospray ionization mass spectrometry (ESI-MS) was performed on a Finnigan LTQ-FT (Thermo Fischer Scientific). EI-MS was performed on an AccuTOF GCv (JEOL).

### Fluorescence Measurements

Fluorescence measurements and tritations were performed on a Jasco FP-6500 spectrofluorometer equipped with a Thermo Haake WKL 26 water recirculator at 20°C. Unless otherwise noted, a 1400 $\mu$L fluorescence cuvette of HellmaAnalytics (104F-QS) was used and the measurements were performed with the following settings: increment: 1.0 nm; integration time: 0.2 s; speed: 500 nm/min; sensitivity: medium; excitation slit width: 3 nm; emission slit width: 3 nm; excitation wavelength 610 nm. The emission spectra were recorded from 625 to 750 nm at 20°C. From all spectra, the corresponding background signal was substracted. The used hairpin double-stranded DNAs $dsDNA_{hAT}$ and $dsDNAh_{GC}$ had the sequences depicted in Table. 1.

Table 1: Binding sites and sequences of DNA hairpins, which were used for fluorescence titrations experiments.

| DNA | binding site | sequence (5' - 3') |
|---|---|---|
| $dsDNA_{hAT}$ | ATTA | GGCGATTACAGCTTTTTGCTGTAATCGCC |
| $dsDNA_{hGC}$ | GGCCC | GGCAGGCCCAGCTTTTTGCTGGGCCTGCC |

**Fluorescence Quantum Yield**

Fluorescence intensity values for the quantum yields were determined on an ISS PC1 spectrofluorometer with an ISS 11650 lamp connected to an ISS Simpson model 109 and an ISS 80011 photomulitplier connected to a MGW Lauda K2R D and an Amherst Scientific Corp. 7600F for cooling. A VWR Refrigerated Circ 1160S was connected for the cooling of the cuvette chamber. It was measured in a 1400 µL fluorescence cuvette of HellmaAnalytics (104F-QS) at 25°C using the following settings: increment: 2.0 nm; integration time: 1.0 s; excitation slit width: 0.25 mm; emission slit width: 0.5 mm; excitation wavelength 590 nm. The emission spectra were recorded from 610 to 750 nm. From all spectra the corresponding background signal was substracted. Absorbance values were measured on a Jasco V-650 UV-Vis Spectrophotometer equipped with a Jasco PAC-743 temperature controlled cell holder and a Julabo F250 cooling system. UV-Vis titration measurements were performed in a 1400 µL quarz cuvette with a pathlength of 1 cm at 25°C using the following settings: increment: 1.0 nm; bandwidth: 1.0 nm; scanning speed: 1000 nm/min; scanning mode: continuous. From all values, the corresponding background signal was subtracted. The values for the reference compounds were measured in duplicates, the ones for **6-TramTO-3** with and without dsDNACT in triplicates. The quantum yields of **6-TramTO-3** with (30 eq.) and without dsDNACT were determined in 10 mM Tris pH 7.6 and 50 mM KCl. All measurement were following the procedure of Horiba and using the equation **E1:**

$$\Phi_X = \Phi_{ST} \left( \frac{Grad_X}{Grad_{ST}} \right) \left( \frac{(\eta_X)^2}{(\eta_{ST})^2} \right) \qquad (\mathbf{E1})$$

where Φ = fluorescence quantum yield, η = refractive index of the used solvent, Grad = gradient from the plot of integrated fluorescence intensity vs. absorbance and the subscripts ST and X denote standard and tested sample.

As reference compounds, Atto 655 in PBS pH 7.4 (Φ = 0.30) and Alexa Fluor® 647 in 50 mM potassium phosphate pH 7.2 and 150 mM NaCl (Φ = 0.33) were used. All solvents were degased before use. Table 3 (see below) summarizes the determined slopes and their calculated quantum yields.

**UV-VIS Measurements**

UV-Vis titration measurements were performed on a Jasco V-650 UV-Vis spectrophotometer equipped with a Jasco PAC-743 temperature controlled cell holder and a Julabo F250 cooling system to maintain the temperature constant at 20°C during the measurements. UV-Vis titration measurements were performed in a 500 µL quarz cuvette with a pathlength of 1 cm at 20°C. The absorbance spectra were recorded from 400 nm to 800 nm.

The concentration determination and measurement of full UV-Vis spectra were performed on a Tecan 20M at room temperature. The measurements were performed in a 1400 µL quarz cuvette with a pathlength of 1 cm in a volume of 1 mL.

**CD Measurements**

CD titration measurements were performed on a Jasco J-810 Spectropolarimeter equipped with a Jasco CDF-426S peliter controller and a Thermo Haake WKL 26 water recirculator at 20°C using a 350 µL CD cuvette of HellmaAnalytics (110-QS) at a concentration of 36.9 µM of **6-TramTO-3** in 10 mM Tris pH 7.6 and 50 mM KCl. The settings were: range from 750-450 nm, speed of 100 nm/min, response of 4 s, band width of 10 nm and a data pitch of 1 nm. The measurement was performed 2 min after the addition of $dsDNA_{CT}$. Each measurement consisted of a mean of 5 measurements. Three independent measurements were performed.

### Thermal Melting Analysis

Thermal melting analysis was performed on a Jasco J-810 spectropolarimeter equipped with a Jasco CDF-426S peliter controller and a Thermo Haake WKL 26 water recirculator. It was measured at a concentration of 45 μM of $dsDNA_{CT}$ with and without 1.8 μM of **6-TramTO-3** in 10 mM Tris pH 7.5. The measurement was performed at 260 nm, with a response of 2 s, a band width of 10 nm, from 40-95°C, a slope of 2°C/min. Three independent measurements were recorded and the intensity normalized.

### Gel Electrophoresis

Gel electrophoresis was performed with a Mini-Sub® Cell GT Cell (Biorad, USA) system with a power supply: MP-300V (Major Science, USA), for 75 min at 25°C with constant 80 V. For the experiments, 6 μL of 1000 ng, 500 ng and 100 ng of $dsDNA_{bc}$ and $dsDNA_{bl}$ in TAE, containing 10% glycerol were loaded. As DNA ladder, CSL 1kb ladder was used. The gel was resolved in 1% agarose gel using TAE as a running buffer and analyzed by post-staining with either 1 μM of **6-TramTO-3,** or 1 μM) ethidium bromide in TAE buffer. The gel was visualized using a Typhoon TRIO Variable Mode Imager (Amersham Biosciences, UK). To visualize the gel stained by **6-TramTO-3,** we used: the emission filter 670 BP 30 Cy5, the red laser (633 nm), PMT 400, pixel size 25 microns and normal sensitivity. For the case of the gel stained ethidium bromide, we used: no filter, an ECL + Excitation, PMT 400, pixel size 25 microns and a normal sensitivity.

### Bacteria and Host Cell Culture

Bacteria Culture and Staining:

Escherichia coli (E. coli) NEB5α (New England Biolabs) and antibiotic-resistant and -sensitive Klebsiella pneumoniae (K. pneumoniae) were cultured in Luria-Bertani (LB) solid medium (LB-Agar) overnight at 37°C. The source of the K. pneumoniae strands is of no relevance to the invention. The invention may be applied to all types of microorganisms (bacteria, viri, fungi etc.) or other cells, either contained within a sample or isolated from a sample. For staining, growth curve analysis and replating experiments bacteria were suspended in LB liquid medium at an $OD_{600\ nm}$ of 1.0 and 800 μL were transferred into a culture tube containing 7.2 mL of LB liquid medium supplemented with DMSO or candidate dyes (2.5 μM) final concentration). Density of bacterial cultures was measured using an Ultrospec 10 Cell Density Meter with 600 nm LED source (Amersham Biosciences). For bacterial re-plating experiments bacteria were sampled at $OD_{600\ nm}$ = 2.0 and 5 μL of indicated dilutions in LB-medium were spotted onto LB-agar plates (Fig. 5) or 100 μL were plated onto LB-agar plates followed by incubation at 37°C until colonies became visible.

Host Cell Culture and Exposure to Bacteria:

For primary human macrophage cultures buffy coats from blood donations were loaded onto Lymphoprep™ gradient medium (Fresenius Kabi) followed by centrifugation. The peripheral blood mononuclear cell band was isolated from the gradient and monocytes were enriched using CD14 MACS beads (Miltenyi) according to the manufacturer's instructions. To differentiate monocytes into macrophages cells were cultured in 6-well plates (800.000 cells/mL) in 2 mL X-Vivo 15 medium (Lonza) supplemented with 5 % fetal bovine serum (Biochrom) and 15 ng/mL recombinant human GM-CSF (Preprotech). On day 7 macrophages were considered terminally differentiated and treated with stained bacteria. To this end bacteria stained as described above were sampled at an $OD_{600\ nm}$ = 2.0 and washed five times with phosphate buffered saline (PBS). After the last wash bacteria were brought to an $OD_{600\ nm}$ of 2.0 and 16 μL, equivalent to 16.000.000 bacteria were added to each well to achieve a multiplicity of infection of 20. Culture plates were then centrifuged for 10 min at 250 G and 37°C to establish physical contact between macrophages and bacteria. Subsequently, culture plates were transferred back into a 37°C incubator with $CO_2$ atmosphere for the remainder of the stimulation period.

### Fluorescence Microscopy

Cell Preparation:

Macrophage cells were grown and stimulated with bacteria as described above but grown in 35 mm ibidi μ-Dish (800.000 cells per dish in 2 mL of culture medium).

Microscope Settings:

For acquisition of confocal images, we used an LSM880 Laser Scanning Confocal Microscope (Zeiss, Germany). Images were acquired with a 63X 1.4 oil immersion objective, fluorescence excitation was obtained using the 633 nm laser line, fluorescence emission was detected in the 634-735 nm range. Pixel size is 0.22 microns in the image. Image size is 224.9X224.9 microns.

**Flow Cytometry**

Analysis:

Bacteria and macrophages were washed with PBS prior to flow cytometry and analysed using a Guava easyCyte flow cytometer (Millipore). FCS3.0 files were analysed using Flowing Software (http://flowingsoftware.btk.fi/). Cells were first gated in the forward- / side-scatter plot to exclude debries, followed by quadrant gating in plots using the fluorescence channels as shown above. For discrimination of live/dead cell discrimination cells in PBS were supplemented with 0.4 μL/mL propidium-iodide (5 mg/ml solution) followed by 5 min incubation on ice. Propidium iodide signal was recorded in the yellow channel.

Cell Sorting:

For separation of macrophages containing or not containing bacteria cells were washed twice with PBS, removed from the culture dishes using a rubber scraper and sorted using an Aria III Fluorescence Activated Cell Sorter (BD) with 100 μm nozzle and cooling. Sorting was performed with no more than 1000 events per second and 'Single Cell' purity setting.

**Quantitative Real-Time PCR**

For quantitative real-time PCR (qRT-PCR) analysis cell pellets were dissolved in Trizol reagent (Thermo Fisher) and RNA was extracted with chloroform. RNA from the aqueous phase was precipitated with isopropanol and washed once with 70% ethanol. Air-dried RNA was resuspended in water and treated with DNaseI (Thermo Fisher) according to the manufacturer's instructions. RNA was extracted with 25:24:1 phenol, chloroform, isoamylalkohol (Roth) and precipitated with 30:1 ethanol, sodium-acetate (3M, pH5.5). RNA-pellet was washed once with 70% ethanol, air-dried and resuspended in double-distilled water. Nucleic acid concentration was determined using a Nanodrop-2000 spectrometer.

To determine U6 and GyrA gene expression RNA samples were subjected to qRT-PCR analysis using the POWER SYBR Green RNAto-CT kit (Thermo Fisher) according to the manufacturer's instructions and a QuantStudio3 device (Applied Biosystems). Primer sequences (ordered from Metabion) were: U6 forward: GCTTCGGCAGCACATATACTAAAAT, U6 reverse: ATATGGAACGCTTCAC-GAATTTG, GyrA forward: TTACACCGGTCAACATTGAGG, GyrA reverse: TTCATGGCGTAAAGTACGCG. Gene expression differences were calculated based on the Threshold-Cycle (CT) values according to the $2^{-\Delta\Delta CT}$ formula.

**Bacterial DNA Preparation**

To obtain pure DNA for staining experiments chemically competent E. coli NEB5α cells were transformed with pUC18 plasmid DNA by heat-shock (42°C, 120 seconds) and subsequently shifted to LB medium containing Ampicillin (100 μg/ml). After shaking for 12 h at 37°C and 180 rpm bacteria were pelleted by centrifugation and pUC18 plasmid DNA was isolated using the Plasmid Pure column purification kit (Macherey Nagel) according to the manufacturer's instructions. For experiments with linearized bacterial DNA pUC18 plasmid DNA was incubated overnight with KpnI restriction enzyme (Thermo Fisher) at 37°C, followed by purification using the GeneJet PCR purification kit (Thermo Fisher) according to the manufacturer's instructions. Linearized DNA was eluted from the purification column with double-distilled water.

**Exemplary Synthesis**

Exemplary Synthesis of the Quinolinium Iodide (**5**) (cf. Fig. 31):

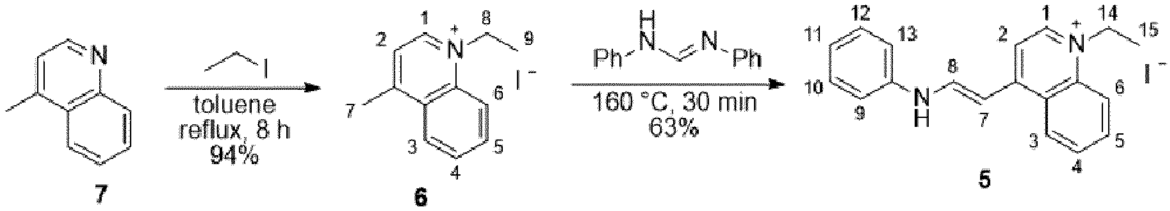


1-ethyl-4-methylquinolin-1-ium iodide (***6***): Following the procedure of PENG et al., lepidine (**7**, 143 mg, 1.00 mmol) was dissolved in toluene (1 mL) and ethyl iodide (0.10 mL, 1.25 mmol) was added. The mixture was refluxed in a sealed tube for 8 h. The resulting solid was crushed, washed several times with ethyl ether and dried under vacuum to yield the desired product **6** without further purification (280 mg, 0.94 mmol, 94%) as a green solid. $R_f$=0.35 ($CH_2Cl_2$/MeOH 10:1); $^1$H NMR (300 MHz, [D6]DMSO, 25°C): δ=9.42 (d, 1H, 3*J*(H,H)=6.0 Hz; H-1), 8.60 (d, 1H, 3*J*(H,H)=8.9 H; H-6), 8.55 (d, 1H, 3*J*(H,H)=8.6 Hz; H-3), 8.26 (dd, 1H, 3*J*(H,H)=8.9 Hz, 3*J*(H,H)=8.6 Hz; H-5), 8.01 - 8.11 (m, 2H; H-1 and H-4), 5.05 (q, 2H, 3*J*(H,H)=7.2 Hz; H-8), 3.01 (s, 3H; H-7), 1.59 (t, 3H, 3*J*(H,H)=7.2 Hz; H-9); $^{13}$C NMR (75 MHz, [D6] DMSO, 25°C): *δ*=158.3 (Cq), 148.1 (CH), 136.5 ($C_q$), 135.1 (CH), 129.5 (CH), 128.9 ($C_q$), 127.1 (CH), 122.8 (CH), 119.2 (CH), 52.5 ($CH_2$), 19.6 ($CH_3$), 15.2 ($CH_3$); HRMS-ESI$^+$ (*m*/*z*): [M]$^+$ calcd for $C_{12}H_{14}N$, 172.1121; found, 172.1122.

(E)-1-ethyl-4-(2-(phenylamino)vinyl)quinolin-1-ium iodide (**5**): Following a procedure of KISHINO et al., the iminium salt **6**, (200 mg, 0.67 mmol) and N,N'-diphenylformamidine (131 mg, 0.67 mmol) were mixed under vigorous stirring at 160°C for 30 min. The mixture was allowed to cool down to 25°C. The resulting solid was crushed and washed several times with ethyl ether. The crude product was recrystallized from ethanol. The crystallization was completed within one night at 0°C. The precipitate was filtered off and washed with ethanol and ethyl ether to yield the desired product **5** (169 mg, 0.42 mmol, 63%) as a brown solid. $R_f$=0.33 ($CH_2Cl_2$/MeOH 10:1); $^1$H NMR (300 MHz, [D6]DMSO, 25°C): δ=10.94 (s, 1H; NH), 8.89 (d, 1H, 3J(H,H)=12.5 Hz; H-8), 8.69 (d, 1H, 3J(H,H)=7.1 Hz; H-1), 8.32 (d, 1H, 3J(H,H)=8.6 Hz; H-6), 8.22 (d, 1H, 3J(H,H)=8.7 Hz; H-3), 8.15 (d, 1H, 3J(H,H)=7.1 Hz; H-2), 8.05 (dd, 1H, 3J(H,H)=8.7 Hz, 3J(H,H)=7.3 Hz; H-4), 7.81 (dd, 1H, 3J(H,H)=7.3 Hz, 3J(H,H)=8.6 Hz; H-5), 7.39 - 7.48 (m, 4H; H-9, H-10, H-12 and H-13), 7.13 (dd, 1H, 3J(H,H)=7.2 Hz, 3J(H,H)=7.2 Hz; H-11), 6.74 (d, 1H, 3J(H,H)=12.5 Hz; H-7), 4.70 (q, 2H, 3J(H,H)=7.2 Hz; H-14), 1.48 (t, 3H, 3J(H,H)=7.2 Hz; H-15); $^{13}$C NMR (75 MHz, [D6]DMSO, 25°C): δ=153.9 ($C_q$), 145.8 (CH), 143.3 (CH), 139.9 ($C_q$), 137.4 ($C_q$), 134.0 (CH), 129.6 (3C; CH), 127.3 (CH), 125.0 (CH), 123.8 (CH), 123.8 ($C_q$), 118.3 (CH), 116.6 (CH), 109.9 (CH), 95.9 (CH), 49.8 ($CH_2$), 14.8 ($CH_3$); HRMS-ESI$^+$ (m/z): [M]$^+$ calcd for $C_{19}H_{19}N_2$, 275.1543; found, 275.1545.

**Exemplary Synthesis of the N-alkylated Benzothiazolium Intermediate (8)** (cf. Fig. 32)

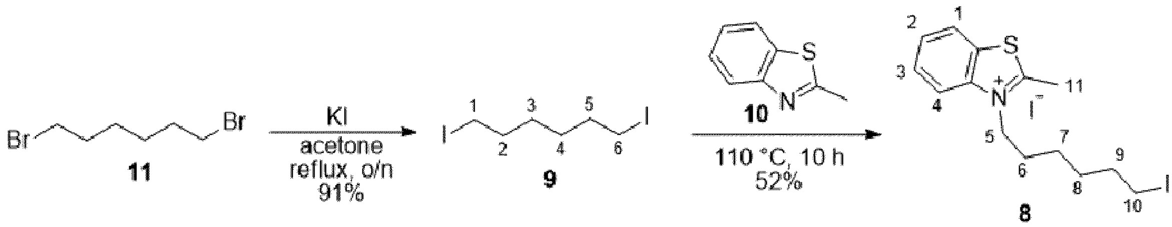


1,6-diidohexane (**9**): Following the procedure of SCHEUFFLER et al. 1,6-dibromohexane (11, 20.0 mL, 130 mmol) was dissolved in acetone (500 mL) and potassium iodide (65.7 g, 396 mmol) was added. The suspension was refluxed overnight. After full conversion of the starting material the solvent was removed under reduced pressure. The remaining solid was resuspended in chloroform and filtered. The resulting solution was washed with destilled water; the aqueous layer was extracted once more with chloroform. The combined organic layers were dried over magnesium sulfate and the solvent was removed under reduced pressure to yield the desired product **9** without further purification (39.8 g, 118 mmol, 91%) as a pale orange liquid. $R_f$=0.86 (n-pentane); $^1$H NMR (300 MHz, $CDCl_3$, 25°C): δ=3.19 (t, 4H, 3J(H,H)=7.0 Hz; H-1 and H-6), 1.79 - 1.89 (m, 4H; H-2 and H-5), 1.39 - 1.47 (m, 4H; H-3 and H-4); $^{13}$C NMR (75 MHz, $CDCl_3$, 25°C): δ=33.3 (2C; $CH_2$), 29.5 (2C; $CH_2$), 6.8 (2C; $CH_2$); HRMS-EI$^+$ (m/z): [M]$^+$ calcd for $C_6H_{l2}I_2$, 337.90284; found, 337.90185.

3-(6-iodohexyl)-2-methylbenzo[d]thiazol-3-ium (**8**): Under inert atmosphere, 2-methylbenzothiazole (10, 500 μL, 3.93 mmol) was added to 1,6-diidohexane (**9**, 3.24 mL, 19.7 mmol) and stirred at 110°C for 21.5 h. The reaction mixture was cooled to 25°C over 30 min and cooled for another 1 h at 4°C. The precipitate was filtered off and washed with cold ethyl ether to yield the desired product **8** (730 mg, 2.03 mmol, 52%) as pale orange solid. $R_f$=0.39 ($CH_2Cl_2$/MeOH 10:1); $^1$H NMR (300 MHz, [D6]DMSO, 25°C): δ=8.45 (d, 2H, 3J(H,H)=8.0 Hz; H-4), 8.35 (d, 2H, 3J(H,H)=8.5 Hz; H-1), 7.89 (dd, 2H, 3J(H,H)=8.5 Hz, 3J(H,H)=7.2 Hz; H-2), 7.80 (dd, 2H, 3J(H,H)=8.0 Hz, 3J(H,H)=7.2 Hz; H-3), 4.70 (t, 2H, 3J(H,H)=7.8 Hz; H-5), 3.28 (t, 2H, 3J(H,H)=6.8 Hz; H-10), 3.22 (s, 3H; H-11), 1.80 - 1.91 (m, 2H; H-6), 1.71 - 1.80 (m, 2H; H-9), 1.36 - 1.53 (m, 4H; H-7 and H-8); $^{13}$C NMR (75 MHz, [D6]DMSO, 25°C): δ=177.1 ($C_q$), 140.8 ($C_q$), 129.4 (CH), 129.1 ($C_q$), 128.1 (CH), 124.6 (CH), 116.9 (CH), 49.1 ($CH_2$), 32.6 ($CH_2$), 29.4 ($CH_2$), 27.6 ($CH_2$), 24.7 ($CH_2$), 16.9 ($CH_3$), 8.8 ($CH_2$); HRMS-ESI$^+$ (m/z): [M]$^+$ calcd for $C_{14}H_{19}INS$, 360.0277; found, 360.0275.

**Exemplary Synthesis of the Azido Cyanine Dye Derivative 6-AzTO-3 (1)** (cf. Fig. 33)

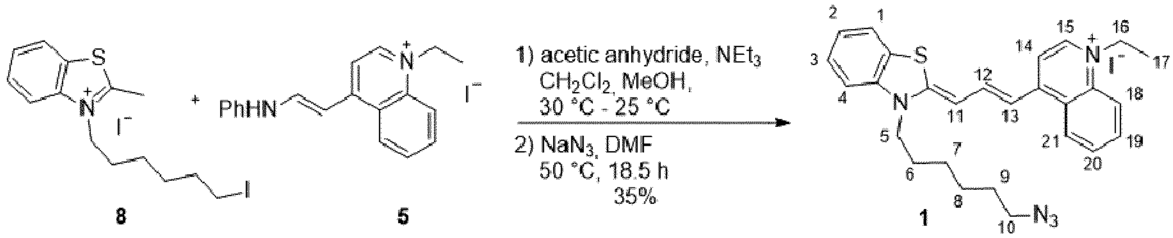


8 5 1

4-((1E,3Z)-3-(3-(6-azidohexyl)benzo[d]thiazol-2(3H)-ylidene)prop-1-en-1-yl)-1-ethylquinolin-1-ium iodide (**1**): The quinolinium (**5**, 250 mg, 0.62 mmol) and thiazolium (**8**, 303 mg, 0.62 mmol) were dissolved in dichloromethane (1.5 mL) and methanol (1.5 mL). Acetic anhydride (585 μL, 6.20 mmol) and triethylamine (605 μL, 4.36 mmol) were added and the resulting mixture was heated at 30°C for one min. Afterwards, the reaction flask was wrapped in aluminum foil and stirring in the dark at 25°C for another 1.5 h, then the solvent was removed. The crude was washed with destilled water, dissolved in dichloromethane, dried over magnesium sulfate and purified by flash column chromatography (allox, MeOH in $CH_2Cl_2$: 0% → 0.1% → 0.2% → 0.3% → 0.4%). Under inert atmosphere, the obtained product was dissolved in DMF (3 mL) and sodium azide (53.0 mg, 0.82 mmol) was added. The resulting mixture was heated at 50°C for 18.5 h. The solvent was removed and the product purified by flash column chromatography (allox, MeOH in $CH_2Cl_2$: 0% → 0.1% → 0.2% → 0.3% → 0.4%) to yield the desired product (1, 130 mg, 0.22 mmol, 35%) as blue solid. $R_f$=0.37 ($CH_2Cl_2$/MeOH 10:1); $^1$H NMR (300 MHz, [D4]MeOD, 25°C): δ=8.32 (d, 1H, 3J(H,H)=8.4 Hz; H-18), 8.05 (d, 1H, 3J(H,H)=7.2 Hz; H-15), 7.96 (dd, 1H, 3J(H,H)=12.3 Hz, 3J(H,H)=13.3 Hz; H-12), 7.75 - 7.78 (m, 2H; H-20 and H-21), 7.51 - 7.56 (m, 2H; H-4 and H-19), 7.49 (d, 1H, 3J(H,H)=7.2 Hz; H-14), 7.26 (dd, 1H, 3J(H,H)=8.2 Hz, 3J(H,H)=7.2 Hz; H-2), 7.16

(d, 1H, 3J(H,H)=8.2 Hz; H-1), 7.12 (dd, 1H, 3J(H,H)=7.9 Hz, 3J(H,H)=7.2 Hz; H-3), 6.95 (d, 1H, 3J(H,H)=13.3 Hz; H-13), 6.43 (d, 1H, 3J(H,H)=12.3 Hz; H-11), 4.40 (q, 2H, 3J(H,H)=7.2 Hz; H-16), 4.10 (t, 2H, 3J(H,H)=7.8 Hz; H-5), 3.30 - 3.33 (m, 2H; H-10), 1.73 - 1.79 (m, 2H; H-6), 1.58 - 1.65 (m, 2H; H-9), 1.46 - 1.53 (m, 7H; H-7, H-8 and H-17); $^{13}$C NMR (75 MHz, [D4]MeOD, 25°C): δ=163.2 (Cq), 152.4 ($C_q$), 145.1 (CH), 142.8 ($C_q$), 142.4 (CH), 139.3 ($C_q$), 134.4 (CH), 128.7 (CH), 127.6 (CH), 126.8 (CH), 126.4 ($C_q$), 126.0 ($C_q$), 125.3 (CH), 123.4 (CH), 118.2 (CH), 113.1 (CH), 110.6 (2C; CH), 99.8 (CH), 52.4 ($CH_2$), 50.9 ($CH_2$), 47.0 ($CH_2$), 29.8 ($CH_2$), 28.3 ($CH_2$), 27.6 ($CH_2$), 27.3 ($CH_2$), 15.0 ($CH_3$); $t_R$=33.3 min (5% to 75% B in 30 min); HRMS-ESI$^+$ (m/z): [M]$^+$ calcd for $C_{27}H_{30}N_5S$, 456.2216; found, 456.2215.

**Exemplary Synthesis of the Amine Cyanine Dyes: 6-TramTO-3 (2) and 6-NH$_2$TO-3 (3) (cf. Fig. 34)**

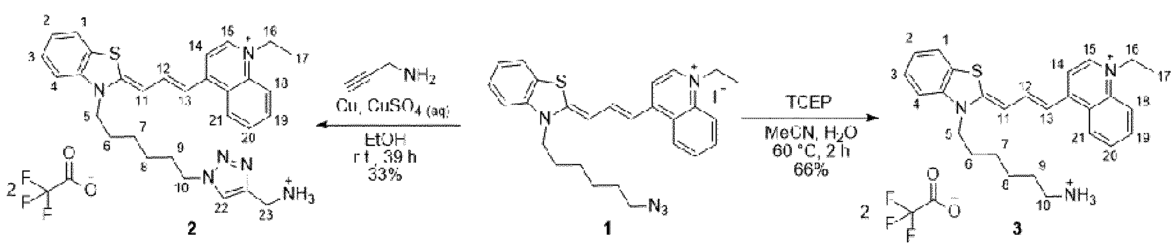


2 1 3

4-((1E,3Z)-3-(3-(6-(4-(aminomethyl)-1H-1,2,3-triazol-1-yl)hexyl)benzo[d]thiazol-2(3H)-ylidene)prop-1-en-1-yl)-1-ethylquinolin-1-ium iodide (**6-TramTO-3**): Under inert atmosphere, azido derivative cyanine dye **6-AzTO-3** (**1,** 49.0 mg, 84.0 μmol) was dissolved in ethanol (415 μL) and propargylamine (10.7 μL, 168 μmol), copper powder (6.4 mg, 101 μmol) and copper sulfate (100 mM, 42 μL, 4.20 μmol) were added. The resulting mixture was stirred for 39 h, filtered and the solvent was removed. The product was purified by preparative HPLC (5 -75%) to yield the desired product (**2**, 20.5 mg, 27.8 μmol, 33%) as blue solid. $R_f$=0.08 ($CH_2Cl_2$/MeOH 10:1); $^1H$ NMR (300 MHz, [D6]DMSO, 25°C): δ=8.48 (d, 1H, 3J(H,H)=8.4 Hz; H-18), 8.45 (d, 1H, 3J(H,H)=7.2 Hz; H-15), 8.31 (s, 2H; NH2), 8.09 - 8.18 (m, 3H; H-12, H-21 and H-22), 7.97 (dd, 1H, 3J(H,H)=8.4 Hz, 3J(H,H)=7.3 Hz; H-20), 7.86 - 7.88 (m, 2H; H-4 and H-14), 7.71 (dd, 1H, 3J(H,H)=8.4 Hz, 3J(H,H)=7.3 Hz; H-19), 7.58 (d, 1H, 3J(H,H)=8.2 Hz; H-1), 7.48 (dd, 1H, 3J(H,H)=8.2 Hz, 3J(H,H)=7.2 Hz; H-2), 7.31 (dd, 1H, 3J(H,H)=8.0 Hz, 3J(H,H)=7.2 Hz; H-3), 7.13 (d, 1H, 3J(H,H)=13.2 Hz; H-13), 6.50 (d, 1H, 3J(H,H)=12.3 Hz; H-11), 4.61 (q, 2H, 3J(H,H)=7.2 Hz; H-16), 4.40 (t, 2H, 3J(H,H)=7.1 Hz; H-10), 4.22 (t, 2H, 3J(H,H)=7.6 Hz; H-5), 4.09 - 4.13 (m, 2H; H-23), 1.79 - 1.86 (m, 2H; H-6), 1.68 - 1.76 (m, 2H; H-9), 1.44 - 1.49 (m, 5H; H-8 and H-17), 1.29 - 1.36 (m, 2H; H-7); $^{13}C$ NMR (75 MHz, [D6]DMSO, 25°C): δ=160.9 ($C_q$), 150.5 ($C_q$), 144.0 (CH), 142.3 (CH), 141.5 ($C_q$), 140.1 ($C_q$), 137.7 ($C_q$), 133.5 (CH), 127.7 (CH), 126.7 (CH), 125.3 (CH), 124.8 ($C_q$), 124.3 ($C_q$), 124.2 (2C; CH), 122.7 (CH), 117.9 (CH), 112.5 (CH), 110.0 (CH), 109.5 (CH), 98.5 (CH), 49.4 (2C; $CH_2$), 45.5 ($CH_2$), 34.0 ($CH_2$), 29.7 (CH2), 26.9 ($CH_2$), 25.6 ($CH_2$), 25.5 ($CH_2$), 14.8 ($CH_3$); tR=24.2 min (5% to 75% B in 30 min); HRMS-ESI$^+$ (m/z): $[M]^+$ calcd for $C_{30}H_{35}N_6S$, 511.2638; found, 511.2641.

mono(4-((1E,3Z)-3-(3-(6-ammoniohexyl)benzo[d]thiazol-2(3H)-ylidene)prop-1-enyl)-1-ethylquinolinium) mono(2,2,2-trifluoroacetate) (**6-NH$_2$TO-3**): Azido derivative cyanine dye (**1**, 40 mg, 87.6 μmol) was dissolved in acetonitrile (600 μL) and a solution of TCEP in milli-Q water (0.44 M, 600 μL, 263 μmol) was added. The resulting mixture was stirred for 2 h at 60°C afterwards it was purified by preparative HPLC (5 -75%) to yield the desired product (**3**, 31.5 mg, 57.8 μmol, 66%) as blue solid. $R_f$=0.05 ($CH_2Cl_2$/MeOH 10:1); $^1H$ NMR (500 MHz, [D6]DMSO, 25°C): δ=8.43 - 8.52 (m, 2H; H-15 and H-18), 8.13 - 8.20 (m, 1H; H-12), 8.10 (d, 1H, 3J(H,H)=8.6 Hz; H-21), 7.97 (dd, 1H, 3J(H,H)=7.7 Hz, 3J(H,H)=8.6 Hz; H-20), 7.86 - 7.92 (m, 2H; H-4 and H-14), 7.67 - 7.79 (m, 4H; H-19 and NH3), 7.60 (d, 1H, 3J(H,H)=8.0 Hz; H-1), 7.49 (dd, 1H, 3J(H,H)=7.3 Hz, 3J(H,H)=8.0 Hz; H-2), 7.31 (dd, 1H, 3J(H,H)=7.3 Hz, 3J(H,H)=7.5 Hz; H-3), 7.14 (d, 1H, 3J(H,H)=13.2 Hz; H-13), 6.52 (d, 1H, 3J(H,H)=12.2 Hz; H-11), 4.61 (q, 2H, 3J(H,H)=7.2 Hz; H-16), 4.24 (t, 2H, 3J(H,H)=7.1 Hz; H-5), 2.75 - 2.82 (m, 2H; H-10), 1.69 - 1.78 (m, 2H; H-6), 1.52 - 1.58 (m, 2H; H-9), 1.37 - 1.50 (m, 7H; H-7, H-8 and H-17); $^{13}C$ NMR (125 MHz, [D6]DMSO, 25°C): δ=158.1 ($C_q$), 150.5 ($C_q$), 144.0 (CH), 142.3 (CH), 141.5 ($C_q$), 137.7 ($C_q$), 133.5 (CH), 127.7 (CH), 126.7 (CH), 125.3 (CH), 124.7 ($C_q$), 124.3 ($C_q$), 124.2 (CH), 122.7 (CH), 118.0 (CH), 112.5 (CH), 110.3 (CH), 109.5 (CH), 98.4 (CH), 49.4 ($CH_2$), 45.5 ($CH_2$), 38.7 ($CH_2$), 26.9 ($CH_2$), 26.9 ($CH_2$), 25.6 (2C; $CH_2$), 14.8 ($CH_3$); $t_R$=23.5 min (5% to 75% B in 30 min); HRMS-ESI$^+$ (m/z): $[M]^+$ calcd for $C_{27}H_{32}N_3S$, 430.2311; found, 430.2302.

**Exemplary Excitation and Emission Spectra of the NIR Cyanine Dye Derivatives**

For each cyanine dye derivative: **6-AzTO-3, 6-TramTO-3** and **6-NH$_2$TO-3,** the excitation and emission spectra of a 10 μM solution of the corresponding compound in 1 mL of 10 mM Tris pH 7.6 and 50 mM KCl were measured. The excitation spectra were recorded at an emission of 670 nm in a rage from 500 - 660 nm (slit 5/10 nm), while the emission spectra were measured at an excitation of 590 nm in a range from 600 - 750 nm (slit 5/10 nm). (cf. Fig. 13)

**Exemplary Fluorescence Emission of the NIR Cyanine Dye Derivatives with and without dsDNA$_{CT}$**

We compared the fluorescence properties of different cyanine dyes: **6-AzTO-3, 6-TramTO-3** and **6-NH$_2$TO-3** in presence of doublestranded calf thymus DNA ($dsDNA_{CT}$). The emission spectra of a 1 μM solution of the corresponding compound in 120 μL of 10 mM Tris pH 7.6 and 50 mM KCl were measured in presence and absence of 15 μM $dsDNA_{CT}$. A 100 μL fluorescence cuvette of HellmaAnalytics (105.250-QS) was used. The spectra were measured using an excitation and emission slits of 5 nm. (cf. Fig. 14)

**Exemplary Fluorescence Titrations of 6-TramTO-3**

All fluorescence titrations followed the same procedure: to 1 mL of 1 μM solution of corresponding cyanine dye derivative in 10 mM Tris pH 7.6 and 50 mM KCl aliquots of a stock solution of the corresponding annealed DNA (dsDNAhAT and $dsDNA_{hGC}$ = 435 μM; $dsDN_{ACT}$ = 0.65 mM) were successively added and the fluorescence spectra were recorded in a range from 625-750 nm after 1 min of each addition. For each compound three independent measurements were carried out. The fluorescence emission plotted is the resulting mean of the three independent measurements. (cf. Fig. 15)

### Binding Affinity Determination by DynaFit

For the determination of the binding affinity, the maximum of each fluorescence spectrum (647 nm in the case of dsDNACT, 647 nm in the case of $dsDNAh_{AT}$ and 650 nm in the case of $dsDNA_{hGC}$) was plotted against the added dsDNA concentration. The mean values from three independent measurements with its standard deviation is shown. The KD-value and its standard deviation was determined using the program DynaFit. Table 2 shows the determined KD-values.

Table 2: Determined binding constants of **6-TramTO-3** with $dsDNA_{CT}$, $dsDNA_{hAT}$ and dsDNAhGC.

| DNA | binding affinity [μM] |
|---|---|
| $dsDNA_{CT}$ | 19.5 ± 2.6 |
| $dsDNA_{hAT}$ | 0.114 ± 0.03 |
| $dsDNA_{hGC}$ | 0.104 ± 0.054 |

All binding affinities were determined by fitting the obtained experimental data in the DynaFit 4.0 software. The program is available free of charge for academical use at http://www.biokin.com/dynafit/ In this program it is necessary to create scripts containing information about the chemical model underlying the experimental data, the concentration of the fixed component, determined spectroscopic data of the titration and information about the location of files. An example of a script is shown below with comments (right column) about the purpose of the keywords:

| | | |
|---|---|---|
| [task] | | |
| | task = fit | nature of the calculation performed by Dynafit |
| | data = equilibria | |
| [mechanism] | | simple 1:1 binding model |
| | P + LL <==> P.LL : KdL dissociation | indicates that it is a dissociation constant initial KD-value for iteration the ? indicates that this value will be optimized |
| [constants] | | |
| | KdL = 2 ? | |
| [concentrations] | | |
| | LL = 1 | fixed concentration of the cyanine dye derivative contribution to the spectroscopic signal (adjustable parameters, as is specified by the question mark after the numerical value) fluorescence intensity of the dye alone fluorescence intensity of the saturated DNA / dye signal location of files and information about the data indicates the species that changes its concentration path of the experimental data file |
| [responses] | | |
| | LL = 5.8 ? | |
| | P.LL = 155.8 ? | |
| [data] | | |
| | variable P | |
| | file ./Measurements/ data/BH574_mean.txt | |
| [output] | | |
| | directory ./ Measurements/ output/BH574_mean | path indicating the location of the output file |
| [end] | | |

### Fluorescence Quantum Yield

The slopes for the determination of the quantum yields and their calculated quantum yield values are summarized in table 3.

Table 3: Determined slopes and corresponding quantum yields for the two reference compounds Alexa Flour® 647 and Atto 655 and for **6-TramTO-3** with and without $dsDNA_{CT}$.

| dye | slope | mean | reference dye | quantum yield | quantum yield (lit.) |
|---|---|---|---|---|---|
| Alexa Fluor® 647 | 52937300 | 51185000 | Atto 655 | 0.32 | 0.33 |
| Alexa Fluor® 647 | 49432700 | | | | |
| Atto 655 | 48486000 | 47618300 | Alexa Fluor® 647 | 0.31 | 0.30 |
| Atto 655 | 46750600 | | | | |
| **6-TramTO-3** alone | 792132 | 809683 | Alexa Fluor® 647 | 0.0052 | |
| **6-TramTO-3** alone | 822593 | | | | |
| **6-TramTO-3** alone | 814323 | | | | |
| **6-TramTO-3** + DNA | 41583400 | 42097433 | Alexa Fluor® 647 | 0.27 | |
| **6-TramTO-3** + DNA | 41800000 | | | | |
| **6-TramTO-3** + DNA | 42908900 | | | | |

**Exemplary Concentration Determination**

For the accurate determination of our compounds **6-TramTO-3** the extinction coefficient was calculated. A specific amount (< 2.00 mg) of the compound was weighted on a Mettler Toledo XP6 and dissolved in a known amount of milli-Q water. Increasing amounts of such stock solution were added into a 1 mL cuvette filled with 1 ml of milli-Q water. The corresponding absorbance of each addition was measured on a Tecan Spark 20M at the maximum at 625 nm and without exceeding 10% of the initial volume. In all the cases the absorbance was between 0.1 and 1 AU. to be in concordance with Lambert-Beer law (equation **E2**).

$$A = \varepsilon\, l\, c \qquad (\mathbf{E2})$$

In this equation (**E2**): A = absorbance of the solution; l = length of the cuvette; c = concentration; ε = molar extinction coefficient.

The lineal regression of the obtained absorbance measurements versus the concentrations of **6-TramTO-3** allowed us to obtain the molar extinction coefficient. These measurements were repeated three times independently i.e. from three different stock solutions. The mean extinction coefficient and the standard deviation of **6-TramTO-3** at a wavelength of 625 nm in milli-Q water was 59403 ±646 $M^{-1}cm^{-1}$.

The mean extinction coefficient and the standard deviation of **6-$NH_2$TO-3** and **6-AzTO-3** were determined following the above described procedure. The extinction coefficient of **6-$NH_2$TO-3** was determined in milli-Q water, whereas the extinction coefficient of **6-AzTO-3** was determined in DMSO due to its low aqueous solubility. Fig. 19 shows the corresponding UV-Vis spectra in 1 mL 10 mM Tris pH 7.6 and 50 mM KCl at a dye concentration of 10 µM. For the spectrum of **6-AzTO-3** 1% of DMSO was added for a better solubility. Fig. 20 and Fig. 21 show the linear regressions from the three independent measurements. Table 4 summarized the molar extinction coefficient obtained.

Table 4: Determined mean extinction coefficients and standard deviations of **6-AzTO-3** in DMSO and **6-TramTO-3** and **6-$NH_2$TO-3** in milli-Q water.

| | **6-AzTO-3** in DMSO | **6-TramTO-3** in milli-Q water | **6-$NH_2$TO-3** in milli-Q water |
|---|---|---|---|
| mean | 110203 ± 1577 $M^{-1}cm^{-1}$ | 59403 ± 646 $M^{-1}cm^{-1}$ | 90043 ± 418 $M^{-1}cm^{-1}$ |

Syto-9™ was used as a 3.34 mM DMSO stock commercial solution. For EtBr the extinction coefficient $\varepsilon_{480nm}$ = 5600 $M^{-1}cm^{-1}$ was used. $dsDNA_{CT}$ was purchased as a solution of 15.4 mM solution.

For the determination of the concentration of the hairpin DNAs, the purchased lyophilized DNA was dissolved in milli-Q water, heated to 95°C for 10 min and slowly cooled down to r.t. Their molar extinction coefficients were determined by using the following formula **E3**:

$$\varepsilon_{260nm} = \{(8.8 * \#T) + (7.3 * \#C) + (11.7 * \#G) + (15.4 * \#A)\} * 0.8 * 10^3\ M^{-1}cm^{-1},$$

wherein # = number of nuleobases determined throughout the DNA sequence, *T* = thymine, *C* = cytosine, *G* = guanine, *A* = adenine.

**UV-Vis Tritations**

UV-Vis titration measurements were performed at a concentration of 12.3 μM of the dye in 10 mM Tris pH 7.6 and 50 mM KCl. Increasing aliquots of a 1.54 mM and 15.4 mM stock solutions of $dsDNA_{CT}$ were successively added and the absorbance spectra were recorded in a range from 400-800 nm after 1 min of each addition. Three independent measurements were carried out. The presented spectra are the resulting mean of the three independent measurements.

We recorded the temperature dependence of the sample: to get a strong signal at 523 nm, a 12.3 μM solution of **6-TramTO-3** in 10 mM Tris pH 7.6 and 50 mM KCl was prepared and 7.5 μM $dsDNA_{CT}$ added. The measurement was started at a temperature at 2°C and the temperature increased in 4°C steps. After the device reached the set temperature we waited for 2 min before measurement. Fig. 23 shows the change of the UV-Vis spectra with increasing temperature. Further, the signal at 523 nm and 625 nm were plotted against the temperature.

**CD Measurements**

The shown CD-Spectra in Fig. 24 represent the exemplary mean of the three independent measurements.

**Gel Electrophoresis**

Fig. 25 shows exemplarily the two gels with different amounts of $dsDNA_{bl}$ and $dsDNA_{cl}$ stained with **6-TramTO-3** or ethidium bromide.

**Thermal Melting Analysis**

Fig. 26 shows exemplarily the determined melting point for $dsDNA_{CT}$ with and without addition of **6-TramTO-3.**

**Fluorescence Microscopy**

Figs. 27A-C show confocal microscopy pictures of bacteria and macrophages stained with **6-TramTO-3** in the bright field, the red channel and the merged image.

**Additional Cell-Based Assays**

Fig. 28A shows the growth of E. coli in the presence of the indicated fluorescence dyes and Fig. 28B the fluorescence shift of the bacteria in the indicated FACS channels.

Figs. 29A-C show the labelling of antibiotic-resistant and -sensitive Klebsiella pneumoniae strains with **6-TramTO-3** (Fig. 29A), the quantification of colonies formed by DMSO- or **6-TramTO-3** labeled bacteria on solid agar (Fig. 29B) and Klebsiella growth in the presence of **6-TramTO-3** or DMSO (Fig. 29C).

**Description of the drawings**

Fig. 1: A) Exemplary schematic representation of growth-compatible bacterial labelling for host-pathogen studies: bacteria are exemplary labeled in vivo with near-infrared (NIR) fluorescent dyes and subsequently shifted into host cell cultures. Host cells containing bacteria may be identified by FACS or microscopy for detailed host-pathogen interaction analysis. B) Chemical structure of the exemplary synthesized and tested NIR dyes.

Fig. 2A: Exemplary measurement of optical density (OD600 nm) of E. coli LB cultures supplemented with DMSO (line marked with dots), **6-TramTO-3** (line marked with squares), CSFE (line marked with rhombuses) or Syto-9TM (line marked with triangles) over time. Mean data-points and standard deviations are derived from three independent experiments.

Fig. 2B: Representative exemplary dot plots of bacteria from Fig. 2A sampled at OD 600 nm = 2.0 and analyzed by FACS in the red1, red2, green and yellow channels.

Fig. 3A: Exemplary Fluorescence emission spectra in Tris buffer, pH 7.6 and 50 mM KCl at 20°C of a 1 μM solution of: **6-TramTO-3** alone (line referenced with "*"); **6-TramTO-3** in presence of 15 eq. of dsDNACT (line referenced with "#"); EtBr alone (line referenced with "+"); EtBr in presence of 15 eq. of dsDNACT (line referenced with "&"). The insert shows gel staining with **6-TramTO-3** of different dsDNAs; 1-3 lines: circular bacterial dsDNA; 4-6 lines: linearized bacterial dsDNA both in 1000 ng, 500 ng and 100 ng amount.

Fig. 3B: Exemplary Emission profiles at 647 nm of **6-TramTO-3** titrations with: dsDNACT (line referenced with "#"); dsDNAhGC (line referenced with "+"); dsDNAhAT (line referenced with "&"). All data are mean values derived from three independent measurements.

Fig. 4A: Exemplary UV-Vis spectra of 12.3 μM solution of: **6-TramTO-3** alone (dashed line, as is labeled in the graph); **6-TramTO-3** in presence of increasing eq. of dsDNACT (<2.5 eq.) (lines, as are labeled in the graph); **6-TramTO-3** in presence of increasing eq. of dsDNACT (>2.5 eq.) (lines, as are labeled in the graph) in Tris buffer, pH 7.6 with 50 mM KCl at 20°C. All data are mean values derived from three independent measurements.

Fig. 4B: Exemplary CD spectra of 36.9 μM solution of: **6-TramTO-3** alone (dashed line, as is labeled in the graph); **6-TramTO-3** in presence of increasing eq. of $dsDNA_{CT}$ (<2.5 eq.) (lines, as are labeled in the graph); **6-TramTO-3** in presence of increasing eq. of dsDNACT (>2.5 eq.) (lines, as are labeled in the graph) in Tris buffer, pH 7.6 with 50 mM KCl at 20°C. All data are mean values derived from three independent measurements.

Fig. 5: A) Exemplarily indicated dilutions of bacteria sampled at OD600 nm = 2.0 and spotted onto LB solid medium without antibiotics (LB), or supplemented with ampicillin (LB Amp), kanamycin (LB Kan) or chloramphenicol (LB Cm), followed by outgrowth for 48 h. E.co = E. coli; K.pnR = multiresistant K. pneumoniae; KpnS = antibiotic-sensitive K. pneumoniae. B) Exemplary FACS analysis (red2 and green channel) of all three strains, grown in the presence of DMSO or 6**-TramTO-3** and sampled at OD600 nm = 2.0.

Fig. 6: A) Human macrophages exemplarily stimulated with **6-TramTO-3-**labeled bacteria shift into the red-channel, indicative of bacterial uptake. B) Quantification of red-shifted macrophages. Data derived from 5 independent experiments. C) Quantification of propidium iodide (PI) positive macrophages with (red-shifted) or without (un-shifted) bacteria. D) Experimental scheme and qRT-PCR using RNA from both fractions in order to determine the relative abundance of human U6 snRNA versus bacterial housekeeper RNA GyrA. E. co. = E. coli; K. pnR = multiresistant K. pneumoniae; K. pnS = antibiotic-sensitive K. pneumoniae. Statistical significances were evaluated using a one-way Anova (B) or a two-tailed student's t-test (C). * = $P \leq 0.05$; ** = $P \leq 0.01$.

Fig. 7: Model illustrating the observed interaction of antibiotic-sensitive and -resistant bacteria with human macrophages.

Fig. 8A: Structure of **6-AzTO-3** (**1**) used in the bacteria cell-based assays and its corresponding HPLC chromatogram.

Fig. 8B: Structure of **6-TramTO-3** (**2**) used in the bacteria cell-based assays and its corresponding HPLC chromatogram.

Fig. 8C: Structure of **6-NH2TO-3** (**3**) used in the bacteria cell-based assays and its corresponding HPLC chromatogram.

Fig. 9A: $^{1}H$ NMR and $^{13}C$ NMR spectra of the compound (**8**).

Fig. 9B: COSY-NMR spectra of the compound (**8**).

Fig. 9C: HMBC-NMR spectra of the compound (**8**).

Fig. 10A: $^1$H-NMR spectrum of **6-AzTO-3** (**1**) and zoom in the region between 6.3 and 8.6 ppm.

Fig. 10B: $^{13}$C-NMR spectrum of **6-AzTO-3** (**1**).

Fig. 10C: COSY-NMR spectrum of **6-AzTO-3** (**1**)

Fig. 10D: HMBC-NMR spectrum of **6-AzTO-3** (**1**).

Fig. 11A: $^1$H-NMR spectrum of **6-TramTO-3** (**2**) and zoom in the in the region between 6.3 and 8.6 ppm.

Fig. 11B: $^{13}$C-NMR spectrum of **6-TramTO-3** (**2**).

Fig. 11C: COSY-NMR spectrum of **6-TramTO-3** (**2**).

Fig. 11D: HMBC-NMR spectrum of the **6-TramTO-3** (**2**)

Fig. 12A: $^1$H-NMR spectrum of **6-$NH_2$TO-3** (**3**) and zoom in the in the region between 6.3 and 8.6 ppm.

Fig. 12B: $^{13}$C-NMR spectrum of **6-$NH_2$TO-3** (**3**).

Fig. 12C: COSY-NMR spectra of **6-$NH_2$TO-3** (**3**).

Fig. 12D: HMBC-NMR spectra of **6-$NH_2$TO-3** (**3**).

Fig. 13: Exemplary Excitation (continuous line) and emission (dashed line) spectra of 10 μM solution of cyanine dye: a) **6-AzTO-3**; b) **6-TramTO-3** and c) **6-$NH_2$TO-3** in 1 mL 10 mM Tris pH 7.6 mM and 50 mM KCl at 20°C.

Fig. 14: Exemplary Emission spectra of 1 μM solution of cyanine dye in presence and absence of 15 μM dsDNACT. All spectra were measured in 120 μL 10 mM Tris pH 7.6 and 50 mM KCl at 20°C.

Fig. 15: Exemplary Fluorescence spectra of the titration of **6-TramTO-3** with a) dsDNACT; b) dsDNAhAT; c) dsDNAhGC. All spectra were measured of a 1 μM solution of cyanine dye in 10 mM Tris pH 7.6 mM and 50 mM KCl at 20°C.

Fig. 16: Exemplarily calculated binding affinities from the fluorescence spectra of the titration of **6-TramTO-3** with a) $dsDNA_{CT}$; b) $dsDNA_{hAT}$; c) $dsDNA^{hGC}$.

Fig. 17: Exemplary UV-Vis spectrum of a 10 μM solution of **6-TramTO-3** in 1 mL 10 mM Tris pH 7.6 and 50 mM KCl.

Fig. 18: Exemplary Determination of the extinction coefficient of compound **6-TramTO-3** in milli-Q water. The absorbance maximum at 625 nm was plotted against the dye concentration. Three independent measurements are shown with the calculated extinction coefficient and their corresponding regression value ($R^2$). The mean extinction coefficient and the standard deviation of **6-TramTO-3** is 59403 ± 646 $M^{-1}cm^{-1}$.

Fig. 19: Exemplary UV-Vis spectra: a) UV-Vis spectra of compound **6-AzTO-3** in 1 mL 10 mM Tris pH 7.6 and 50 mM KCl at a dye concentration of 10 μM and 1% DMSO. b) UV-Vis spectra of compound **6-NH2TO-3** in 1 mL 10 mM Tris pH 7.6 and 50 mM KCl at a dye concentration of 10 μM.

Fig. 20: Exemplary Determination of the extinction coefficient of compound **6-AzTO-3** in DMSO. The absorbance maximum at 639 nm was plotted against the dye concentration. Three independent measurements (a, b, c) are shown with the calculated extinction coefficient and their corresponding regression value ($R^2$). The mean extinction coefficient and the standard deviation of **6-AzTO-3** is 110203 ± 1577 $M^{-1}cm^{-1}$.

Fig. 21: Determination of the extinction coefficient of compound **6-$NH_2$TO-3** in milli-Q water. The absorbance maximum at 625 nm was plotted against the dye concentration. Three independent measurements (a, b, c) are shown with the calculated extinction coefficient and their corresponding regression value ($R^2$). The mean extinction coefficient and the standard deviation of **6-$NH_2$TO-3** is 90043 ± 418 $M^{-1}cm^{-1}$.

Fig. 22: Exemplary UV-Vis spectra of the titrations of a 12.3 μM solution of **6-TramTO-3** in 10mM Tris pH 7.6 and 50mM KCl with increasing amounts of $dsDNA_{CT}$ where a) the equivalents of $dsDNA_{CT}$ are: 0, 0.4, 0.7, 1.1, 1.5, 2.3; b) the equivalents of $dsDNA_{CT}$ are: 2.3, 3.0, 3.8, 5.3, 6.8, 10.5, 18.0, 25.5, 40.6, 55.6, 70.6 and c) is the combined titration of a and b.

Fig. 23: Exemplary Temperature dependence of the UV-Vis signal of **6-TramTO-3** in 10 mM Tris pH 7.6 and 50 mM KCl with 7.5 μM) dsD-NACT. a) full UV-Vis spectra with increasing temperature from 2°C to 78°C; b) plot of the absorbance at 523 nm against the temperature; c) plot of the absorbance at 635 nm against the temperature.

Fig. 24: Exemplary CD spectra of the titrations of a 36.9 μM solution of **6-TramTO-3** in 10mM Tris pH 7.6 and 50 mM KCl with increasing amounts of $dsDNA_{CT}$ where a) the equivalents of $dsDNA_{CT}$ are: 0, 0.4, 0.8, 1.2; b) the equivalents of $dsDNA_{CT}$ are 2.4, 4.9, 9.8, 20.3 and 40.7 c) is the complete titration.

Fig. 25: Exemplary 1% agarose gel electrophoresis with 1000 ng, 500 ng and 100 ng of $dsDNA_{bc}$ and $dsDNA_{bl}$. CSL 1kb DNA ladder was used as a reference. The gel was run for 75 min at 25°C at 80 V and post stained with either a) 1 μM of **6-TramTO-3** in TAE buffer or b) 1 μM ethidium bromide in TAE buffer.

Fig. 26: Exemplary melting temperature measurements of 45 μM $dsDNA_{CT}$ with and without addition of 1.8 μM **6-TramTO-3** in 10 mM Tris pH 7.5. The melting points were calculated to be 68.12°C for $dsDNA_{CT}$ alone and 72.66°C for $dsDNA_{CT}$ with addition of **6-TramTO-3.**

Fig. 17A: Exemplary confocal microscopy of bacteria and macrophages stained with **6-TramTO-3** (bright field).

Fig. 27B: Exemplary confocal microscopy of bacteria and macrophages stained with **6-TramTO-3** (red channel).

Fig. 27C: Exemplary confocal microscopy of bacteria and macrophages stained with **6-TramTO-3** (merged image of Fig. 27A and 27B).

Fig. 28A: E. coli was exemplarily grown in the presence of the indicated dyes and the optical density (OD600) was determined at various time-points. Mean values and standard-deviations from three independent experiments are shown.

Fig. 28B: Exemplary fluorescence shift of E. coli cells grown in the presence of the indicated dyes (Fig. 28A) was recorded by flow-cytometry at an optical density of 2.0 in the indicated channels.

Fig. 29A: Klebsiella pneumoniae strains (R = resistant, S = sensitive) were exemplarily grown in the presence of DMSO or **6-TramTO-3** and fluorescence shift of cells in the indicated channels at an optical density of 2.0 was recorded by flow-cytometry.

Fig. 29B: Exemplarily, 24 h after plating of E. coli and K. pneumoniae strains sampled at and optical density of 2.0, grown in the presence of DMSO or **6-TramTO-3,** onto LB solid agar plates colony forming units per ml of culture were determined (Y-axis). Error bars represent the standard deviation derived from three independent experiments.

Fig. 29C: K. pneumoniae (R = resistant, S = sensitive) was exemplarily grown in the presence of DMSO or **6-TramTO-3** and the optical density (OD600) was determined at various time-points. Mean values and standard-deviations from three independent experiments are shown.

Fig. 30: Application example of extracorporal medical investigation.

Fig. 31: Exemplary Synthesis of the Quinolinium Iodide (**5**)

Fig. 32: Exemplary Synthesis of the N-alkylated Benzothiazolium Intermediate (**8**)

Fig. 33: Exemplary Synthesis of the Azido Cyanine Dye Derivative 6-AzTO-3 (**1**)

Fig. 34: Exemplary Synthesis of the Amine Cyanine Dyes: 6-TramTO-3 (**2**) and 6-NH2TO-3 (**3**)

## Claims

1. A cyanine dye for use in binding to DNA, selected from the group of cyanine dyes being **characterized by** the general formula (**I**),

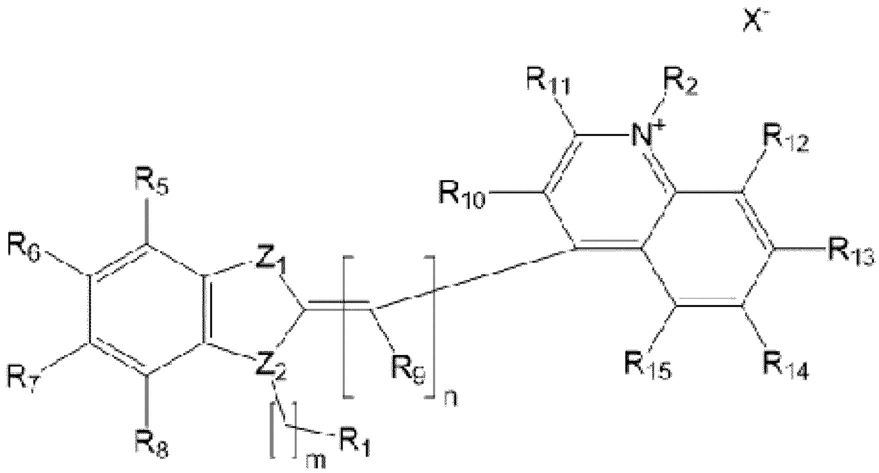

**(I)**

,

wherein

- $Z_1$ is S and $Z_2$ is N; and
- $R_1$ is selected from the list comprising 5-azidopentyl, 5-(4-(aminomethyl)-1H-1,2,3-triazol-1-yl)pentyl or 5-ammoniopentyl; and
- $R_2$ is ethyl and;
- $R_5$ to $R_{15}$ are each a hydrogen atom; and
- $X^-$ is selected from the list comprising halogenate, sulfate, triflate, trifluoro acetate, difluoro acetate or fluoro acetate; and
- n is 3; and
- m is 1.

2. A cyanine dye according to one of the proceeding claims, **characterized in that** it is binding to oligomeric DNA or primer-DNA or DNA-aptameres.

3. A cyanine dye according to one of the proceeding claims in its protonated or deprotonated form, whereat the counterion, resp. counterions, of the protonated or deprotonated form is, resp. are, independently selected from the list comprising halogenate, sulfate, triflate, trifluoro acetate, difluoro acetate or fluoro acetate.

4. A method for in vivo staining of microorganisms using a cyanine dye according to one of the preceding claims, **characterized in that** the method comprises the following steps:

   a) isolation of the microorganisms that are to be stained, whereat this isolation-step optionally includes one or more steps for concentrating and/or breeding of the microorganisms;
   b) staining the microorganisms with the cyanine dye by adding a solution of the cyanine dye to the microorganisms,

   whereat the viability of the microorganisms is not reduced.

5. A kit for carrying out the method according to claim 4, the kit comprising the cyanine dye and optionally supportive substances.

## Patentansprüche

**1.** Cyaninfarbstoff für die Verwendung zur Bindung an DNA, ausgewählt aus der Gruppe der Cyaninfarbstoffe, **gekennzeichnet durch** die allgemeine Formel (**I**),

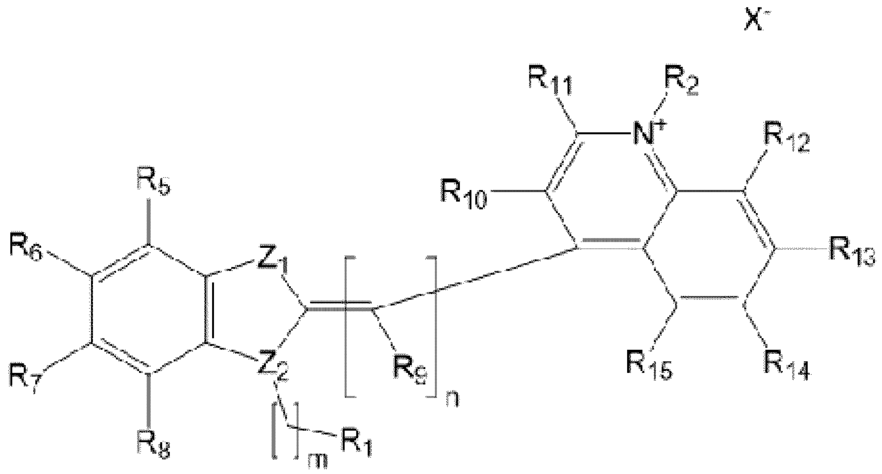

**(I)**

,

wobei

- $Z_1$ S ist und $Z_2$ N ist; und
- $R_1$ ausgewählt ist aus der Liste umfassend 5-Azidopentyl, 5-(4-(Aminomethyl)-1H-1,2,3-triazol-1-yl)pentyl oder 5-Ammoniopentyl; und
- $R_2$ Ethyl ist; und
- $R_5$ bis $R_{15}$ jeweils Wasserstoffatome sind; und
- $X^-$ ausgewählt ist aus der Liste umfassend Halogenat, Sulfat, Triflat, Trifluoracetat, Difluoracetat oder Fluoracetat; und
- n gleich 3 ist; und
- m gleich 1 ist.

**2.** Cyaninfarbstoff gemäß einem der vorangehenden Ansprüche, **dadurch gekennzeichnet dass** er an oligomere DNA oder Primer-DNA oder DNA-Aptamere bindet.

**3.** Cyaninfarbstoff gemäß einem der vorangehenden Ansprüche in seiner protonierten oder deprotonierten Form, wobei das Gegenion, bzw. die Gegenionen, der protonierten oder deprotonierten Form unabhängig voneinander ausgewählt ist, bzw. sind, aus der Liste umfassend Halogenat, Sulfat, Triflat, Trifluoracetat, Difluoracetat oder Fluoracetat.

**4.** Verfahren zum in-vivo-Färben von Mikroorganismen unter Verwendung eines Cyaninfarbstoffs gemäß einem der vorangehenden Ansprüche, **dadurch gekennzeichnet, dass** das Verfahren die folgenden Schritte umfasst:

a) Isolierung der Mikroorganismen, die gefärbt werden sollen, wobei dieser Isolierungsschritt optional einen oder mehrere Schritte zum Aufkonzentrieren und/oder der Vermehrung der Mikroorganismen umfasst;
b) Färben der Mikroorganismen mit dem Cyaninfarbstoff durch Zugabe einer Lösung des Cyaninfarbstoffs zu den Mikroorganismen, wobei die Lebensfähigkeit der Mikroorganismen nicht verringert wird.

**5.** Kit zur Durchführung des Verfahrens gemäß Anspruch 4, wobei das Kit den Cyaninfarbstoff und optional unterstützende Substanzen umfasst.

## Revendications

**1.** Colorant cyanine utilisé pour se lier à l'DNA, choisi dans le groupe des colorants cyanine **caractérisés par** la formule générale (**I**),

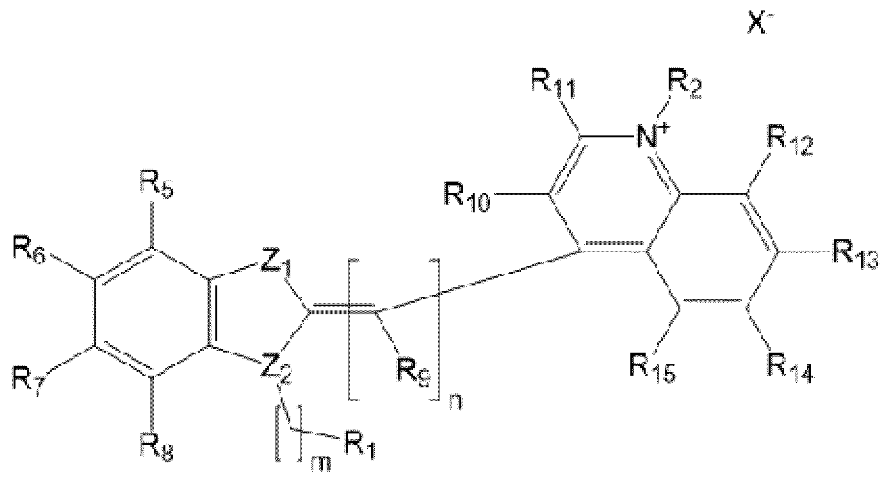

(I)

,

dans laquelle

- $Z_1$ est S et $Z_2$ est N ; et
- $R_1$ est choisi dans la liste comprenant 5-azidopentyle, 5-(4-(aminométhyl)-1H-1,2,3-triazol-1-yl)pentyle ou 5-ammoniopentyle ; et
- $R_2$ est l'éthyle et ;
- $R_5$ à $R_{15}$ sont chacun un atome d'hydrogène ; et
- $X^-$ est choisi dans la liste comprenant l'halogénate, le sulfate, le triflate, l'acétate trifluoré, l'acétate difluoré ou l'acétate fluoré ; et
- n est égal à 3 ; et
- m est égal à 1.

**2.** Colorant cyanine selon l'une des revendications précédentes, **caractérisé par le fait qu'**il se lie à l'DNA oligomérique ou à l'DNA-amorce ou à l'DNA-aptamère.

**3.** Colorant cyanine selon l'une des revendications précédentes sous sa forme protonée ou déprotonée, le contre-ion ou les contre-ions de la forme protonée ou déprotonée étant choisis indépendamment dans la liste comprenant l'halogénate, le sulfate, le triflate, l'acétate trifluoré, l'acétate difluoré ou l'acétate fluoré.

**4.** Méthode de coloration in vivo de micro-organismes à l'aide d'un colorant cyanine selon l'une des revendications précédentes, **caractérisée en ce que** la méthode comprend les étapes suivantes :

a) isolement des micro-organismes à colorer, cette étape d'isolement comprenant éventuellement une ou plusieurs étapes de concentration et/ou d'élevage des micro-organismes ;
b) coloration des micro-organismes à l'aide du colorant cyanine en ajoutant une solution de colorant cyanine aux micro-organismes, la viabilité des micro-organismes n'étant pas réduite.

**5.** Kit pour la mise en oeuvre de la méthode selon la revendication 4, le kit comprenant le colorant cyanine et éventuellement des substances de soutien.

Fig. 1

**A**

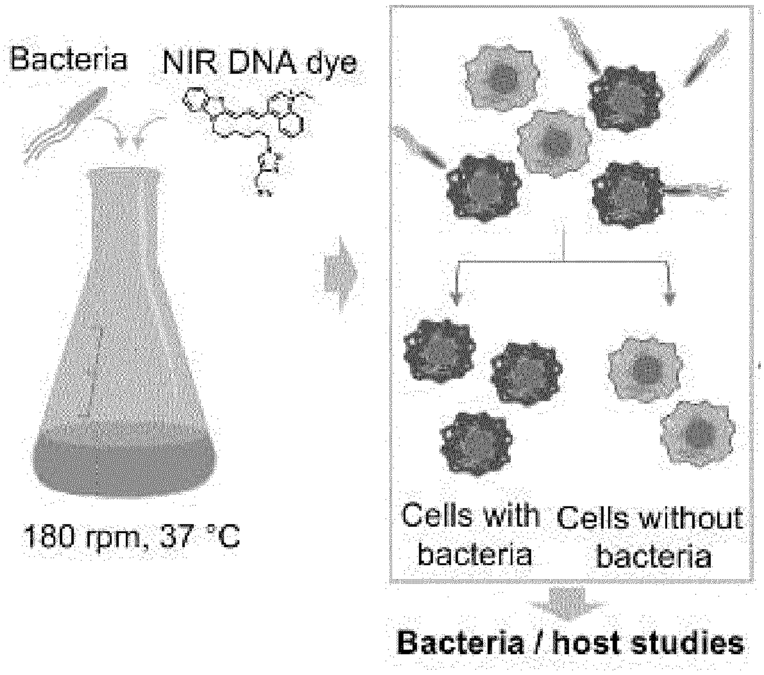


**B**

**6-AzTO-3**

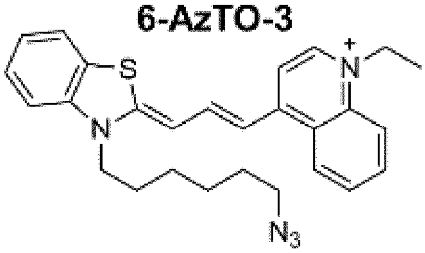

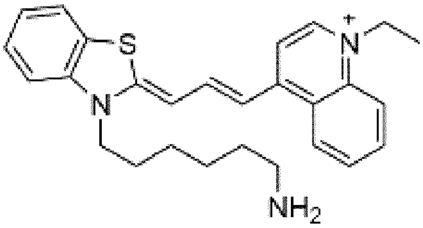

**6-NH$_2$TO-3**

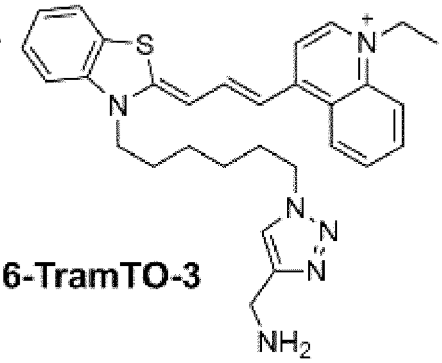

**6-TramTO-3**

Fig. 2A

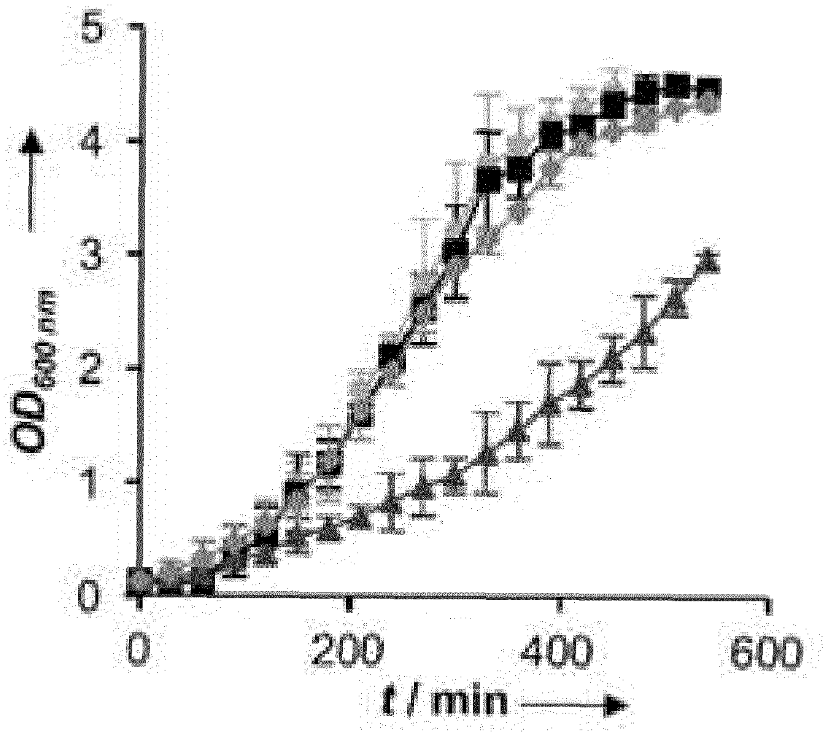
$OD_{600\,nm}$
5
4
3
2
1
0
0
200
400
600
t / min

Fig. 2B

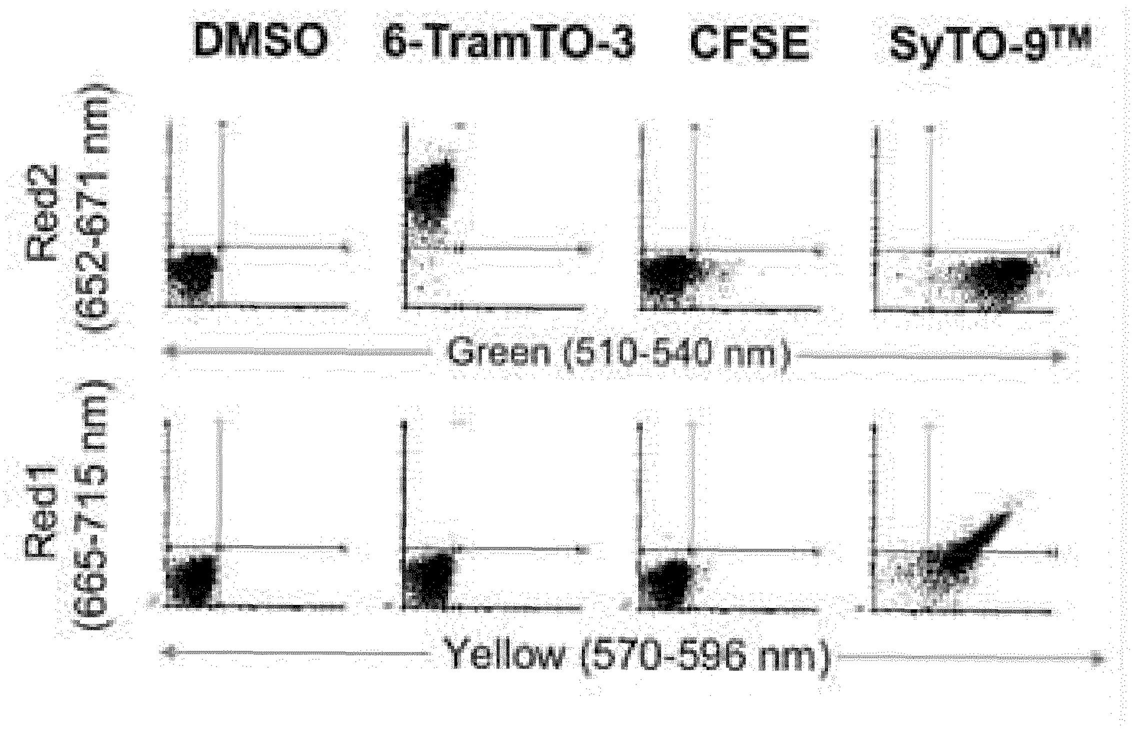
DMSO
6-TramTO-3
CFSE
SyTO-9™
Red2 (652-671 nm)
Green (510-540 nm)
Red1 (665-715 nm)
Yellow (570-596 nm)

Fig. 3A

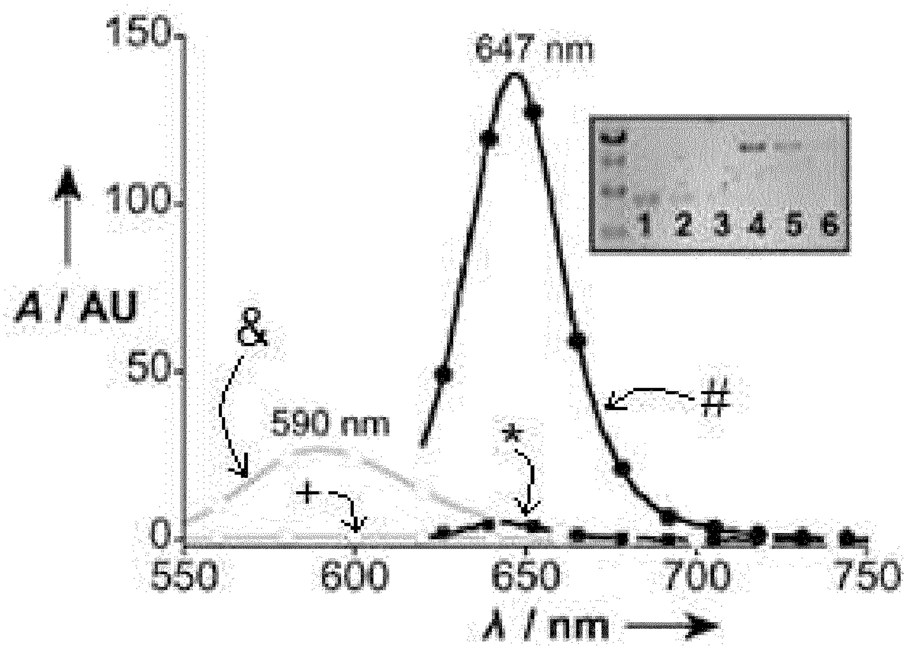
150
100
50
0
A / AU
647 nm
590 nm
&
+
*
#
1 2 3 4 5 6
550
600
650
700
750
λ / nm

Fig. 3B

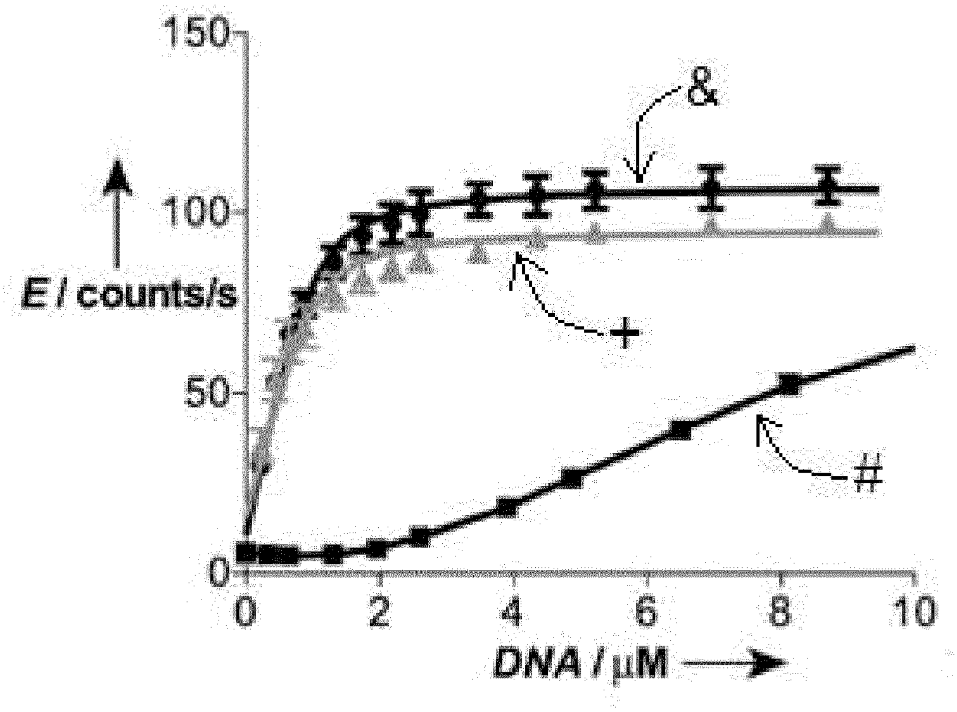
150
100
50
0
E / counts/s
&
+
#
0
2
4
6
8
10
DNA / µM

Fig. 4A

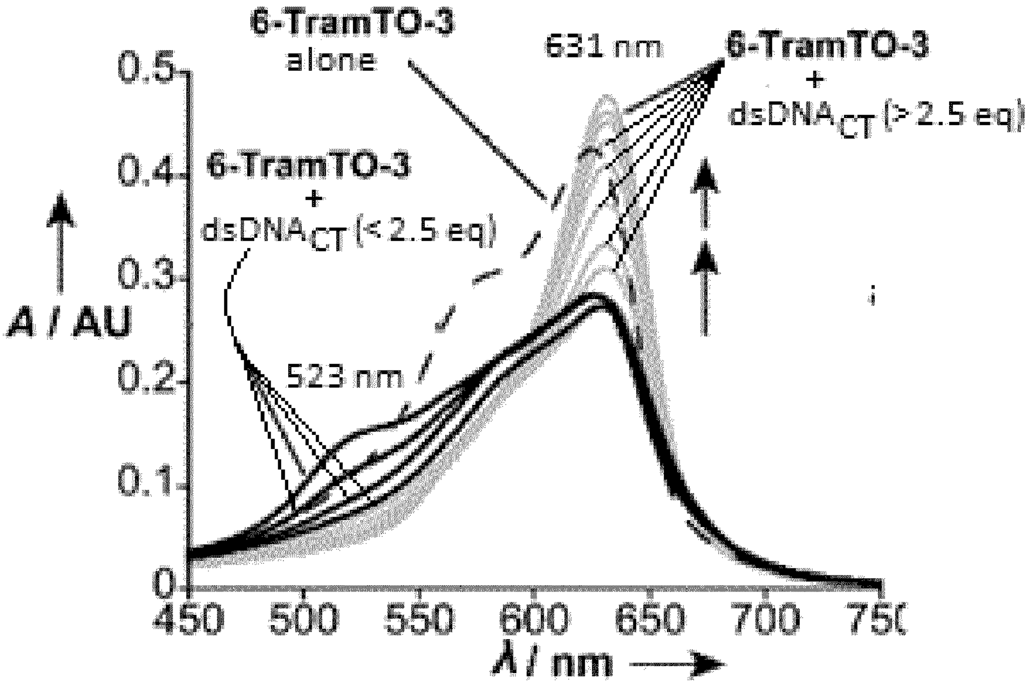
6-TramTO-3
alone
631 nm
6-TramTO-3
+
dsDNACT (> 2.5 eq)
6-TramTO-3
+
dsDNACT (< 2.5 eq)
523 nm
A / AU
0.5
0.4
0.3
0.2
0.1
0
450
500
550
600
650
700
750
λ / nm

Fig. 4B

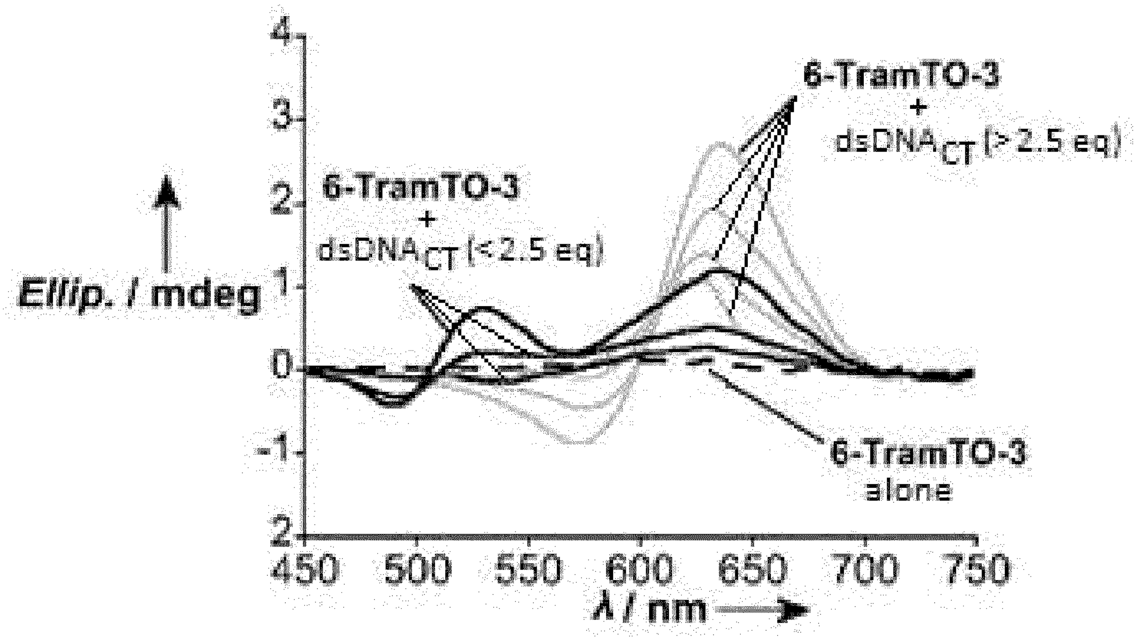
6-TramTO-3
+
dsDNACT (> 2.5 eq)
6-TramTO-3
+
dsDNACT (< 2.5 eq)
Ellip. / mdeg
6-TramTO-3
alone
4
3
2
1
0
-1
2
450
500
550
600
650
700
750
λ / nm

Fig. 5

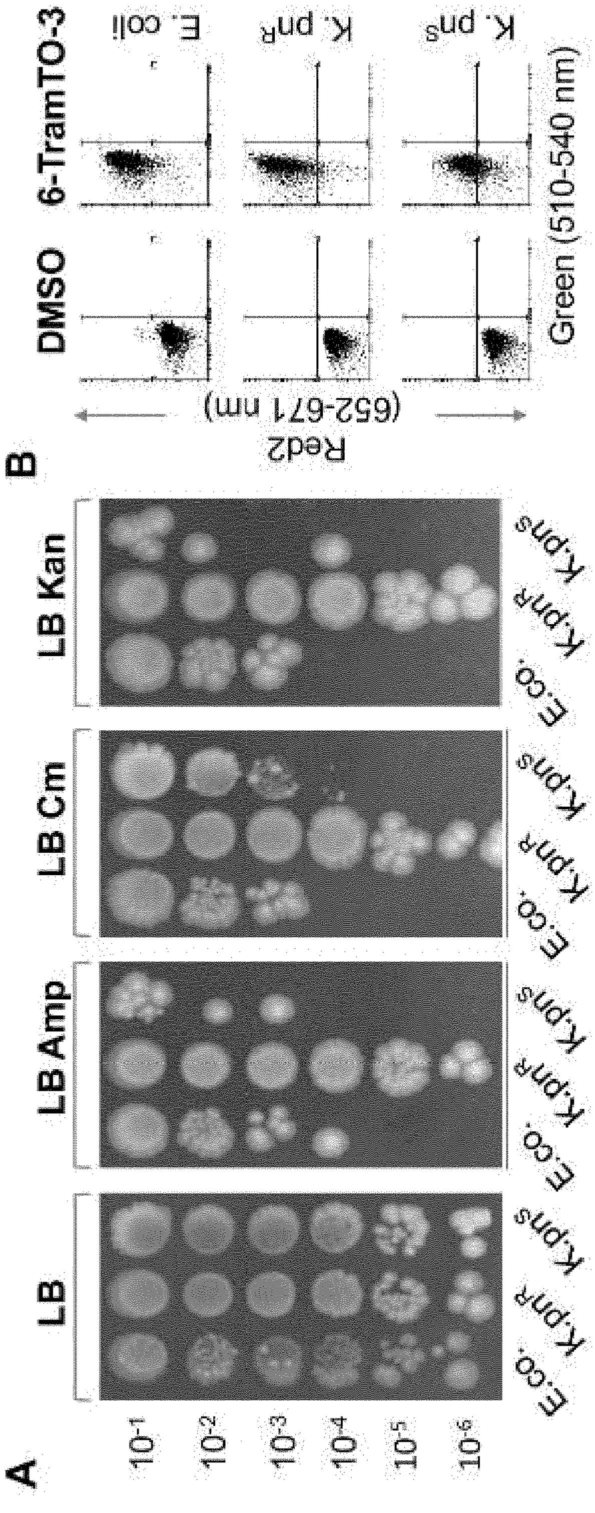
A
B
LB
LB Amp
LB Cm
LB Kan
10-1
10-2
10-3
10-4
10-5
10-6
E.co.
K.pnR
K.pnS
DMSO
6-TramTO-3
E. coli
K. pnR
K. pnS
Red2 (652-671 nm)
Green (510-540 nm)

Fig. 6

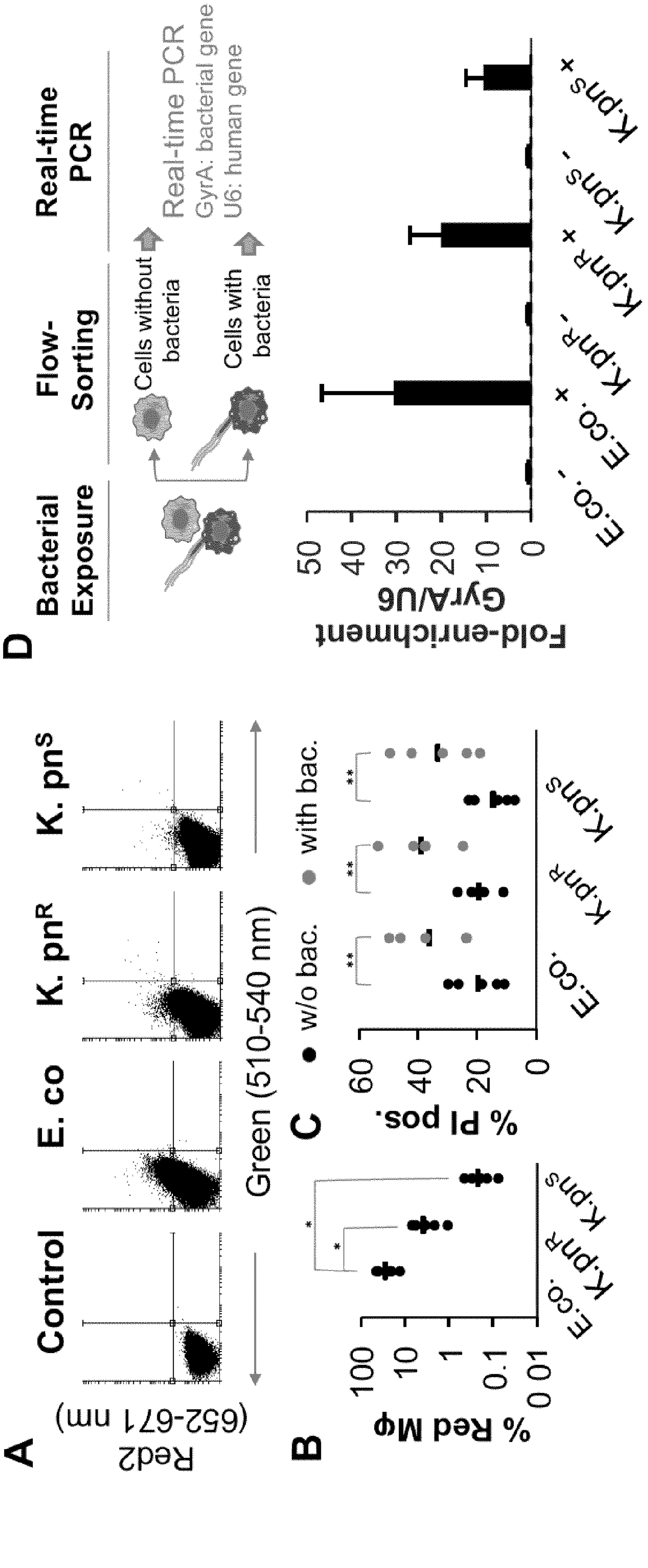
A
Red2 (652-671 nm)
Control
E. co
K. pnR
K. pnS
Green (510-540 nm)
B
% Red Mφ
100
10
1
0.1
0.01
E.co.
K.pnR
K.pnS
C
% PI pos.
w/o bac.
with bac.
60
40
20
0
D
Bacterial Exposure
Flow-Sorting
Cells without bacteria
Cells with bacteria
Real-time PCR
Real-time PCR
GyrA: bacterial gene
U6: human gene
Fold-enrichment GyrA/U6
50
40
30
20
10
0
E.co. -
E.co. +
K.pnR -
K.pnR +
K.pnS -
K.pnS +

Fig. 7

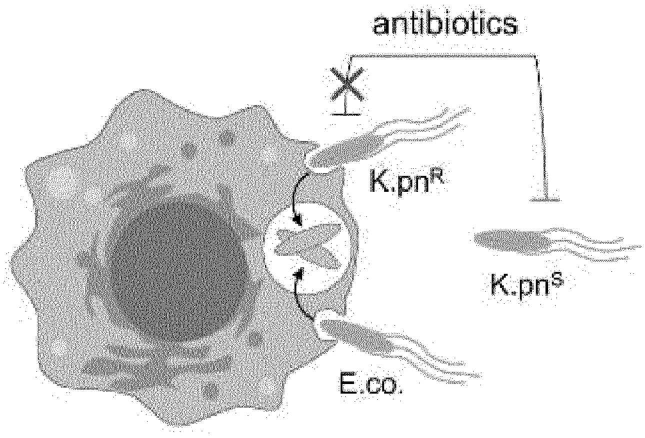

Fig. 8A

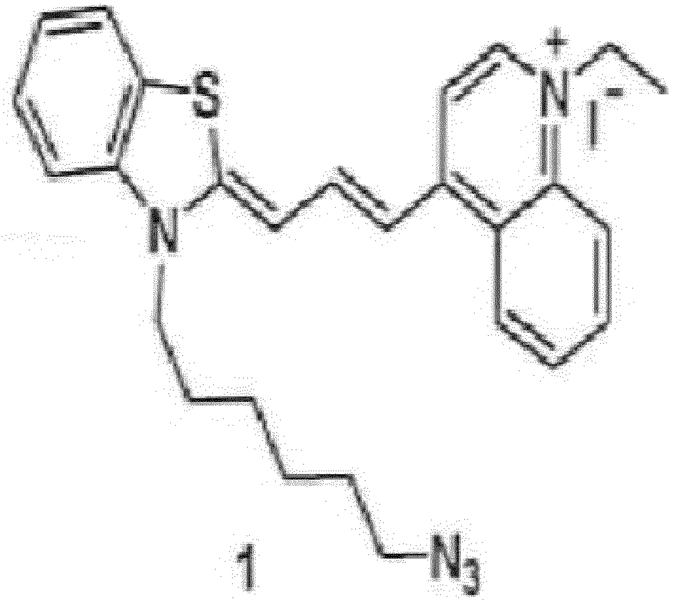

1

6-AzTO-3

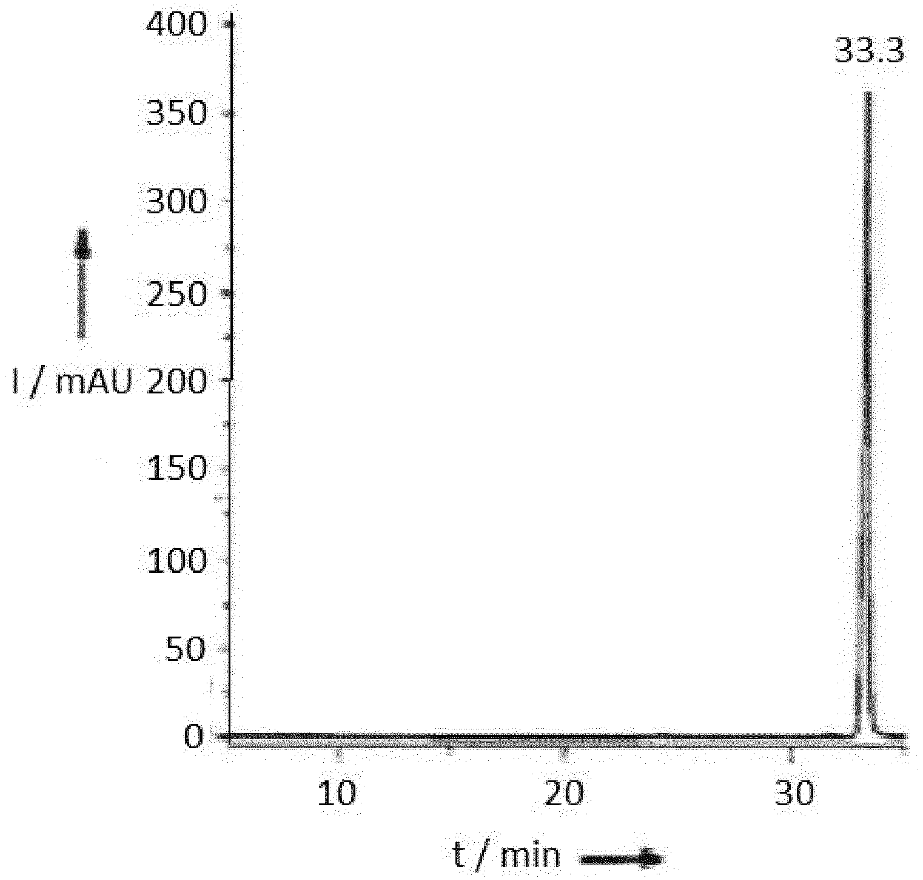

Fig. 8B

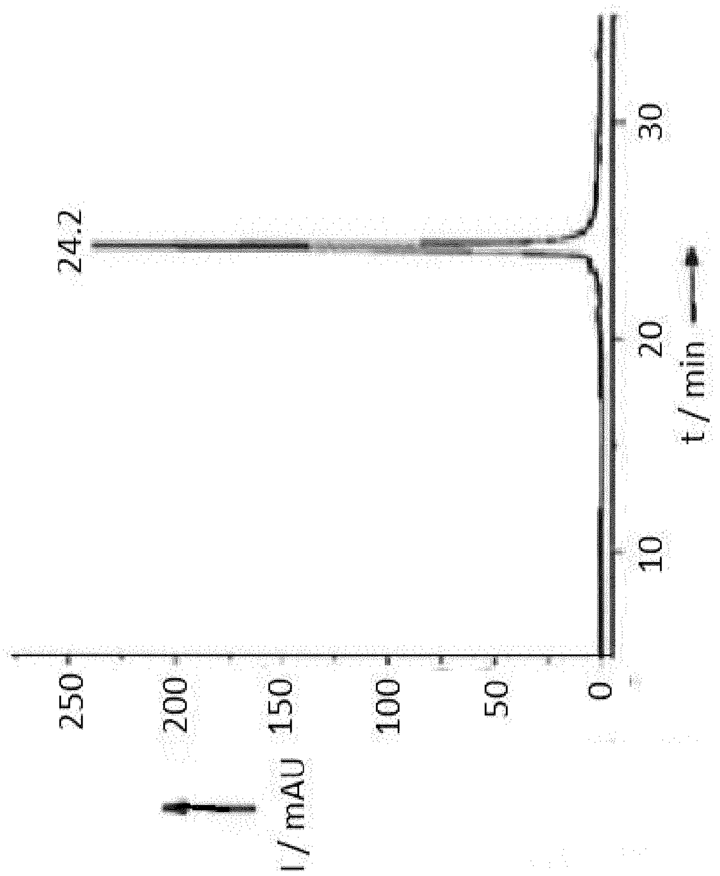


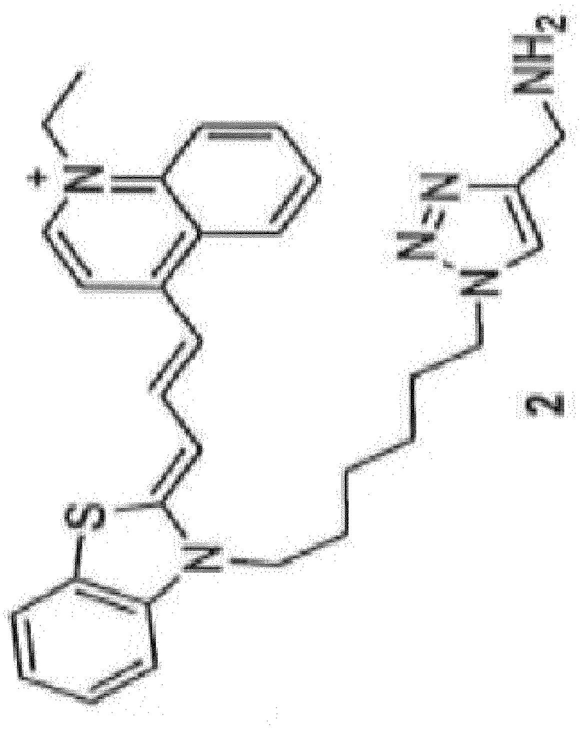

2

6-TramTO-3

Fig. 8C

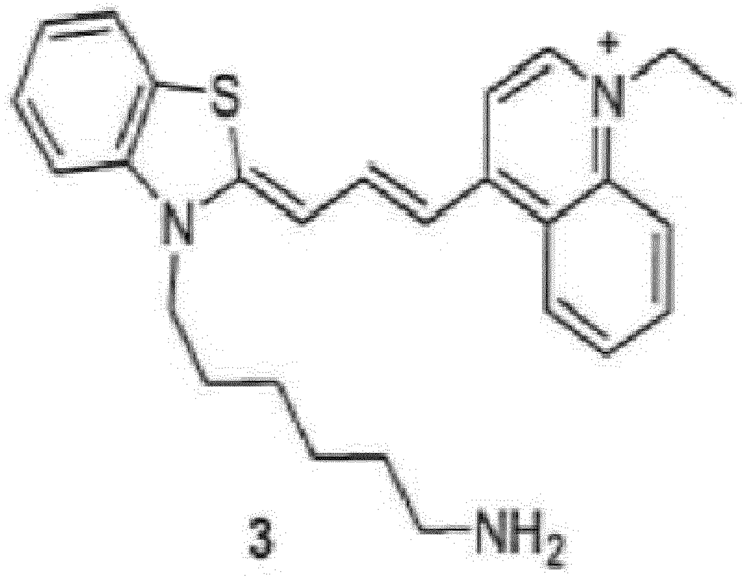

3
6-$NH_2$TO-3

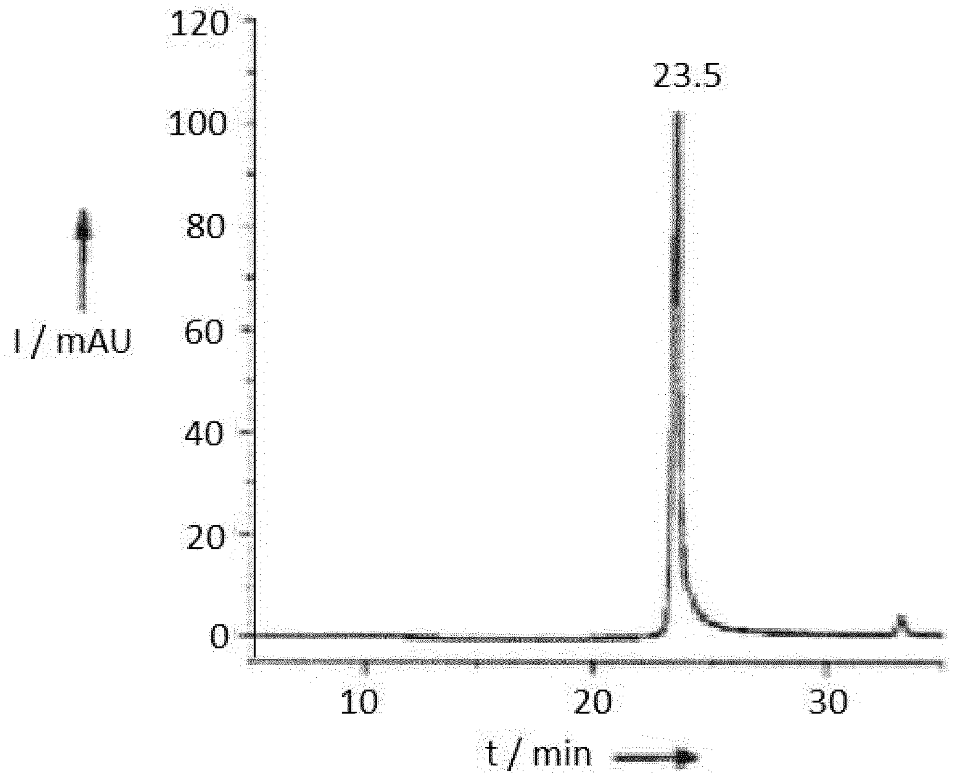

Fig. 9A

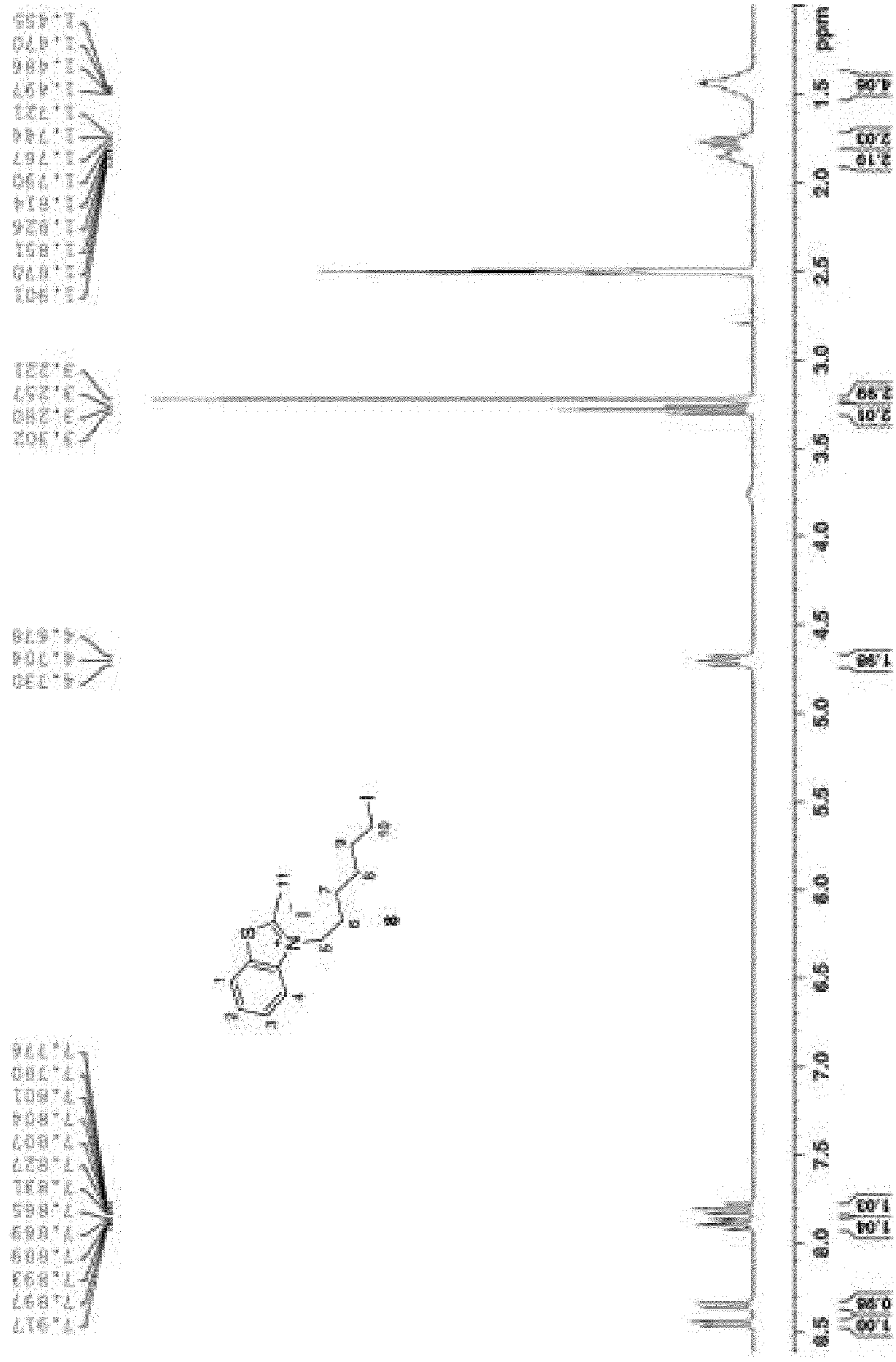

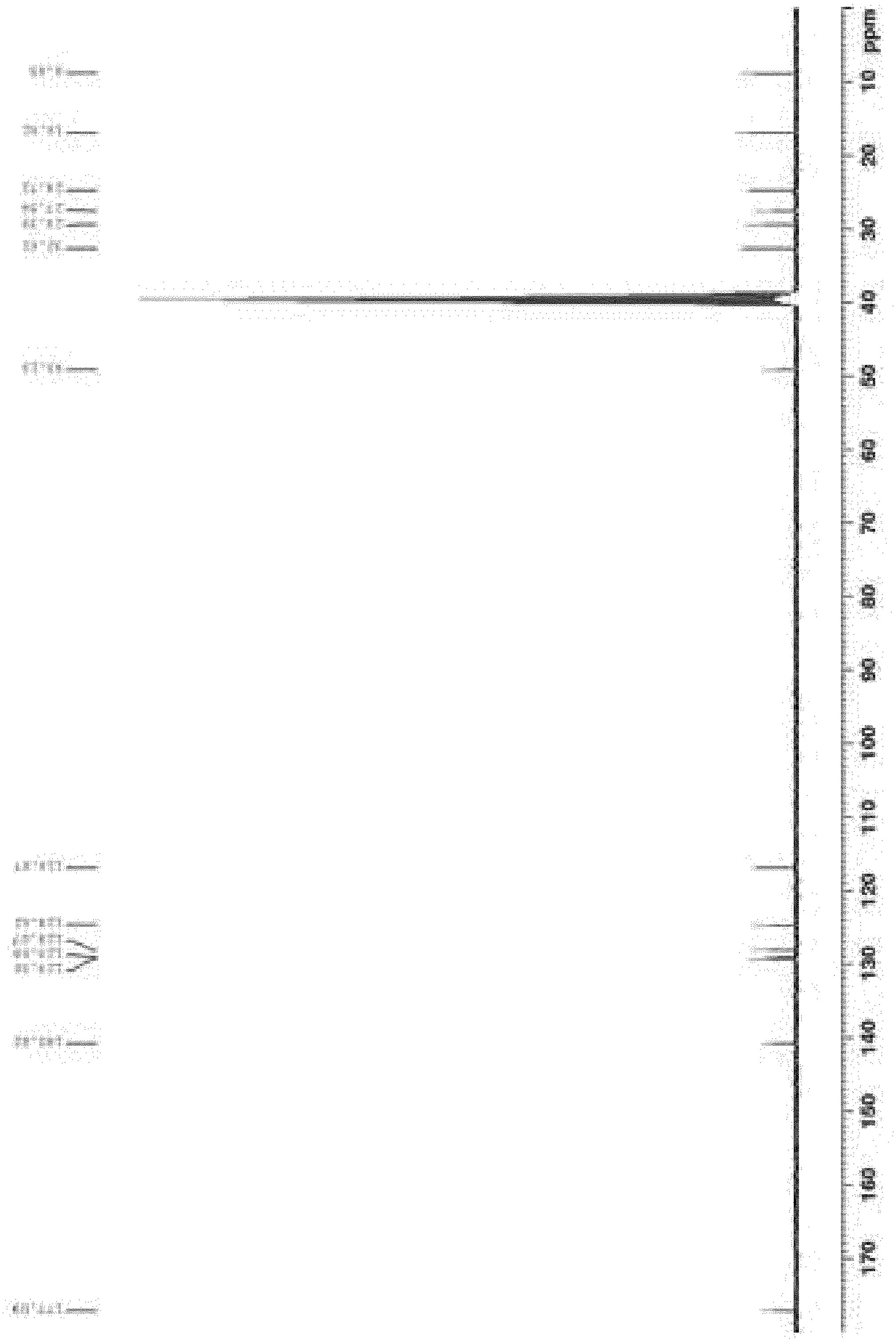


Fig. 9A (cont.)

Fig. 9B

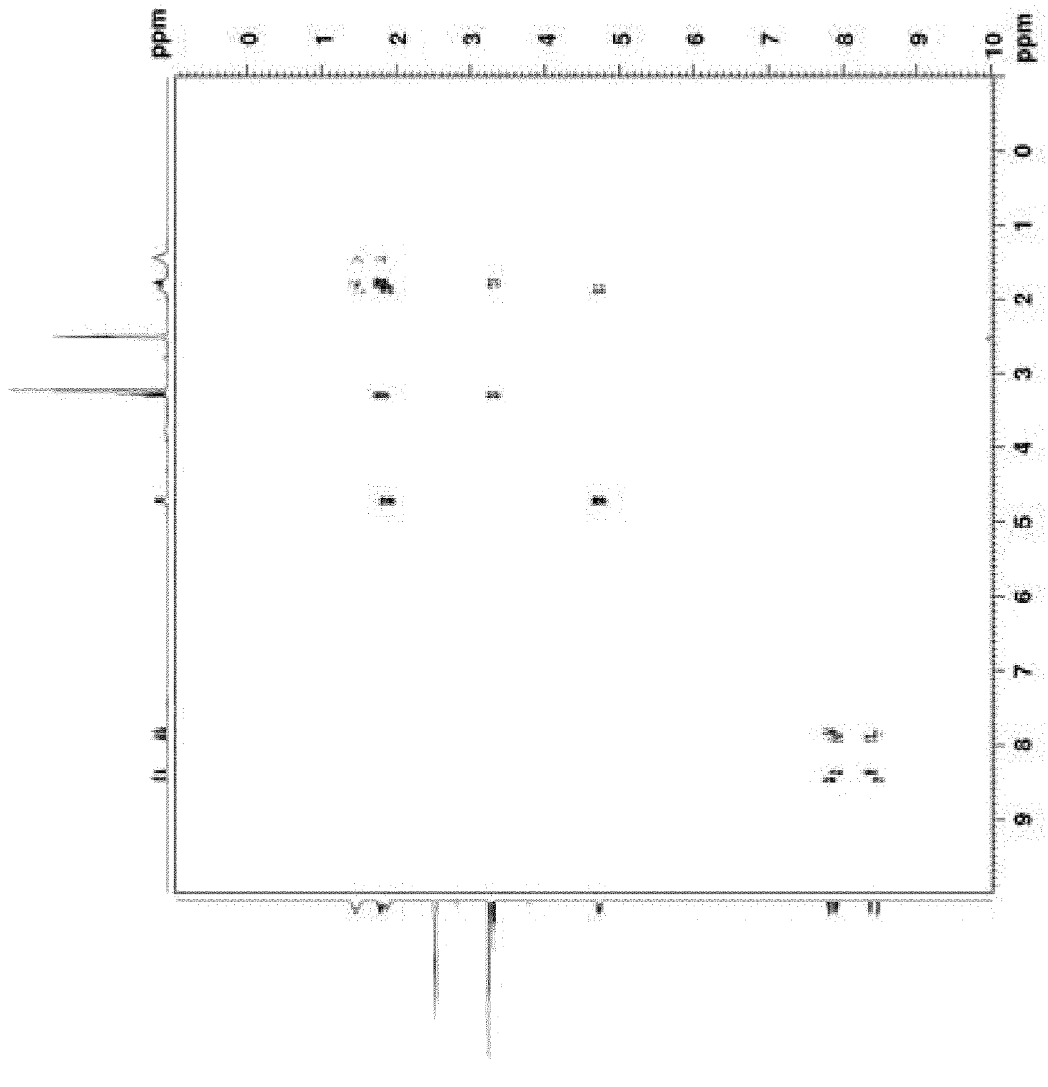

Fig. 9C

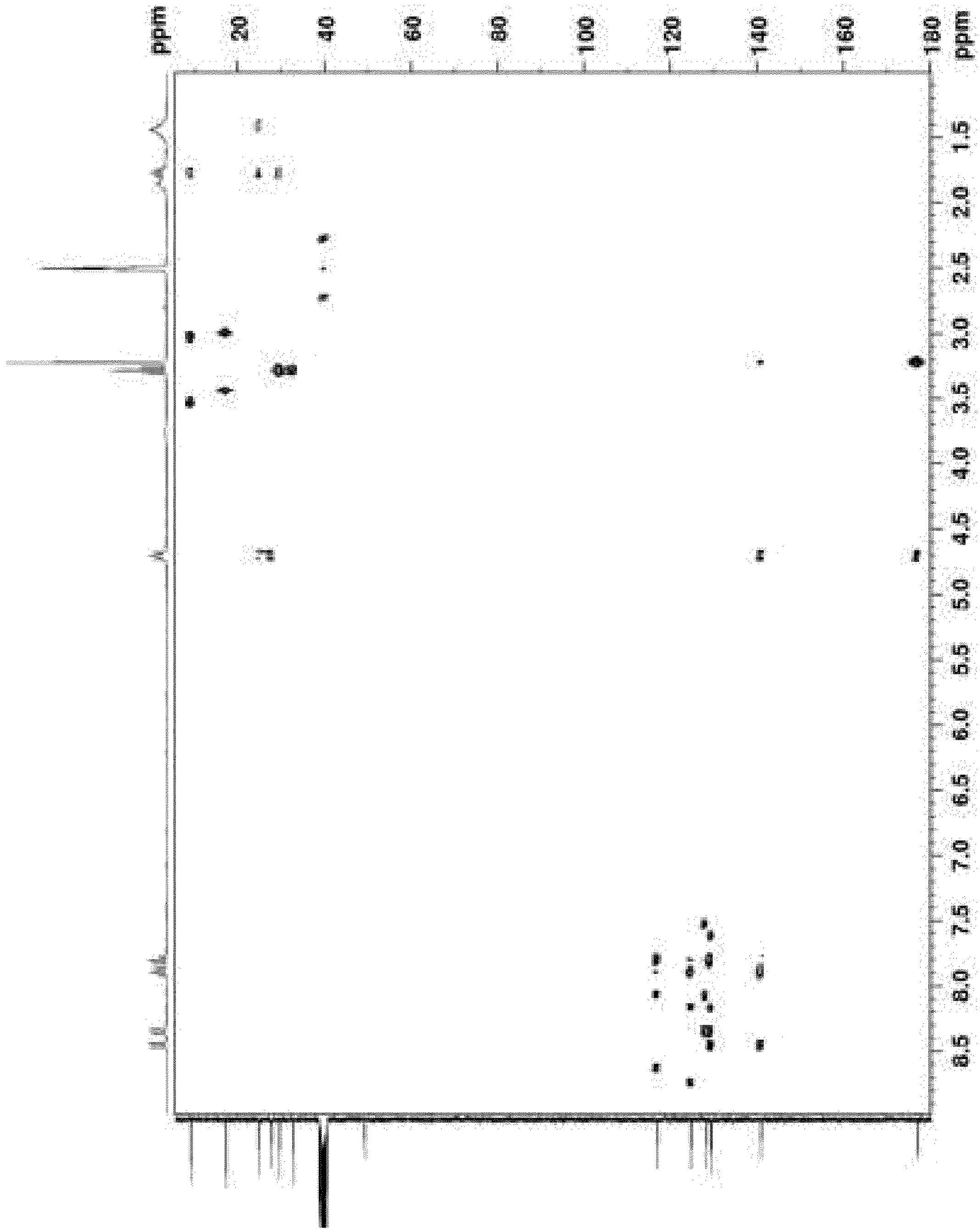
ppm
20
40
60
80
100
120
140
160
180
ppm
1.5
2.0
2.5
3.0
3.5
4.0
4.5
5.0
5.5
6.0
6.5
7.0
7.5
8.0
8.5

Fig. 10A

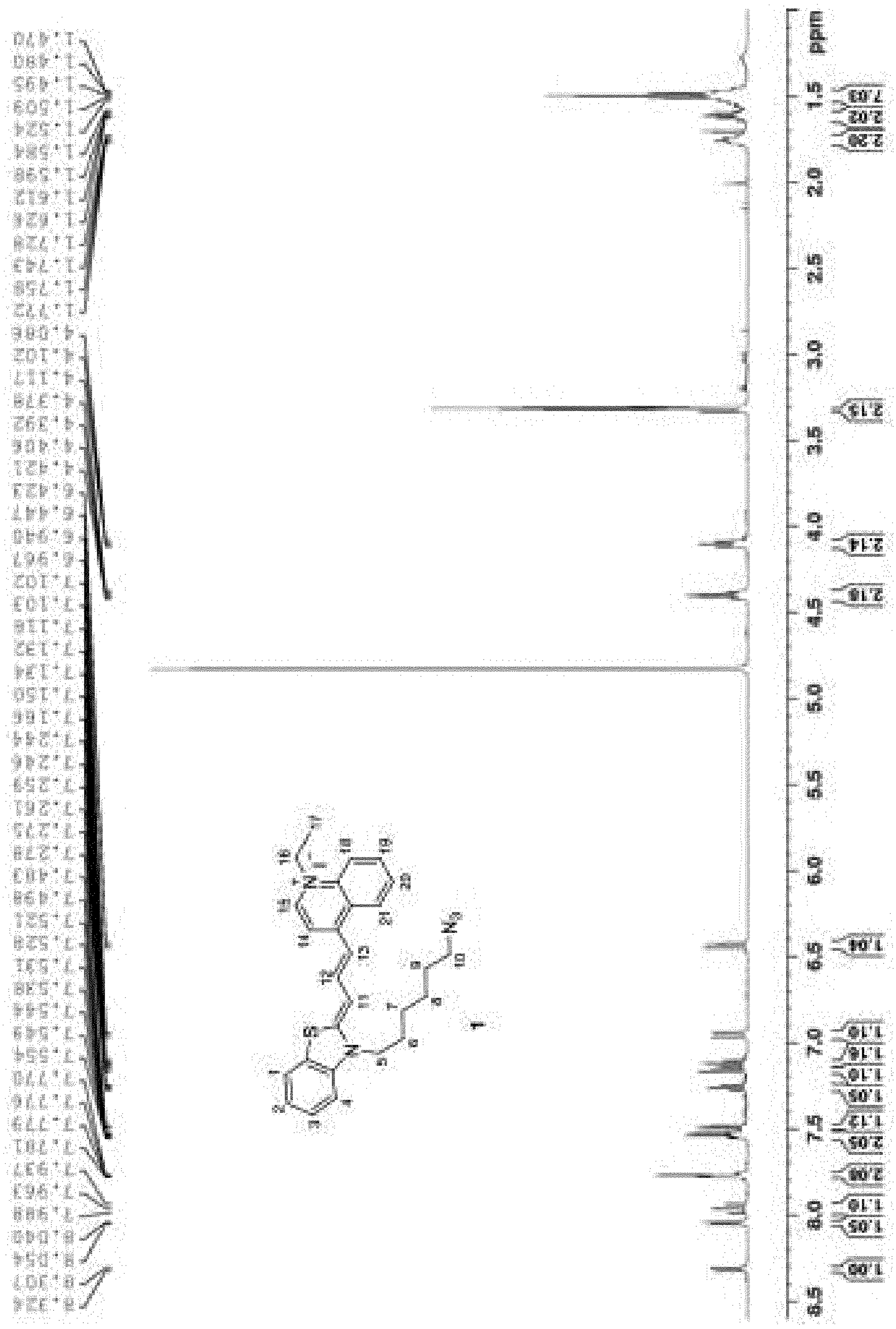

Fig. 10A (cont.)

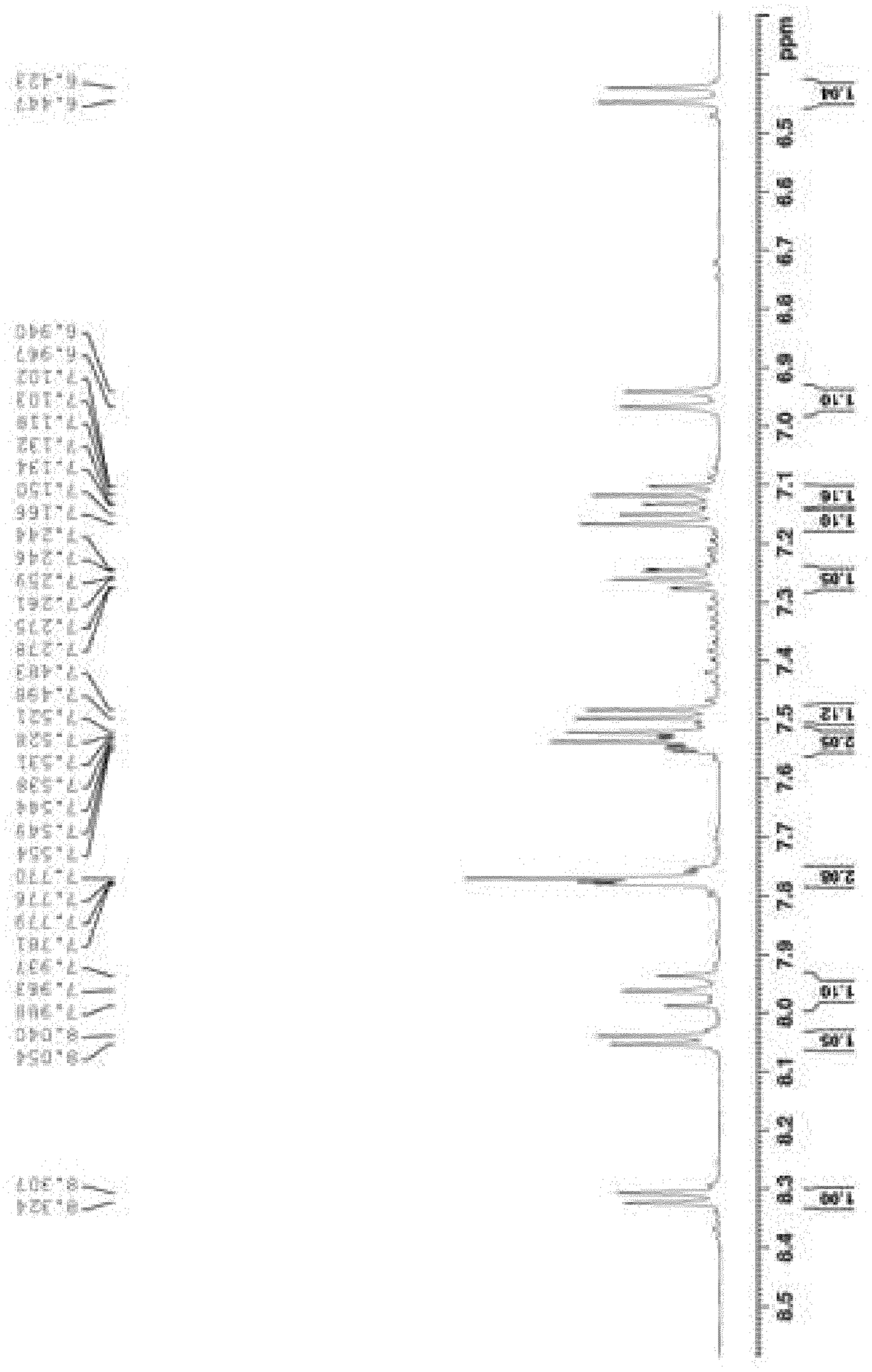

Fig. 10B

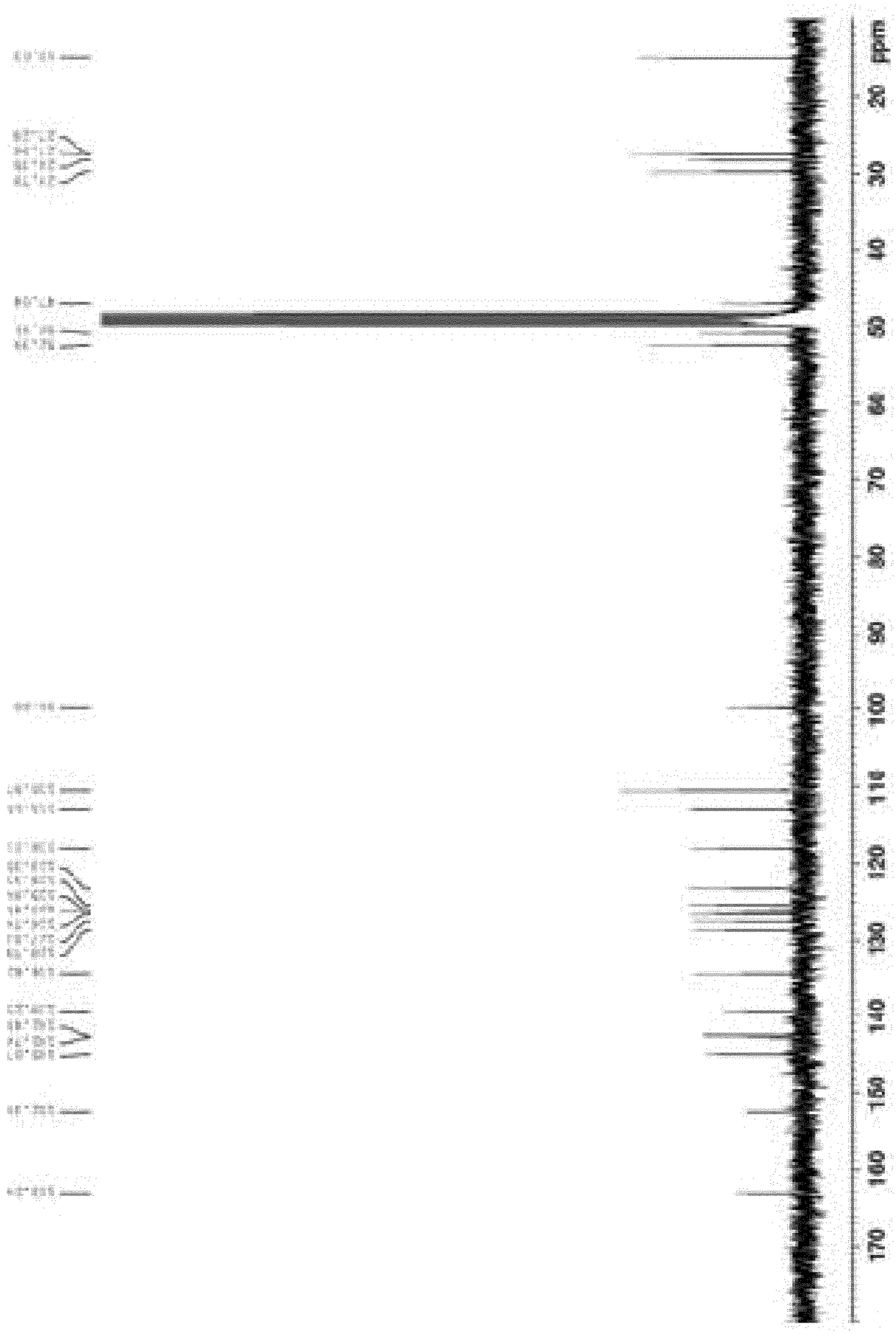

Fig. 10C

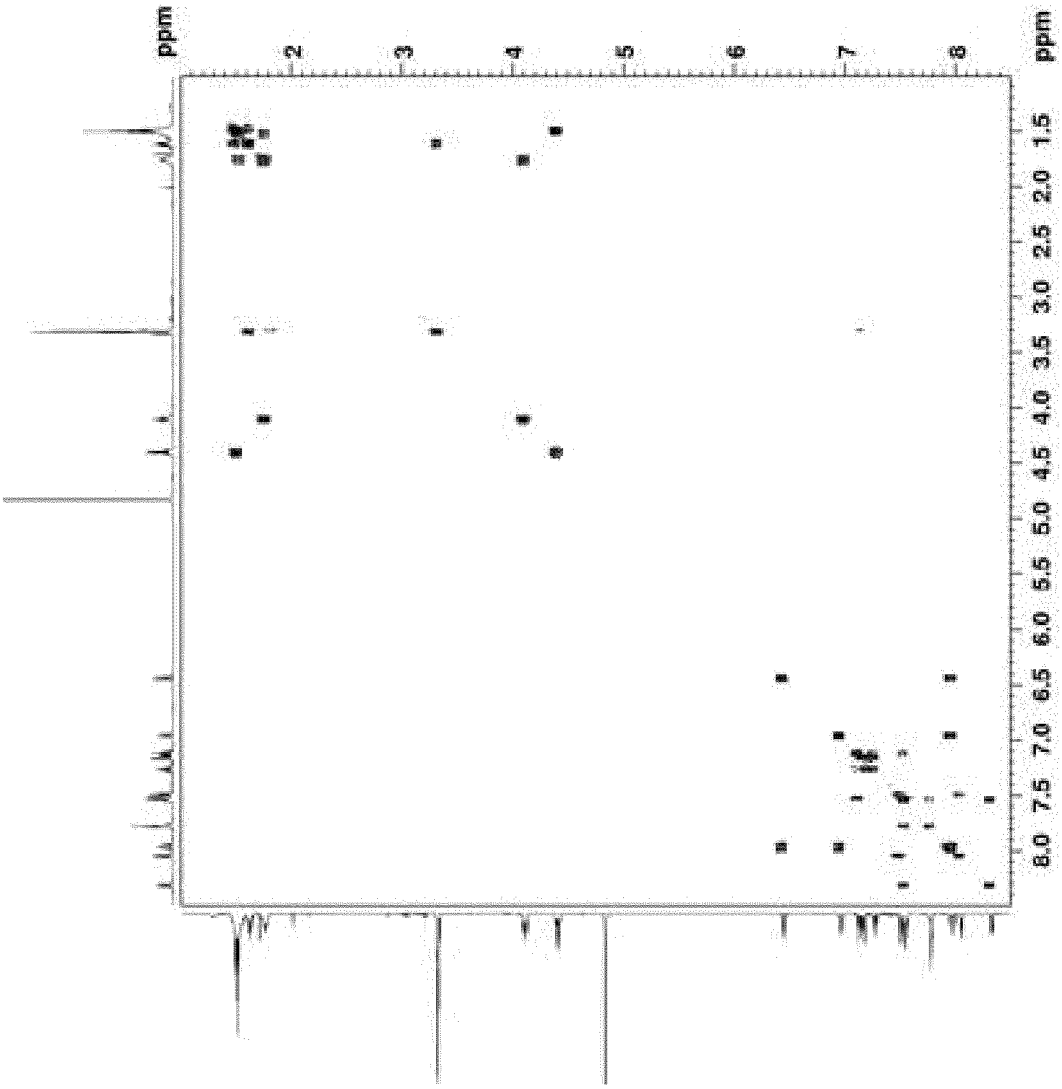

Fig. 10D

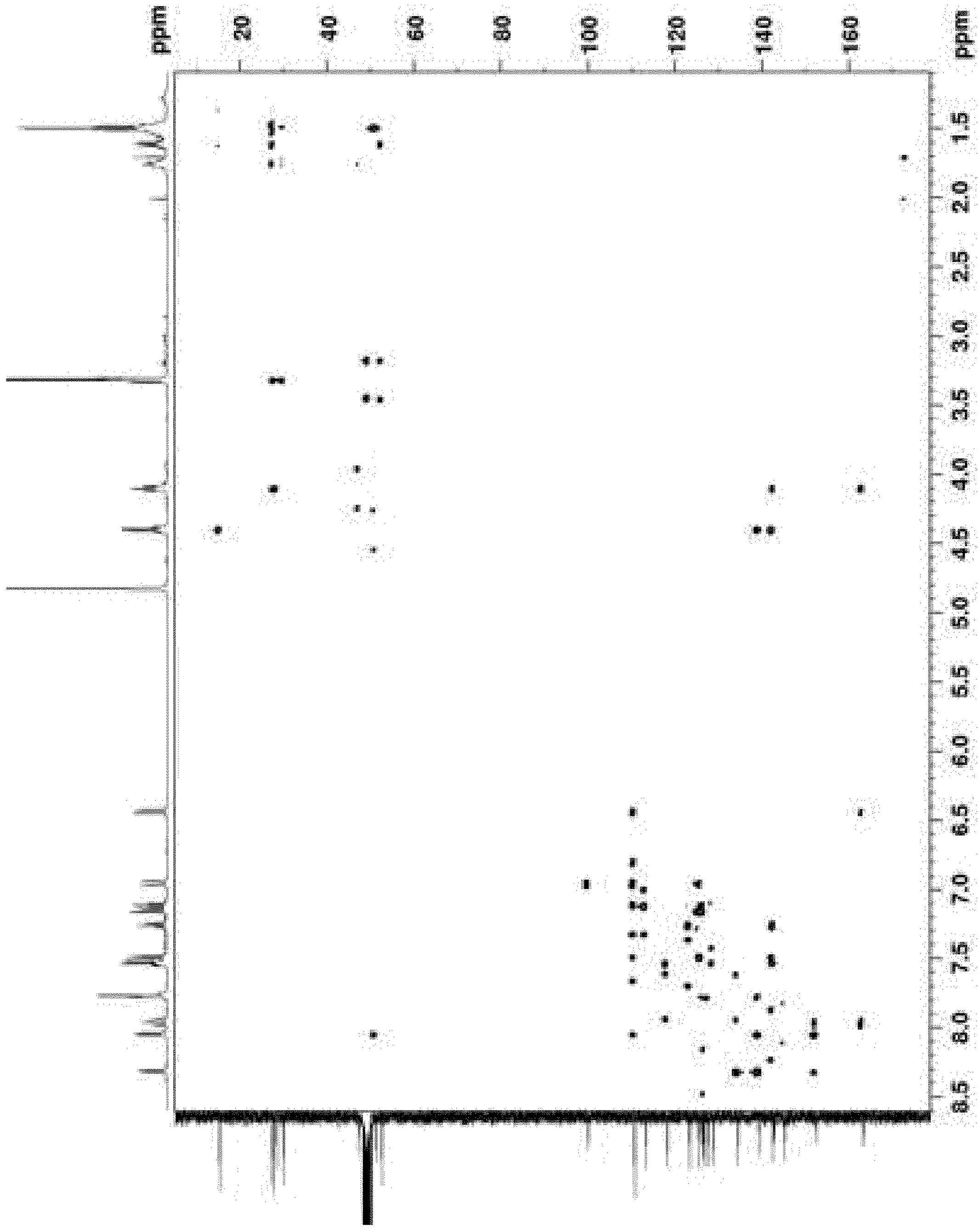
ppm
20
40
60
80
100
120
140
160
ppm
1.5
2.0
2.5
3.0
3.5
4.0
4.5
5.0
5.5
6.0
6.5
7.0
7.5
8.0
8.5

Fig. 11A

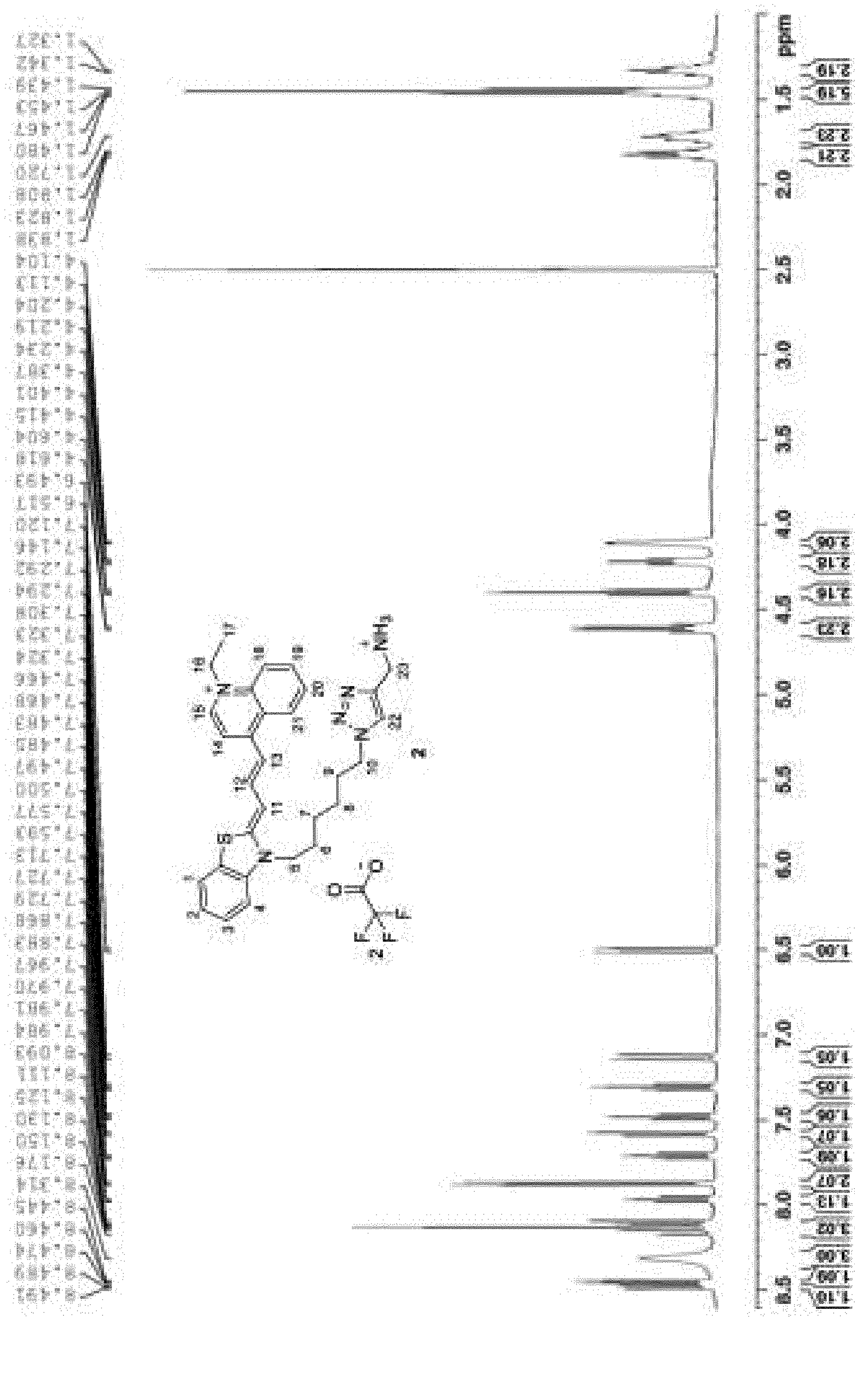

Fig. 11A (cont.)

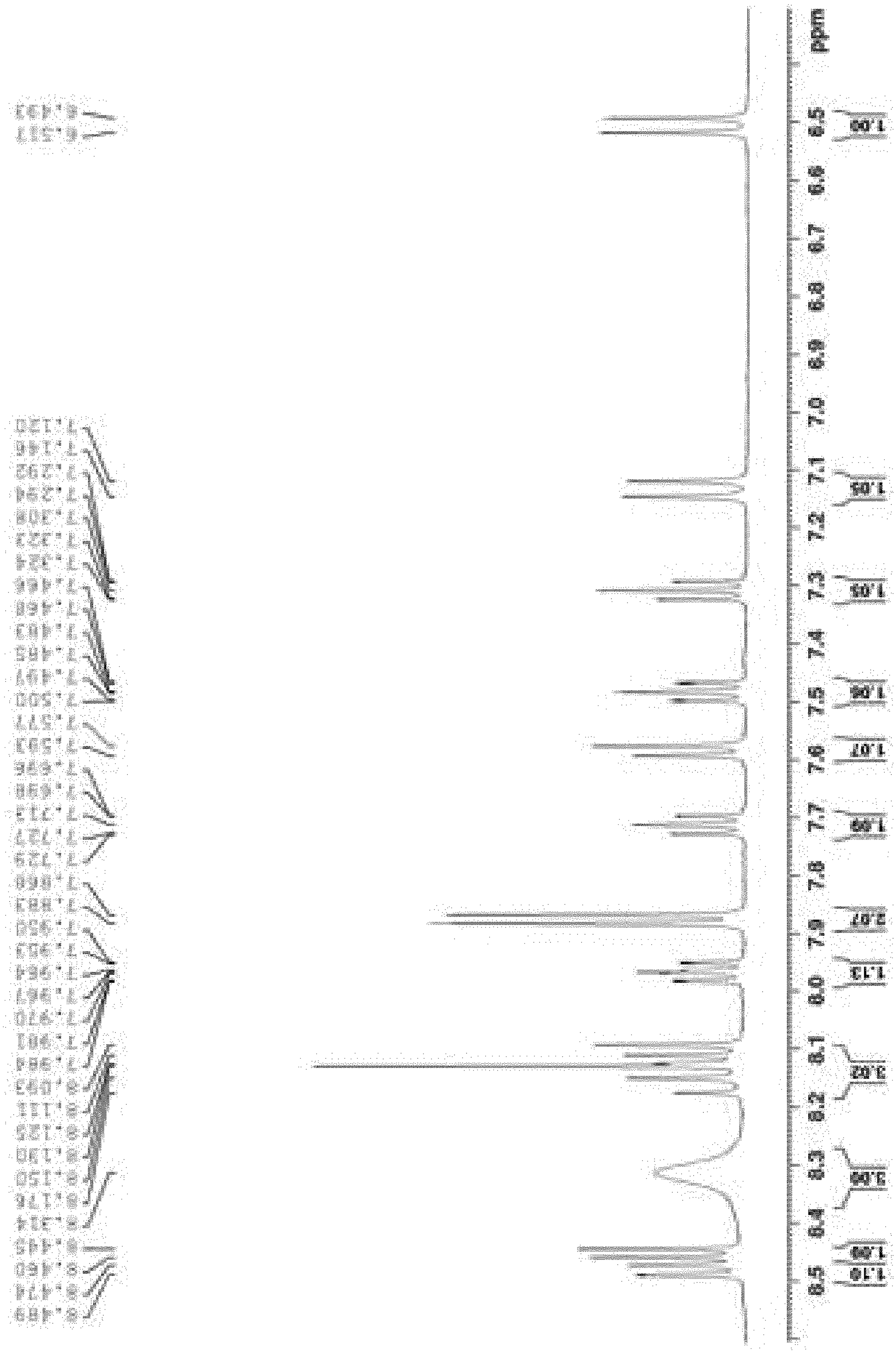

Fig. 11B

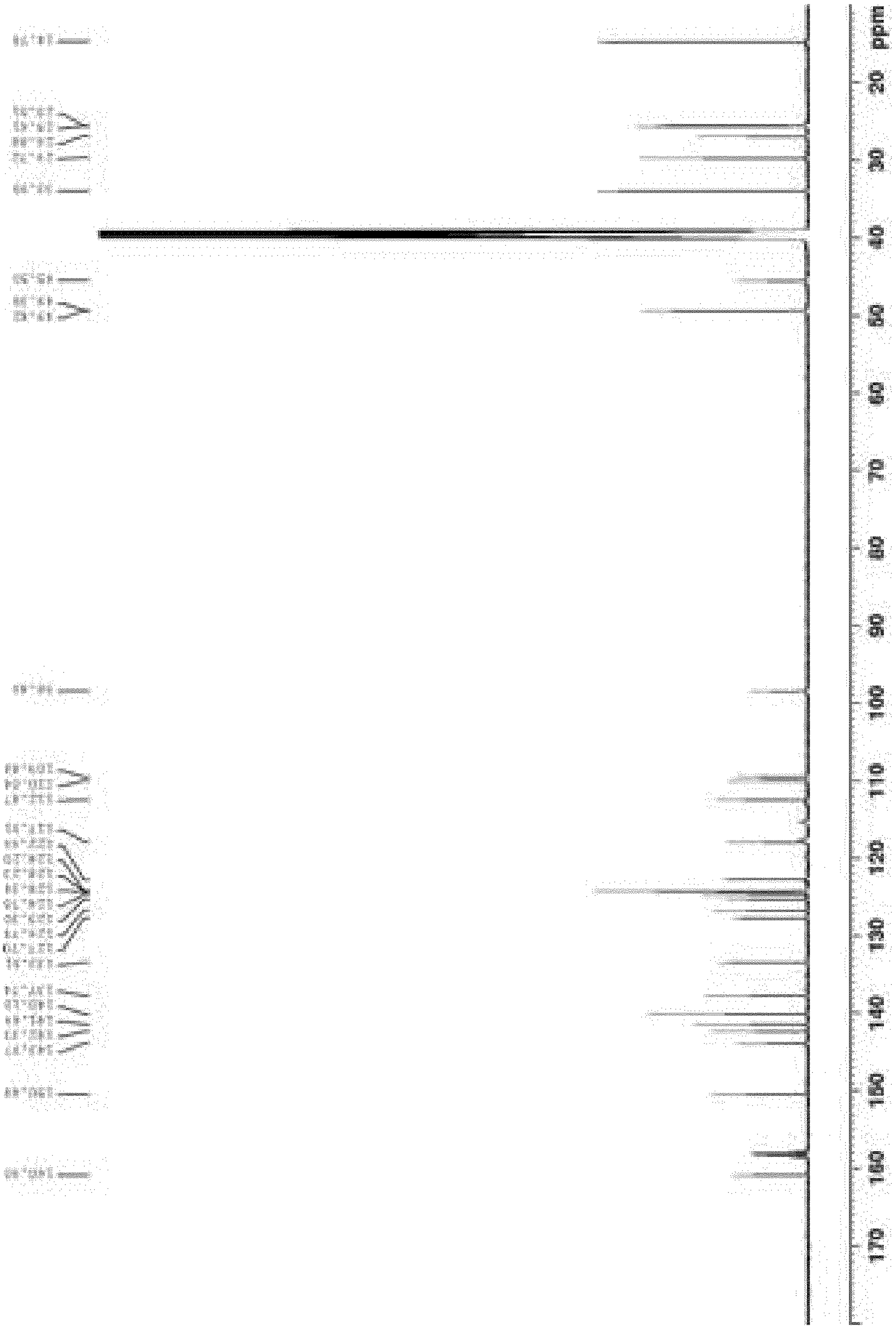

Fig. 11C

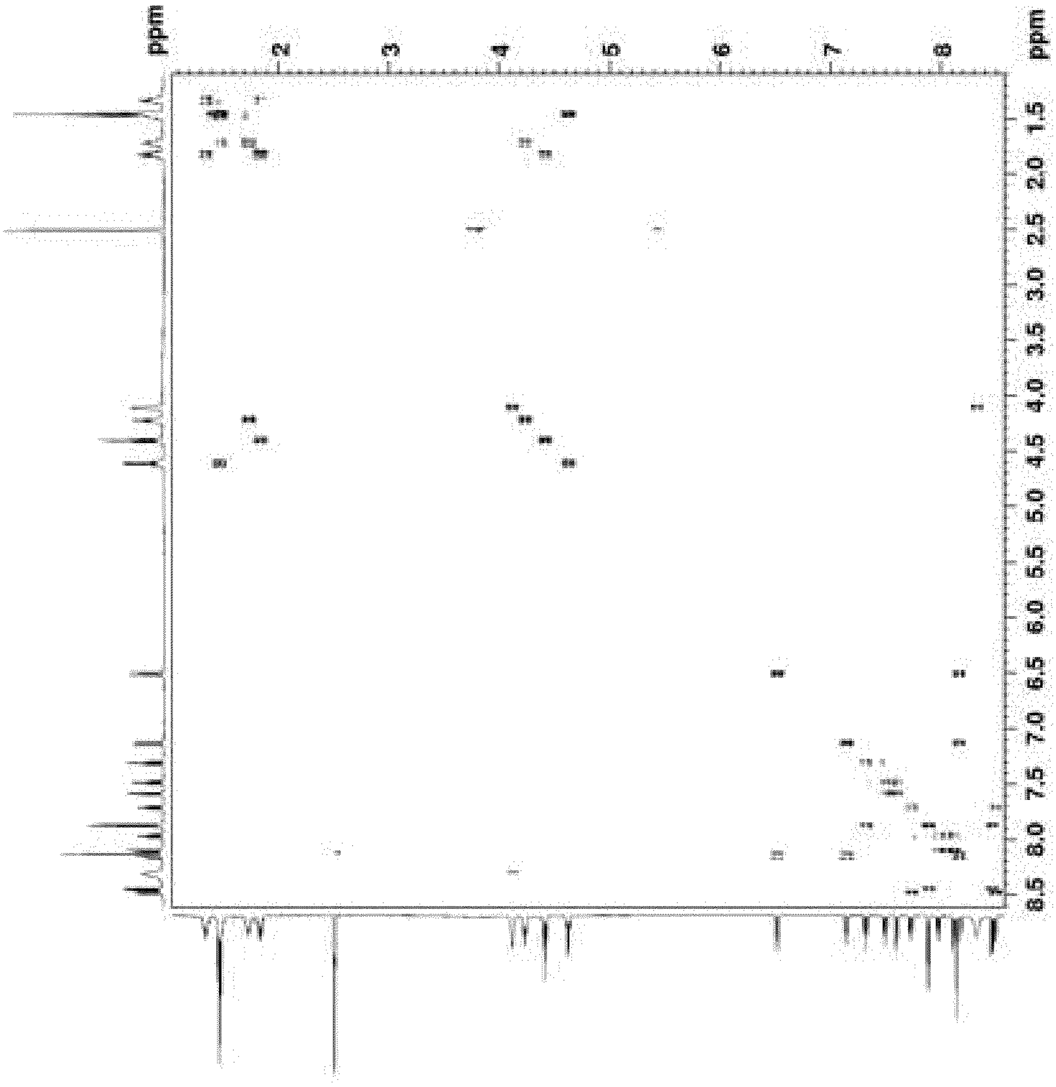

Fig. 11D

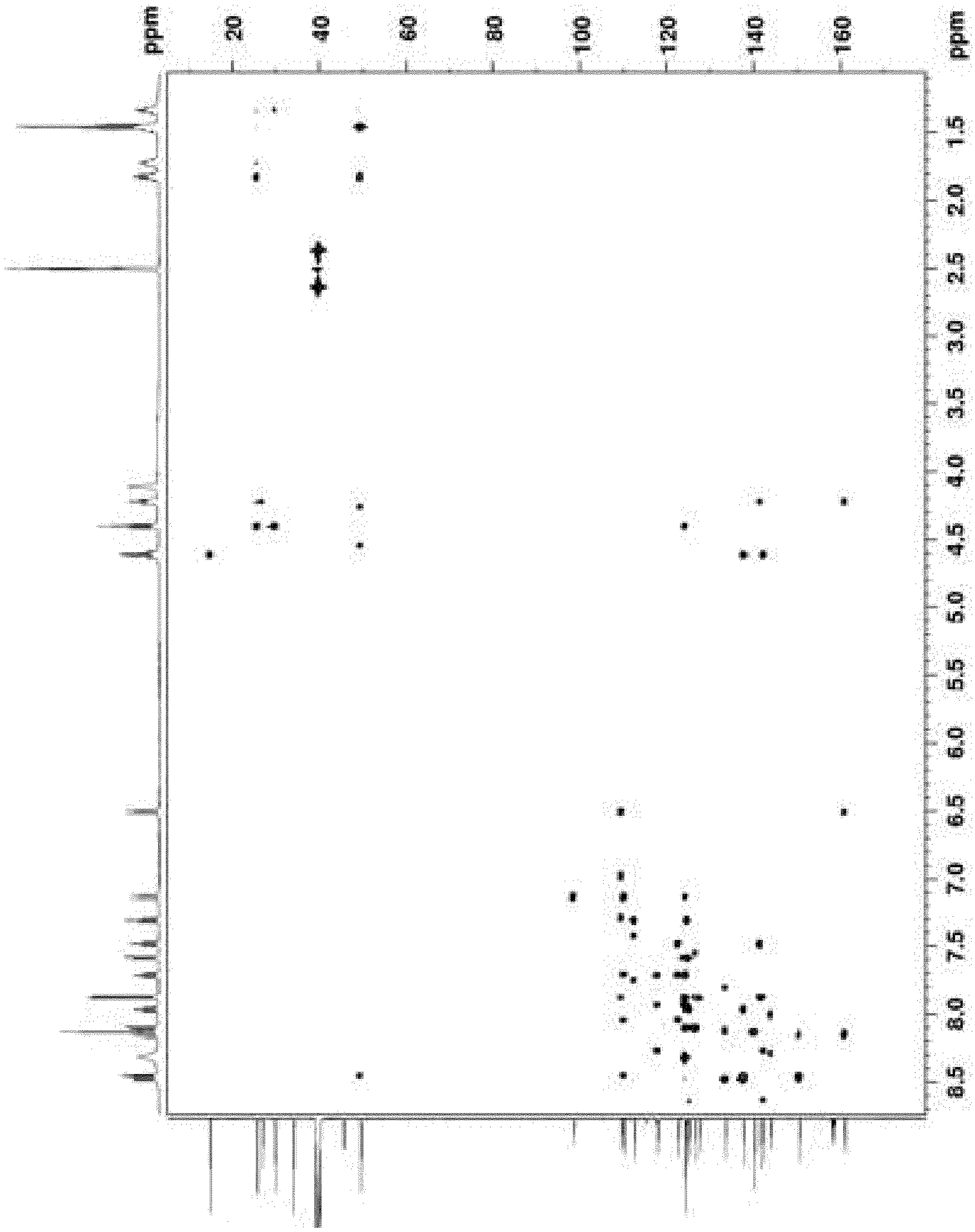
ppm
20
40
60
80
100
120
140
160
ppm
1.5
2.0
2.5
3.0
3.5
4.0
4.5
5.0
5.5
6.0
6.5
7.0
7.5
8.0
8.5

Fig. 12A

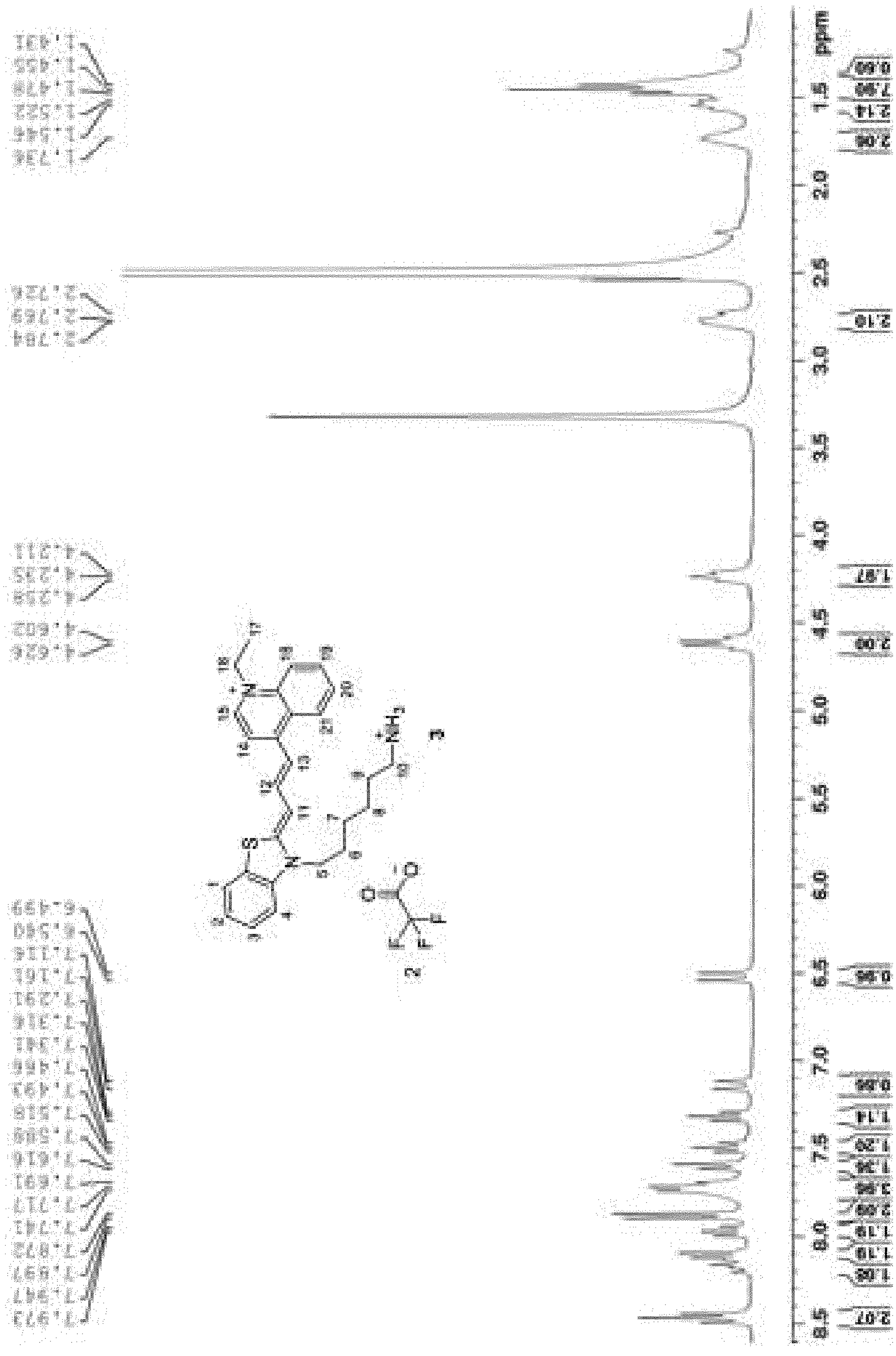

Fig. 12A (cont.)

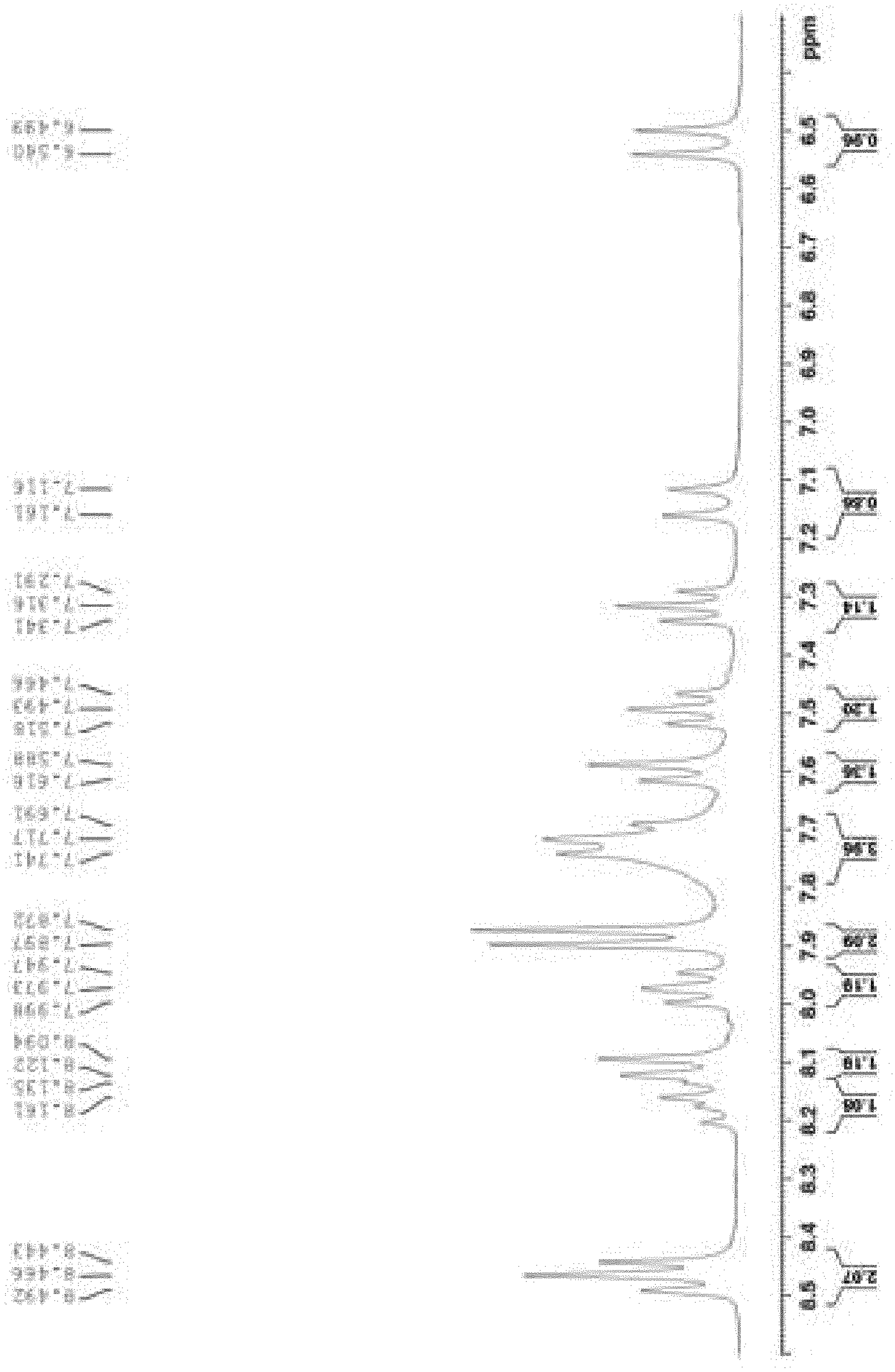

Fig. 12B

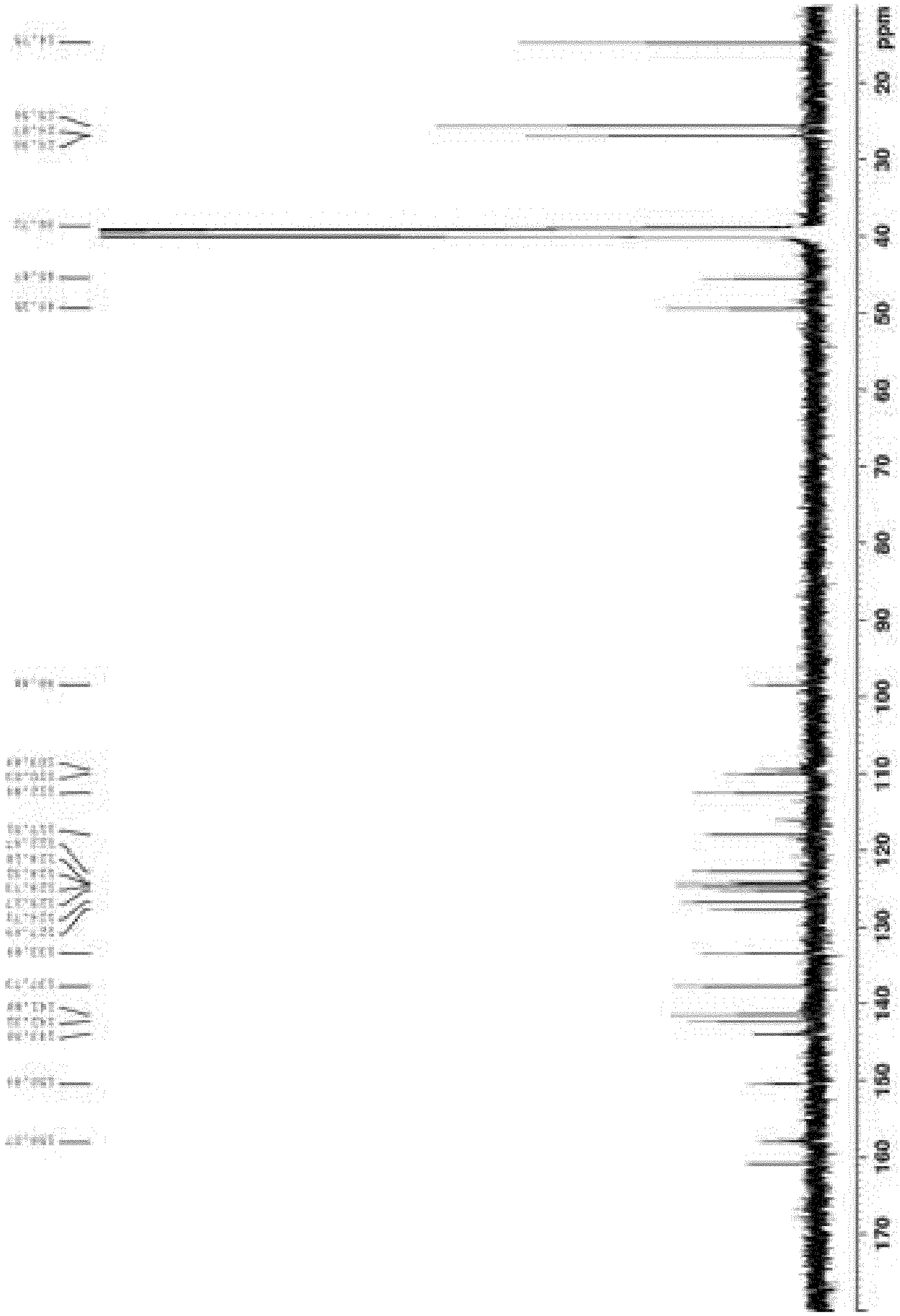

Fig. 12C

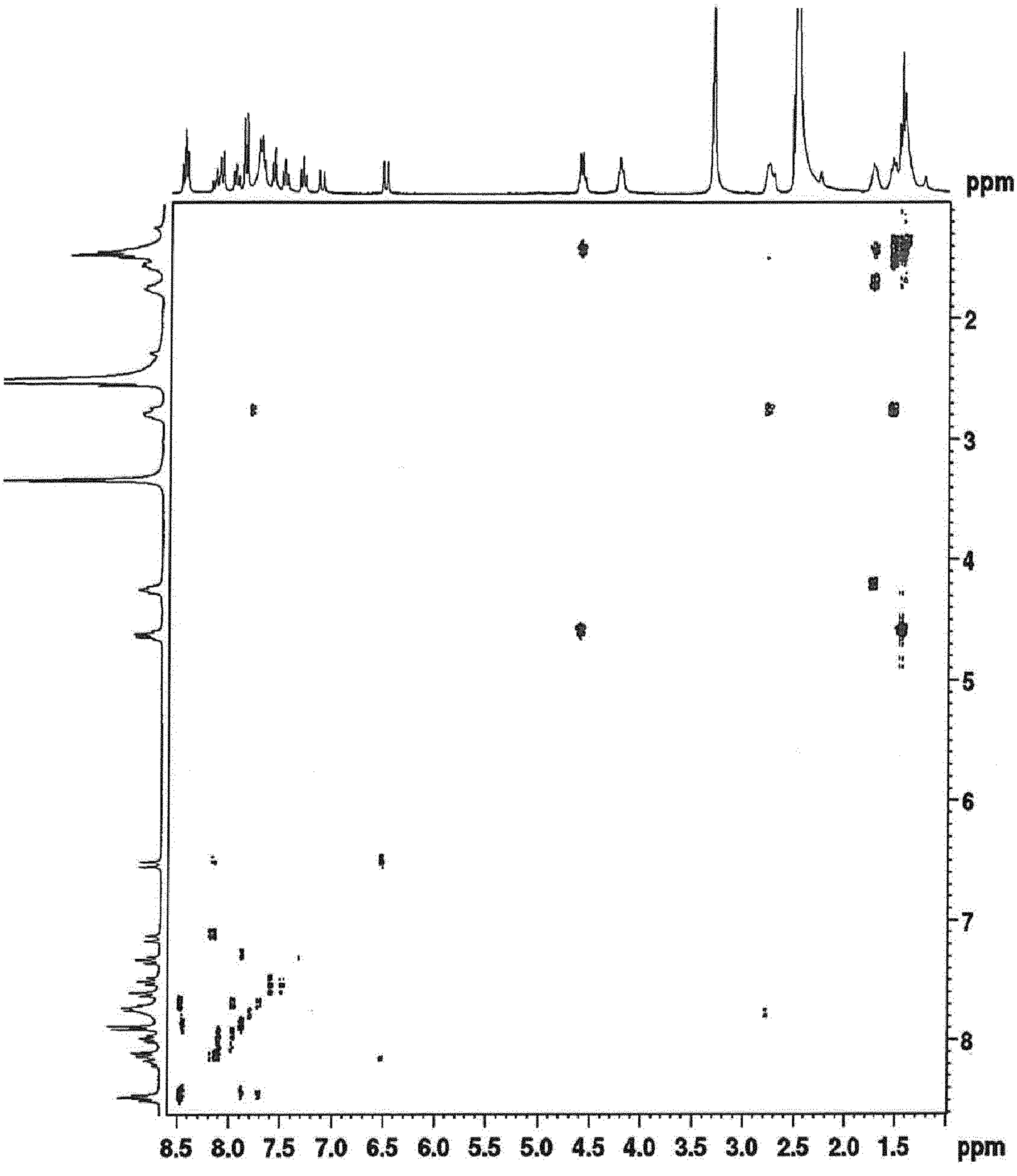

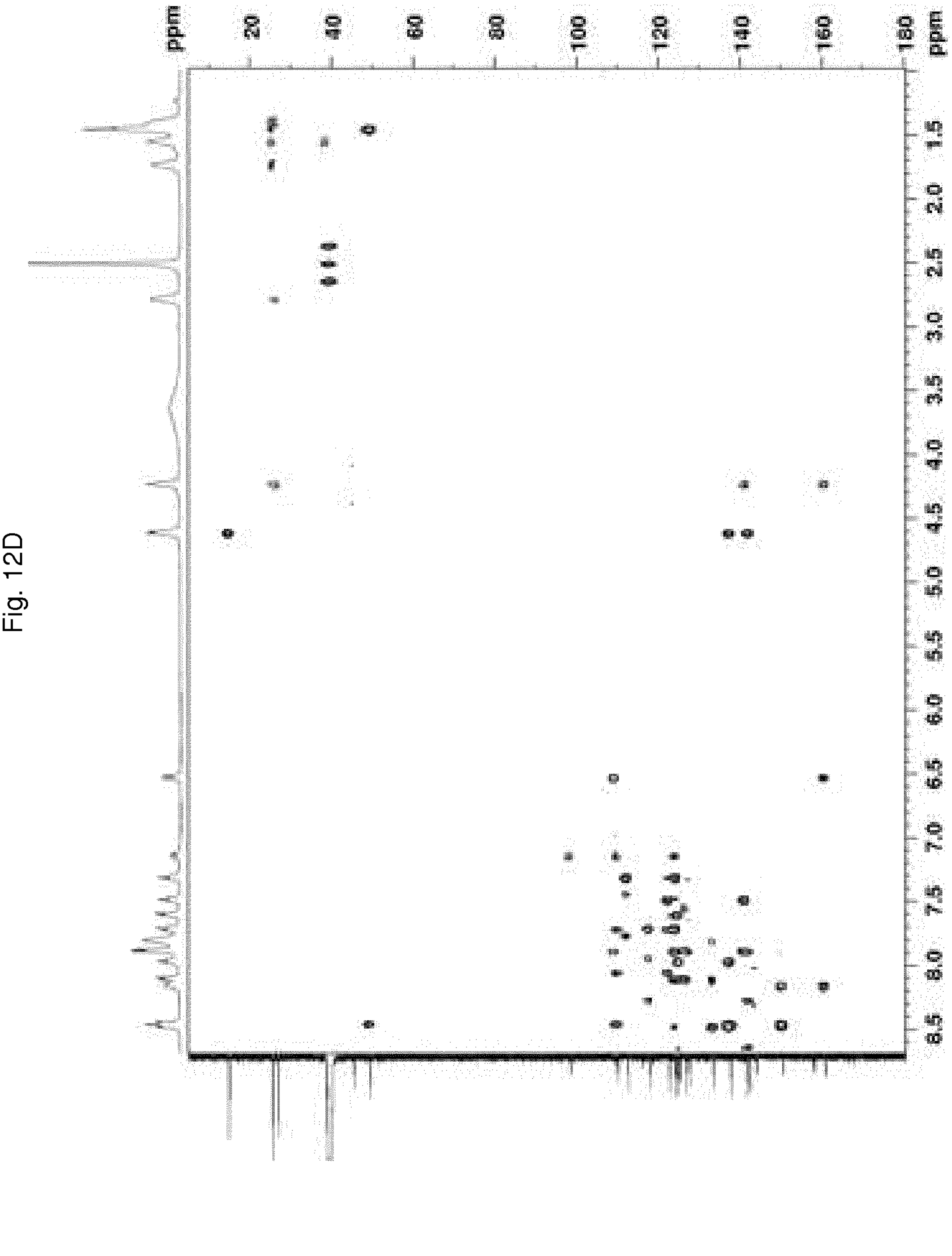
ppm
20
40
60
80
100
120
140
160
180
ppm
1.5
2.0
2.5
3.0
3.5
4.0
4.5
5.0
5.5
6.0
6.5
7.0
7.5
8.0
8.5

Fig. 12D

Fig. 13

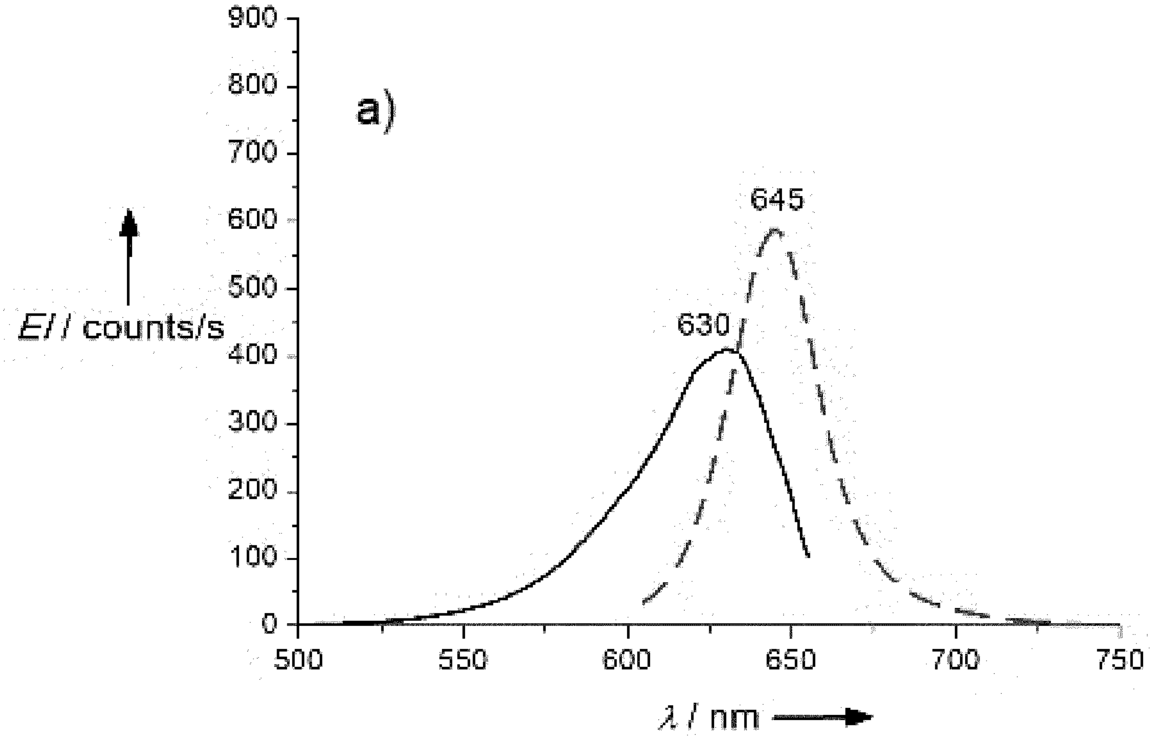
a)
900
800
700
600
500
400
300
200
100
0
EI / counts/s
645
630
500
550
600
650
700
750
λ / nm

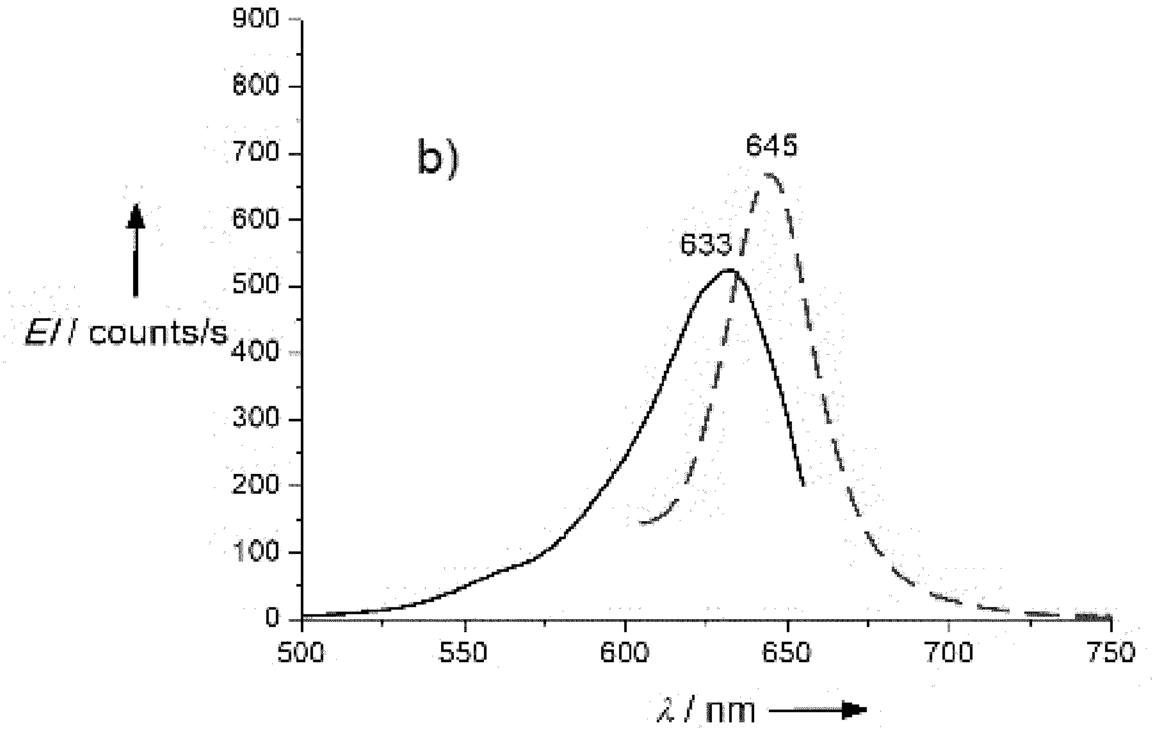
b)
900
800
700
600
500
400
300
200
100
0
EI / counts/s
645
633
500
550
600
650
700
750
λ / nm

Fig. 13 (cont.)

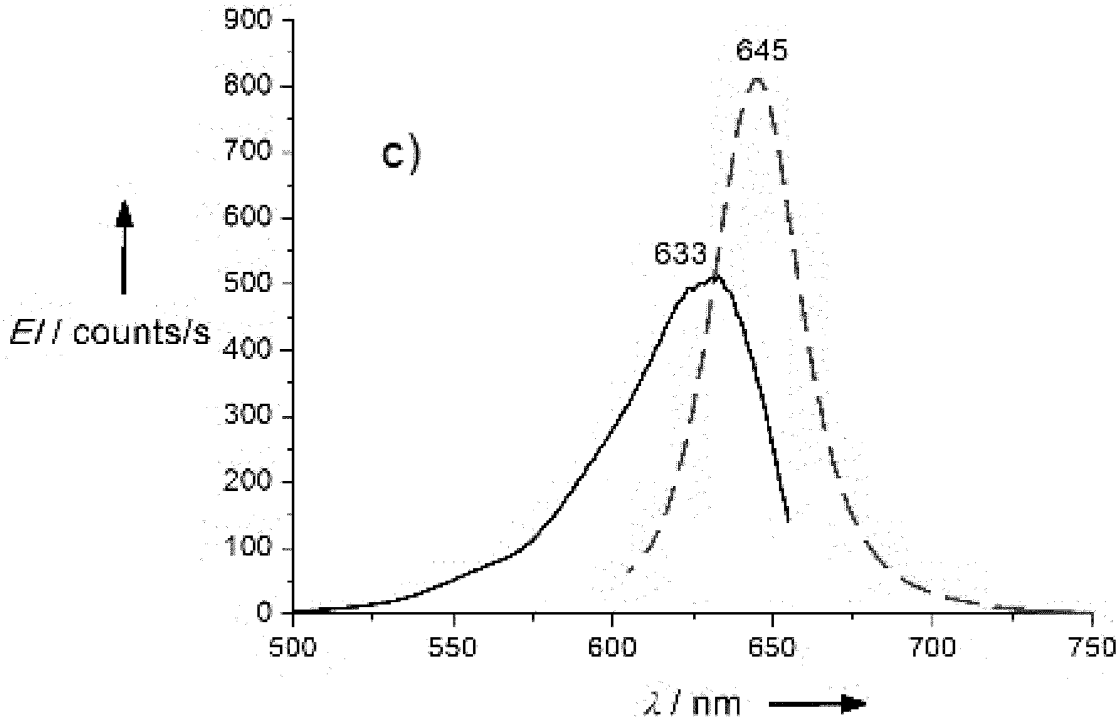


Fig. 14

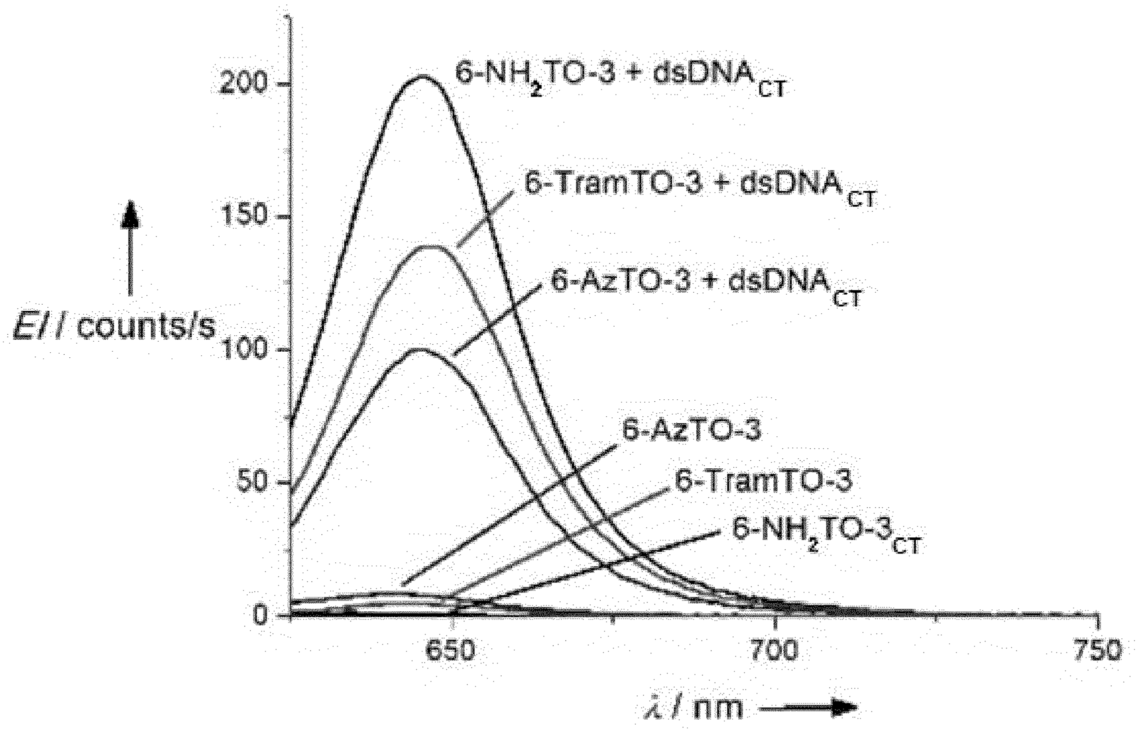

Fig. 15

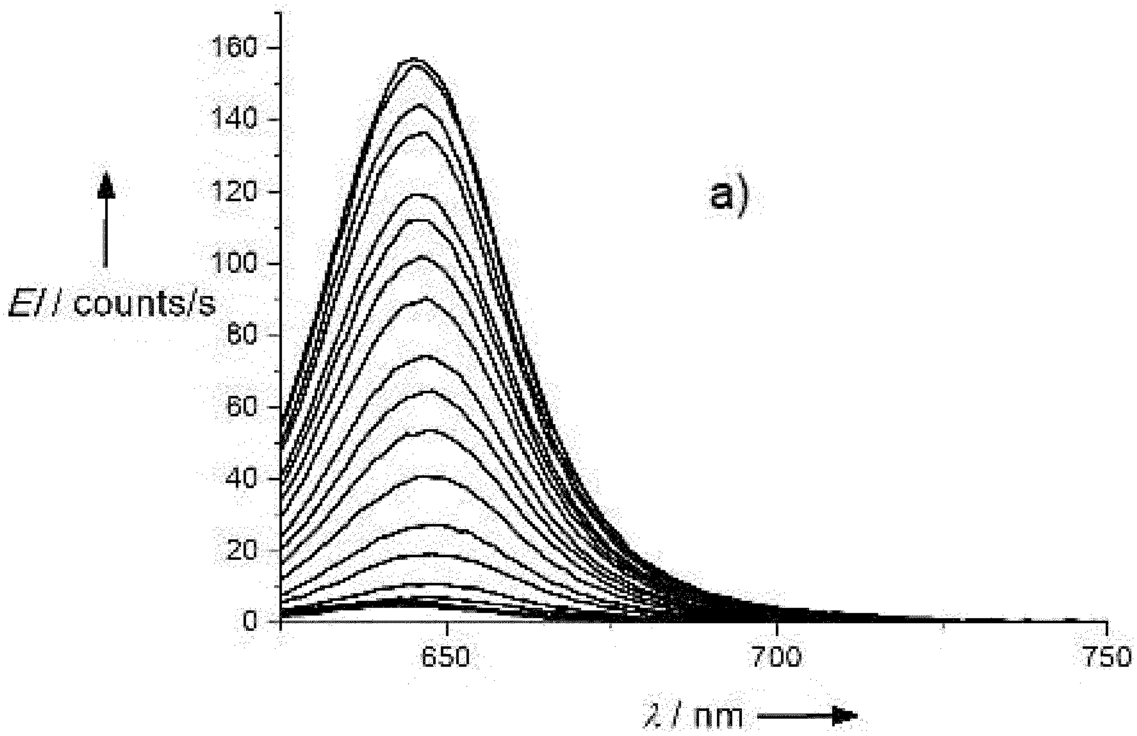
a)
EI / counts/s
0
20
40
60
80
100
120
140
160
650
700
750
λ / nm

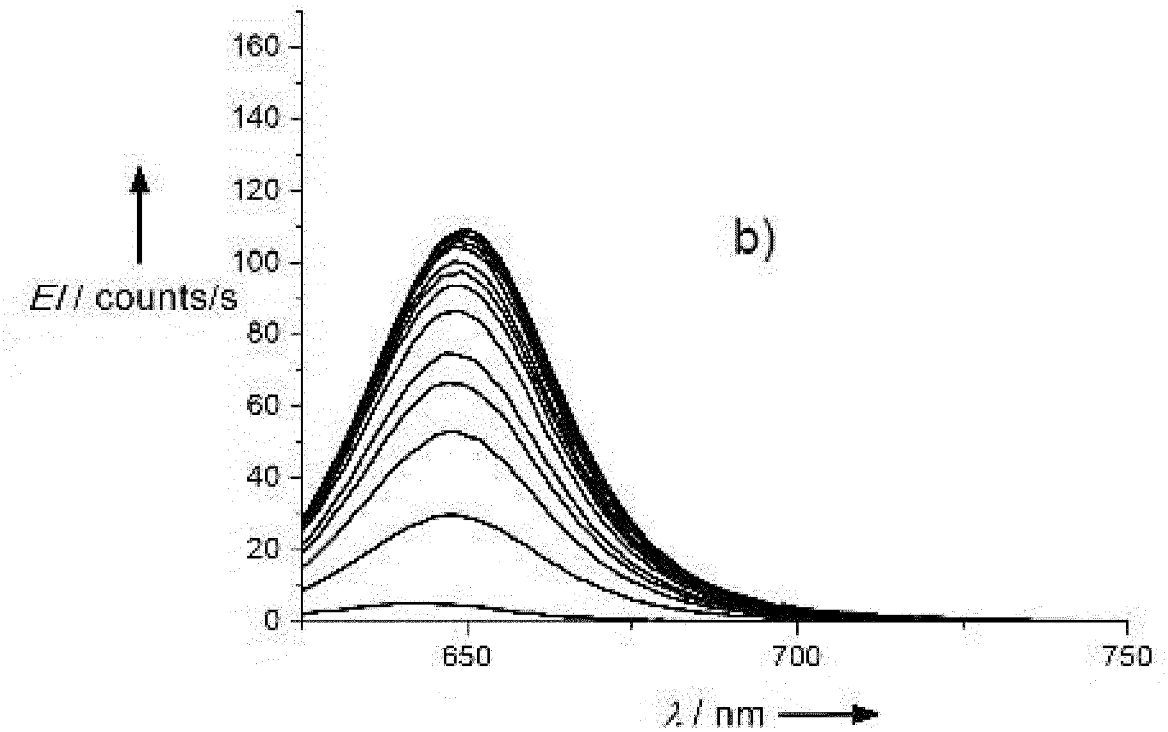
b)
EI / counts/s
0
20
40
60
80
100
120
140
160
650
700
750
λ / nm

Fig. 15 (cont.)

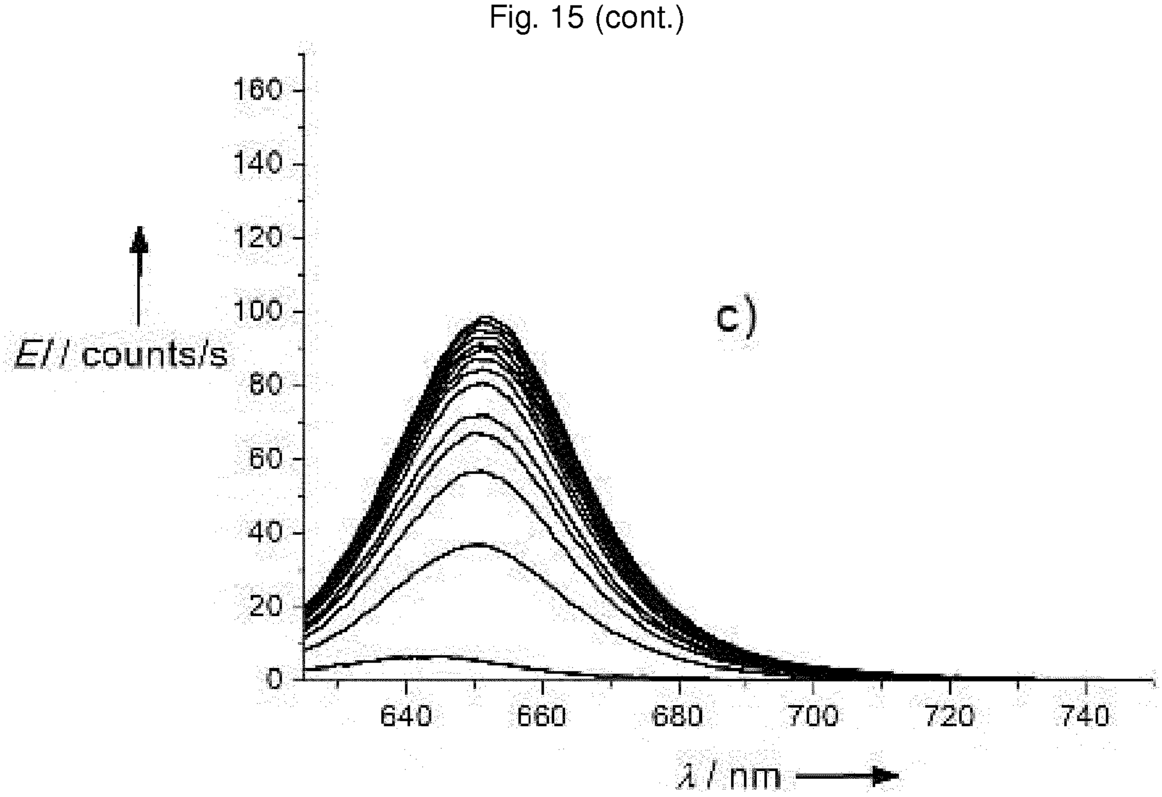


Fig. 16

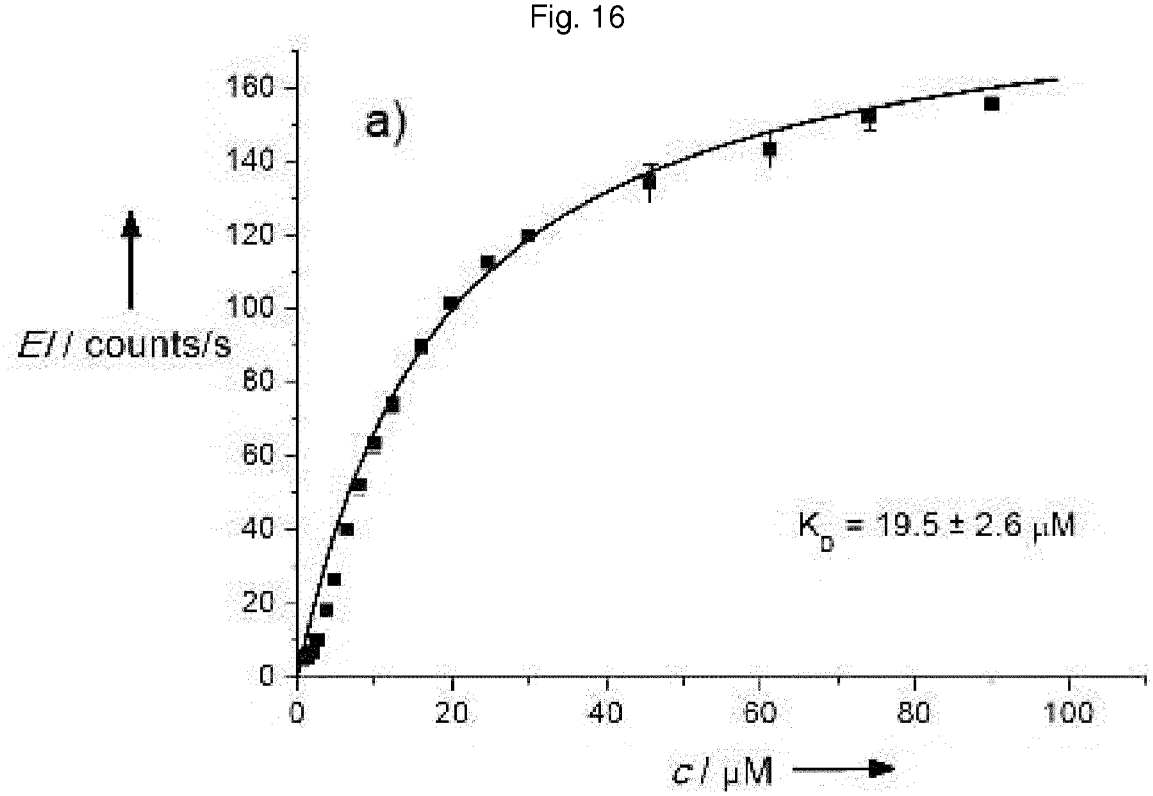

Fig. 16 (cont.)

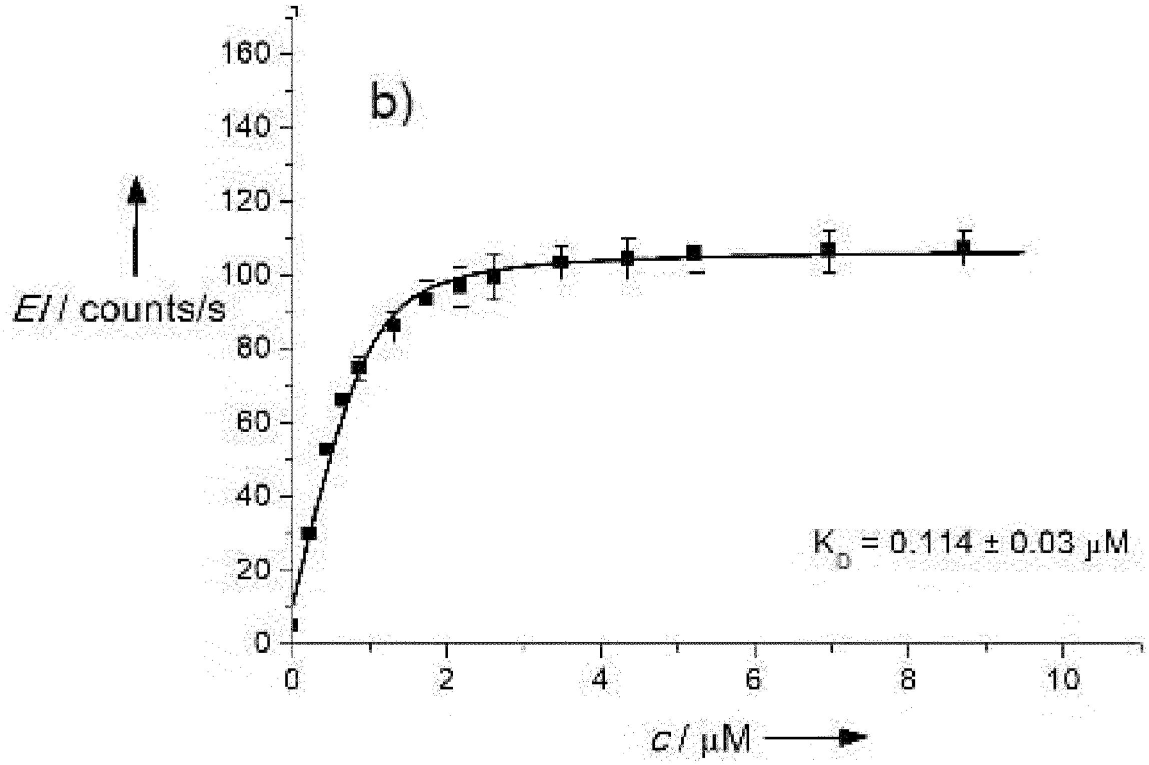
b)
EI / counts/s
c / µM
K_D = 0.114 ± 0.03 µM
0
20
40
60
80
100
120
140
160
0
2
4
6
8
10

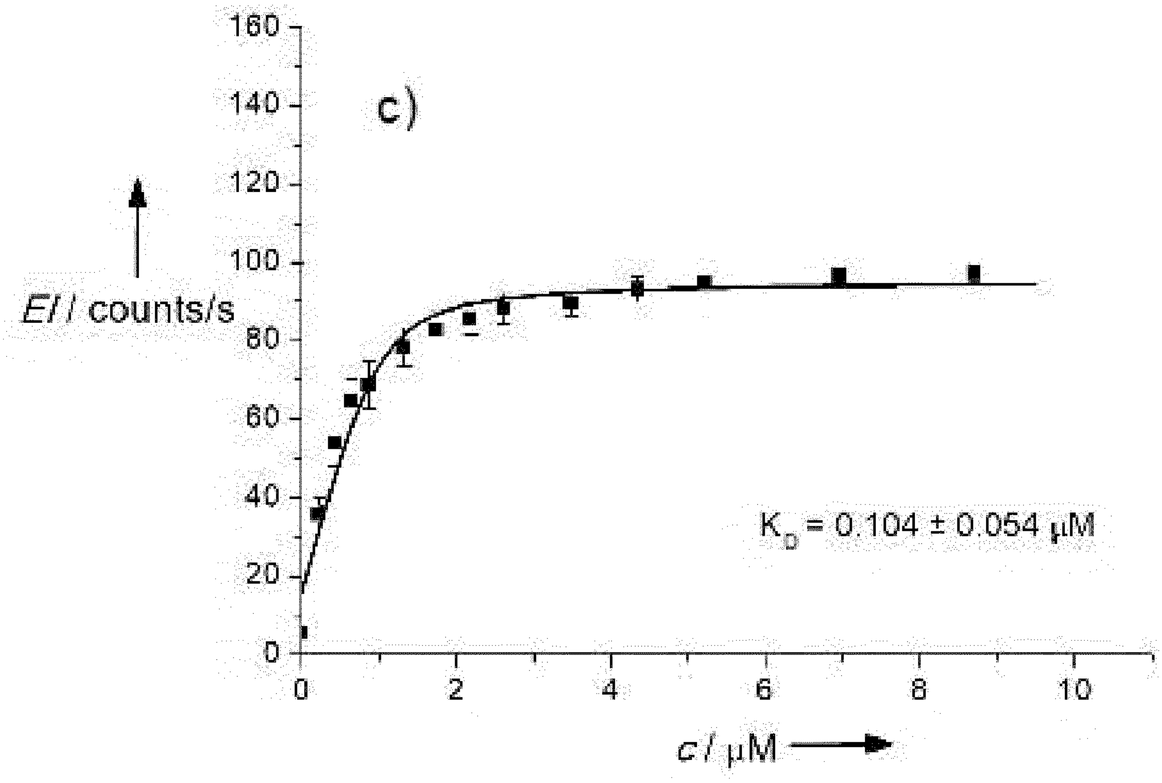
c)
EI / counts/s
c / µM
K_D = 0.104 ± 0.054 µM
0
20
40
60
80
100
120
140
160
0
2
4
6
8
10

Fig. 17

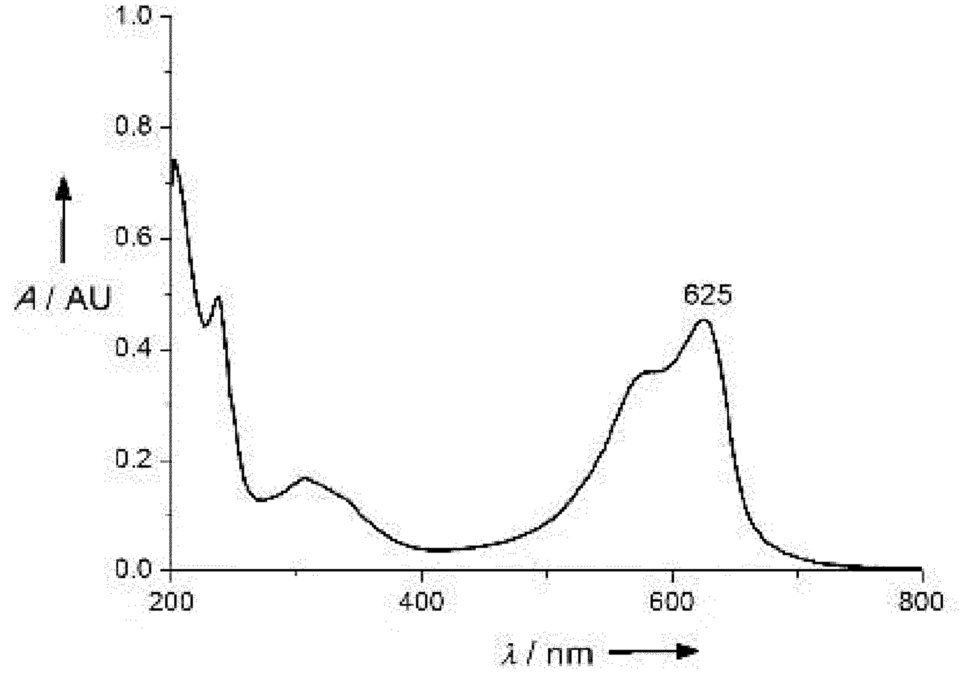
1.0
0.8
0.6
0.4
0.2
0.0
A / AU
625
200
400
600
800
λ / nm

Fig. 18

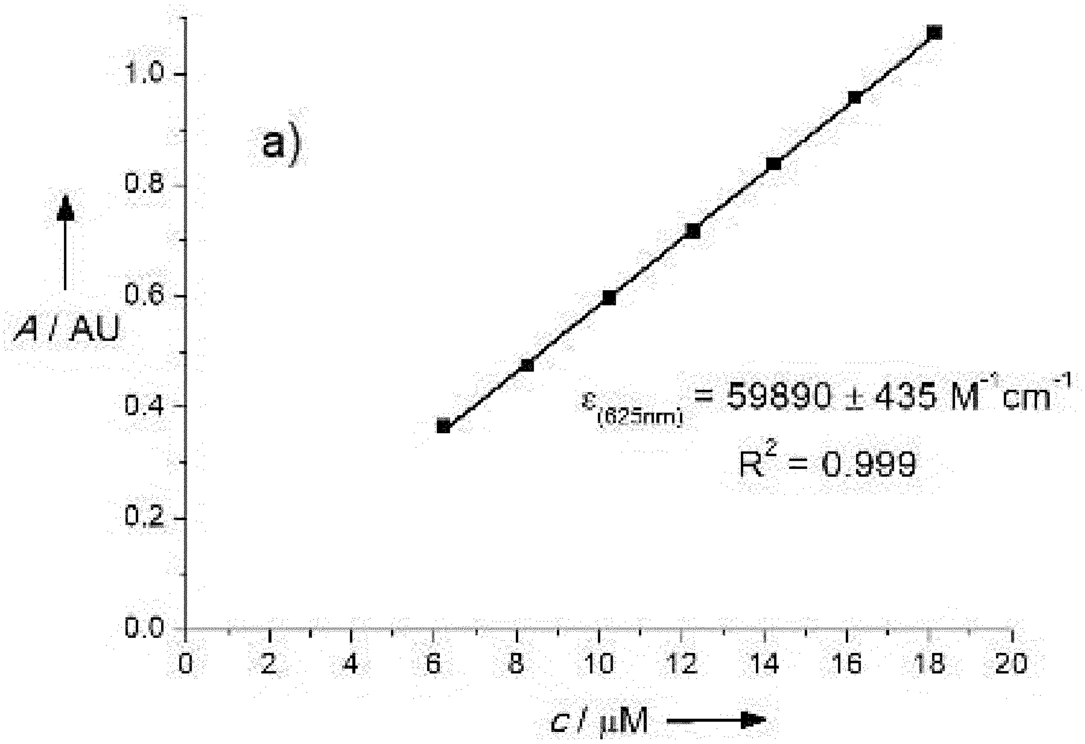
a)
1.0
0.8
0.6
0.4
0.2
0.0
A / AU
ε(625nm) = 59890 ± 435 M-1cm-1
R2 = 0.999
0
2
4
6
8
10
12
14
16
18
20
c / μM

Fig. 18 (cont.)

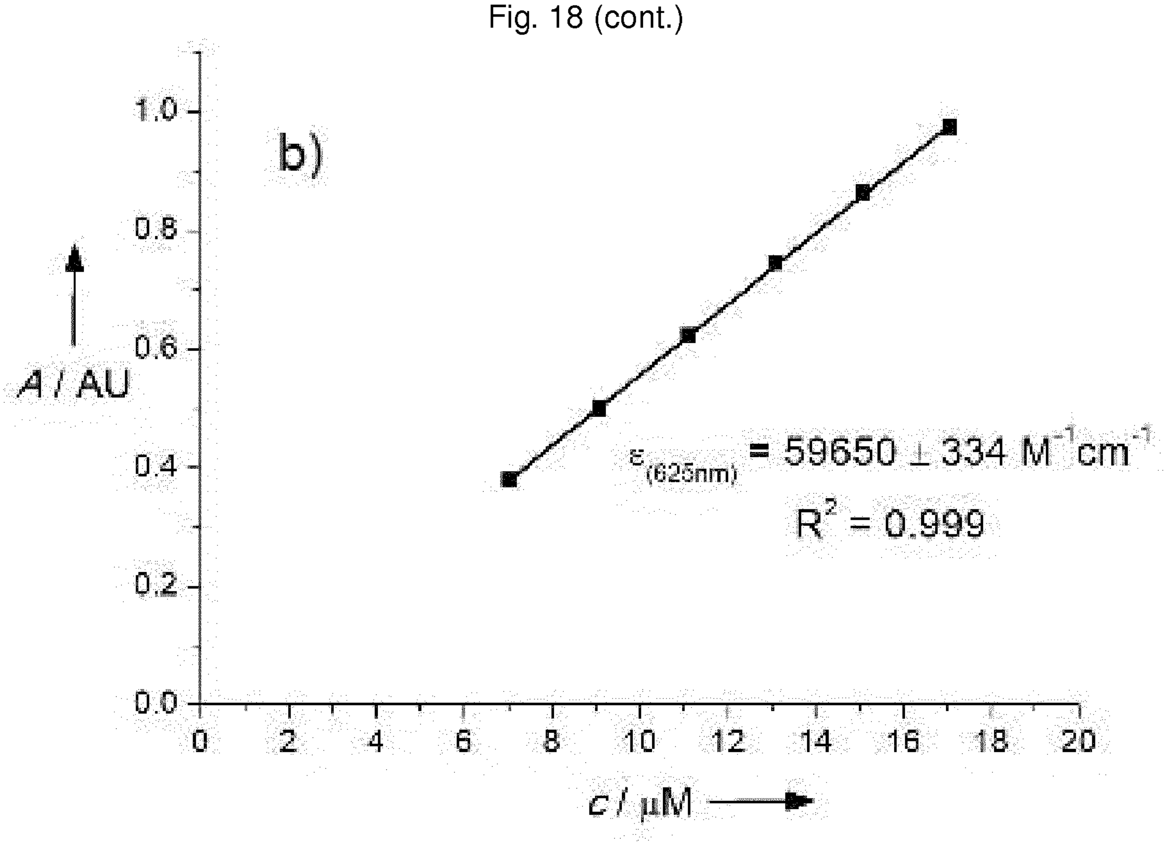
b)
A / AU
c / µM
ε(625nm) = 59650 ± 334 M-1cm-1
R2 = 0.999

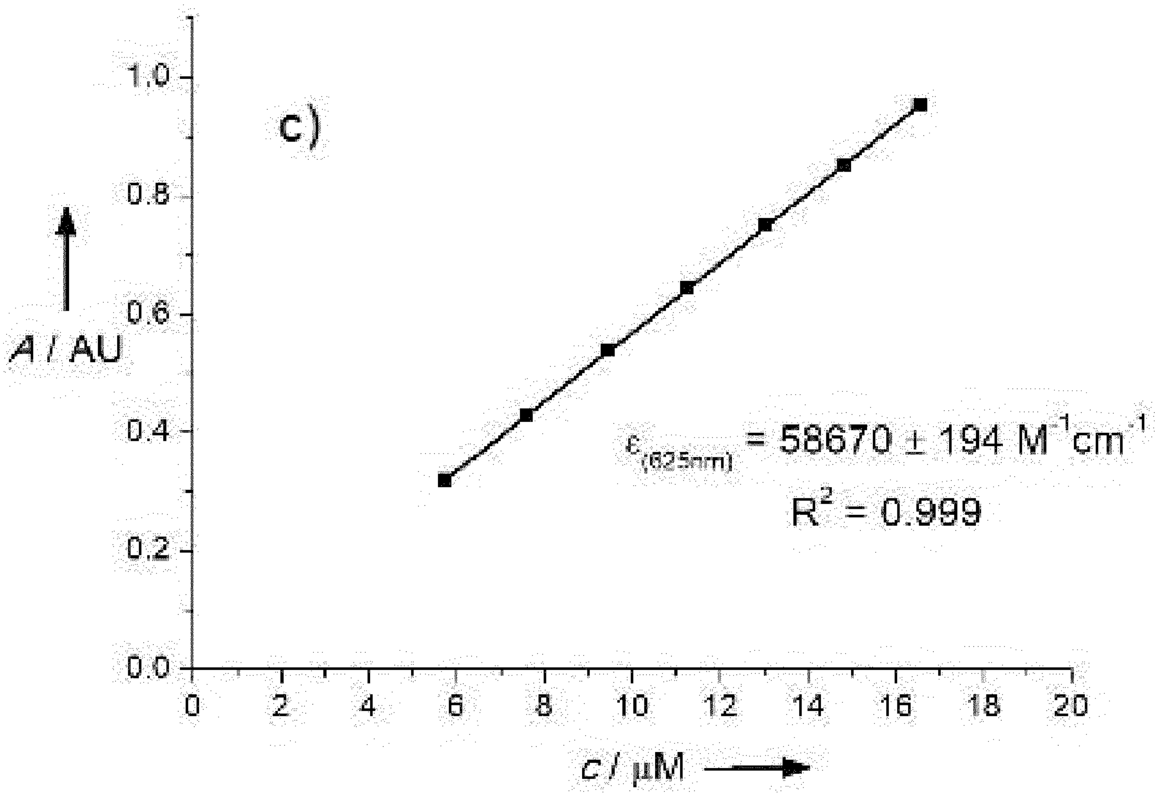
c)
A / AU
c / µM
ε(625nm) = 58670 ± 194 M-1cm-1
R2 = 0.999

Fig. 19

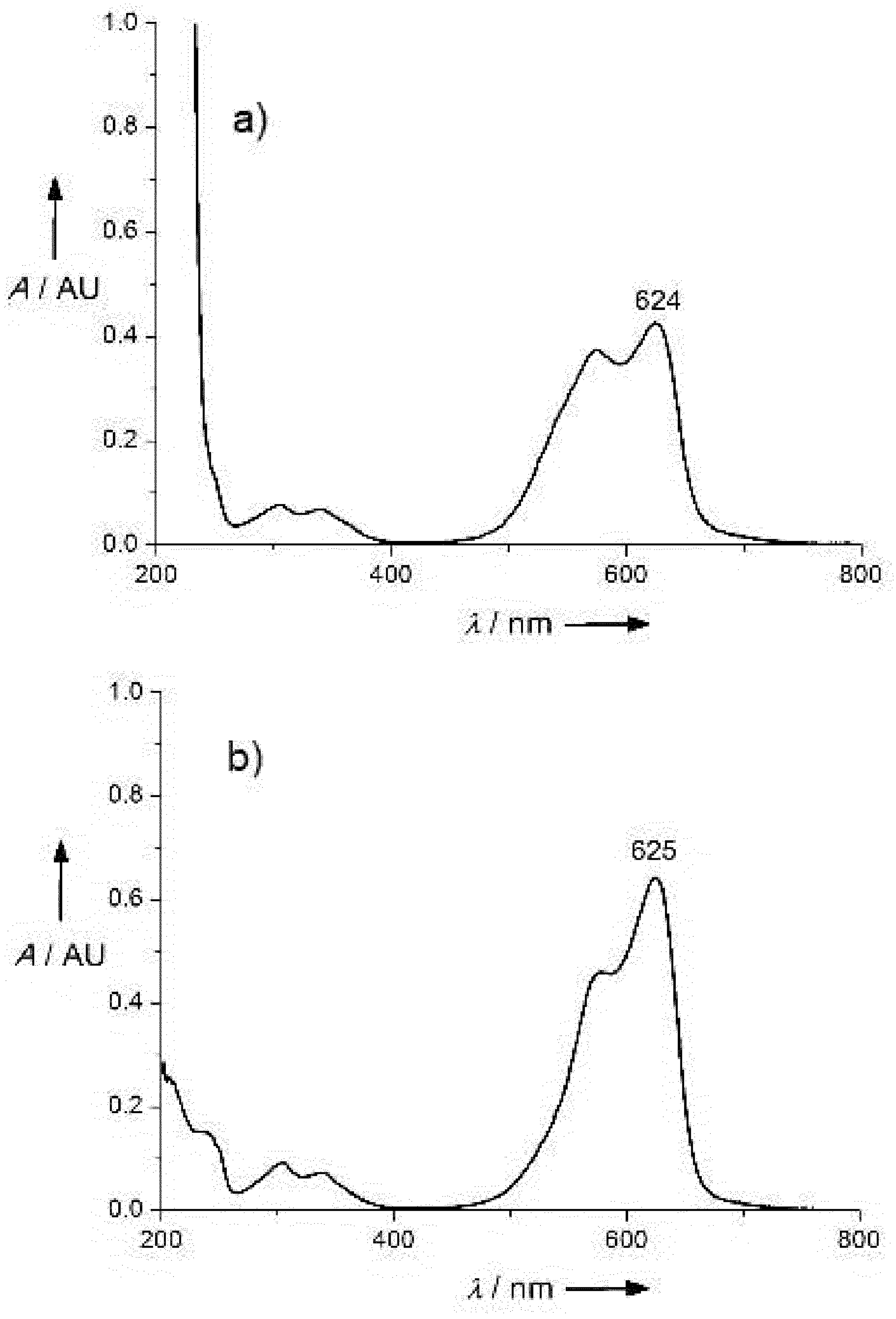

Fig. 20

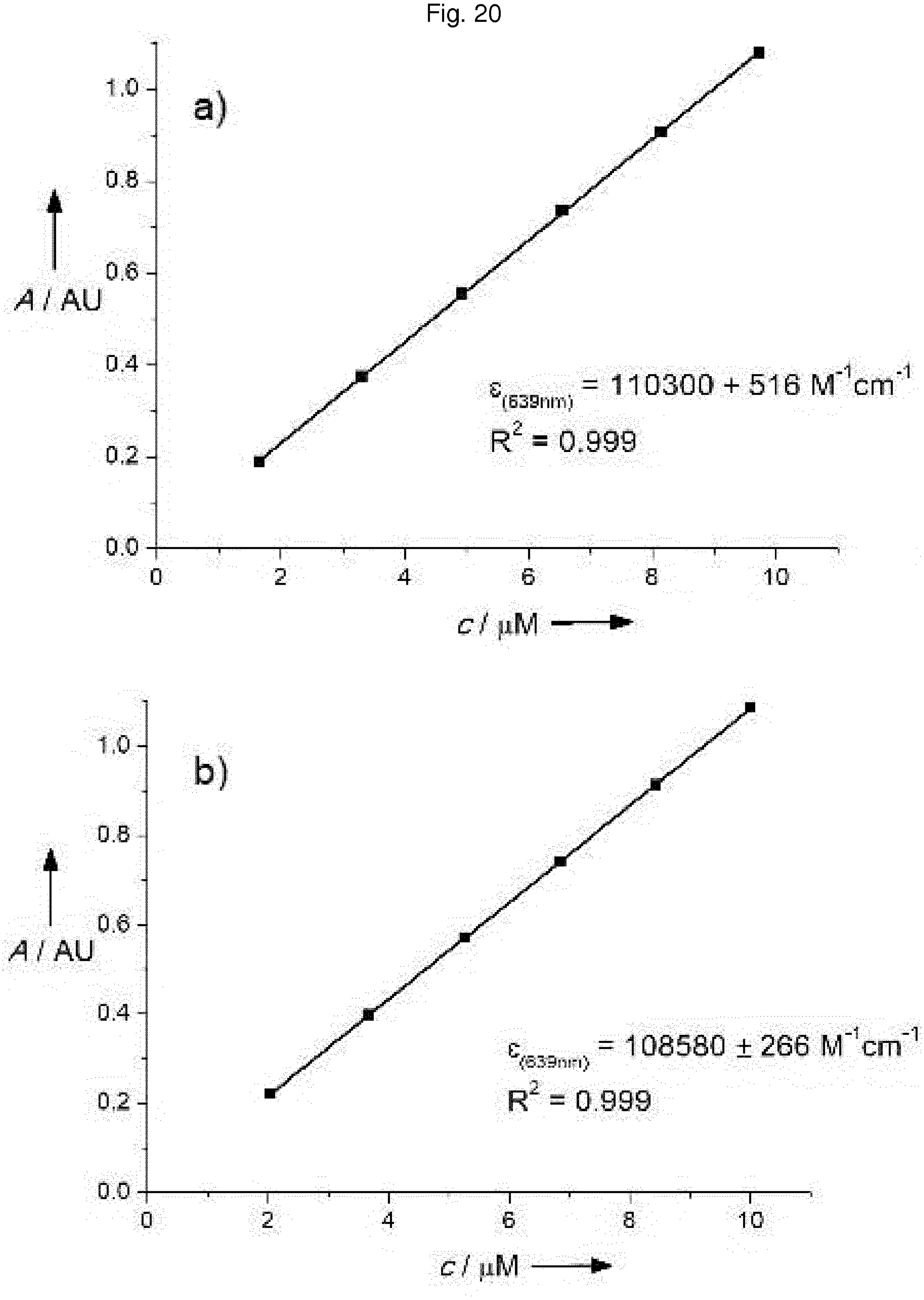
a)
A / AU
c / μM
ε(639nm) = 110300 + 516 M-1cm-1
R2 = 0.999
b)
A / AU
c / μM
ε(639nm) = 108580 ± 266 M-1cm-1
R2 = 0.999

Fig. 20 (cont.)

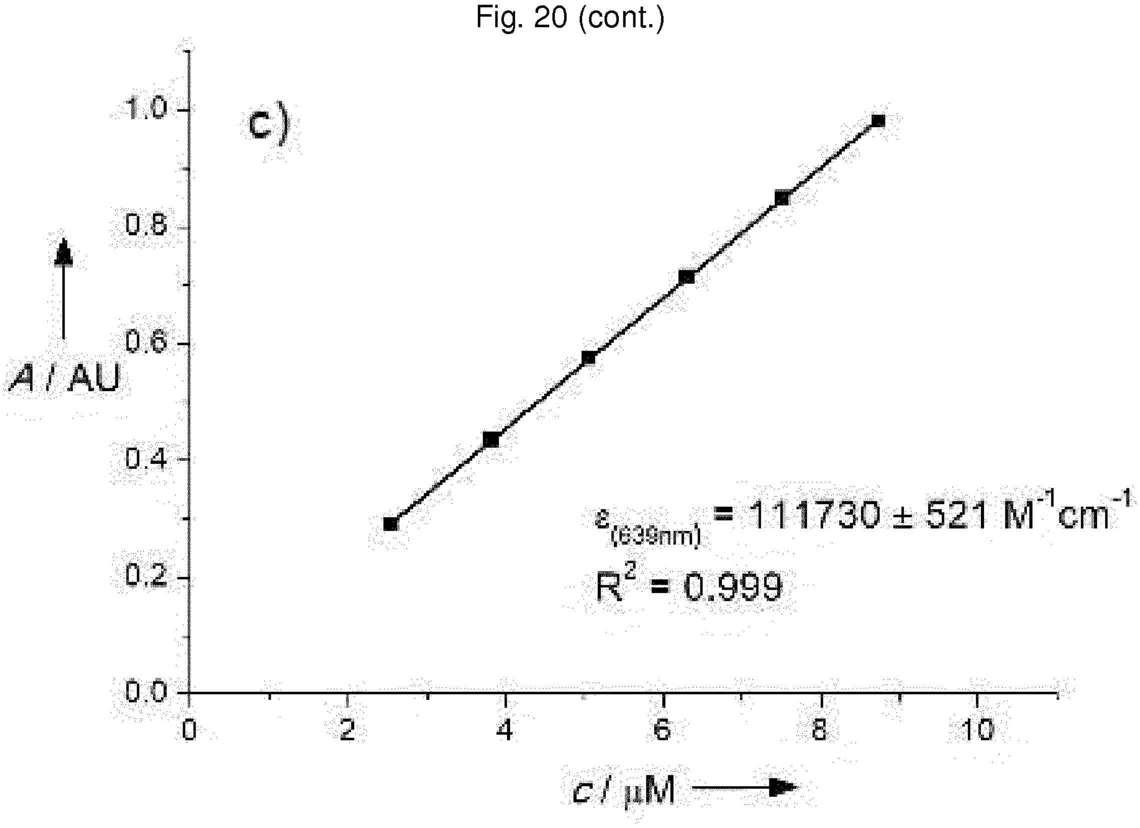


Fig. 21

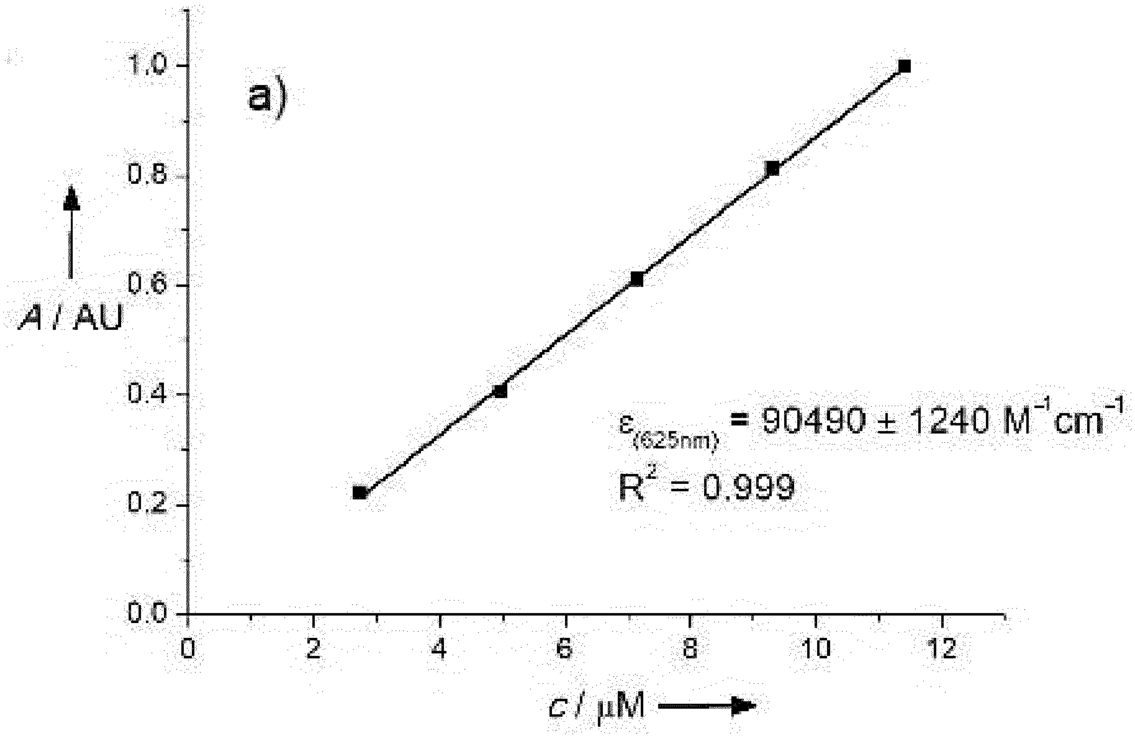

Fig. 21 (cont.)

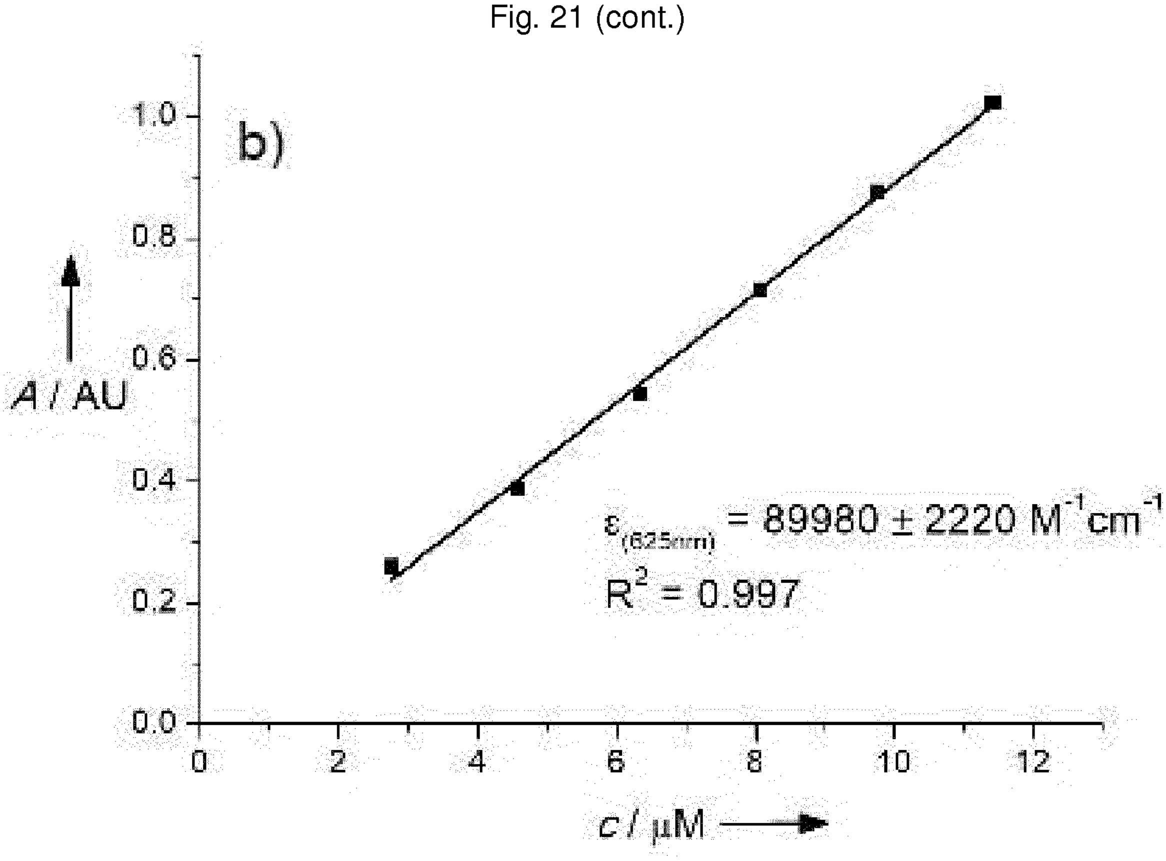
b)
A / AU
c / µM
ε(625nm) = 89980 ± 2220 M-1cm-1
R2 = 0.997
0.0
0.2
0.4
0.6
0.8
1.0
0
2
4
6
8
10
12

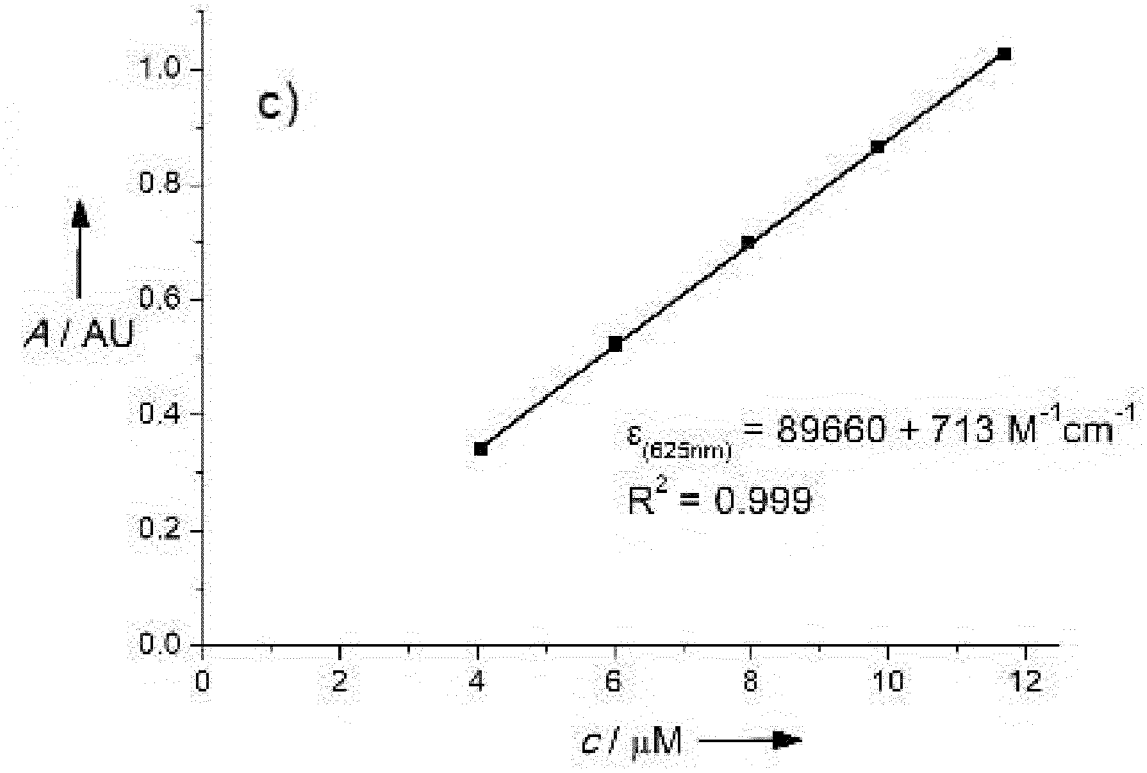
c)
A / AU
c / µM
ε(625nm) = 89660 + 713 M-1cm-1
R2 = 0.999
0.0
0.2
0.4
0.6
0.8
1.0
0
2
4
6
8
10
12

Fig. 22

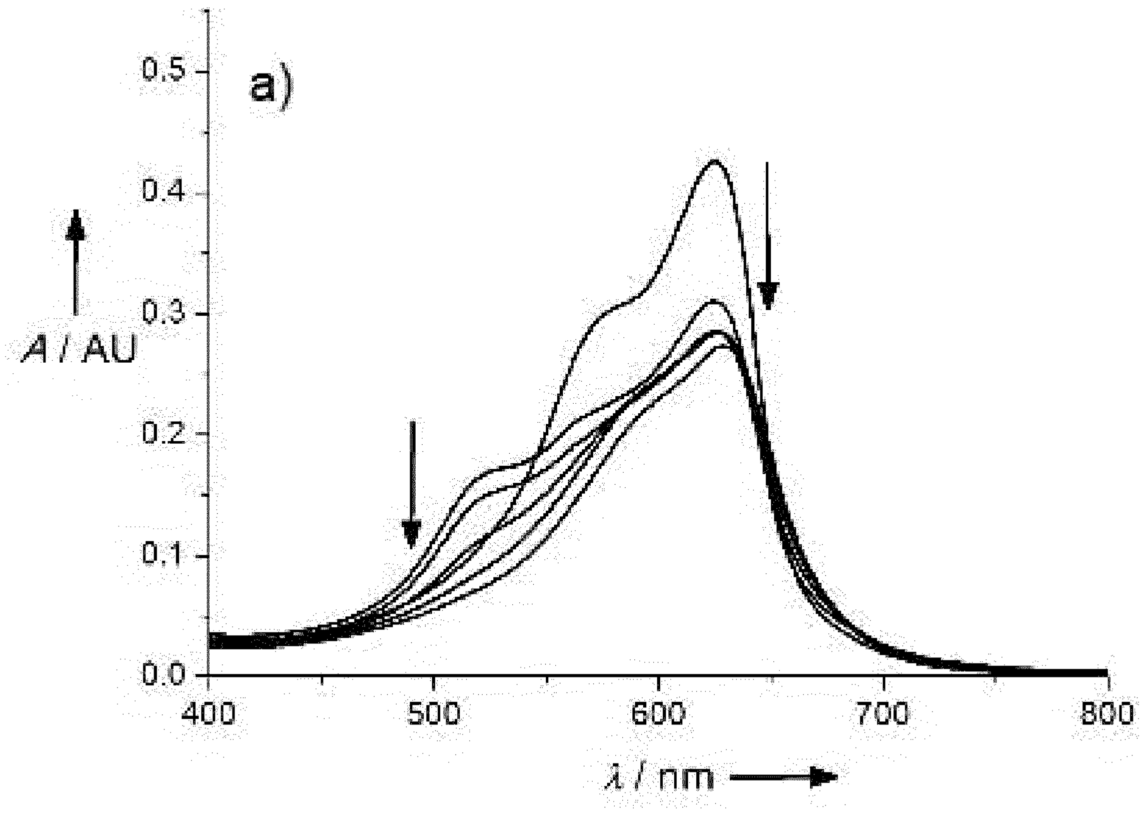
a)
0.0
0.1
0.2
0.3
0.4
0.5
A / AU
400
500
600
700
800
λ / nm

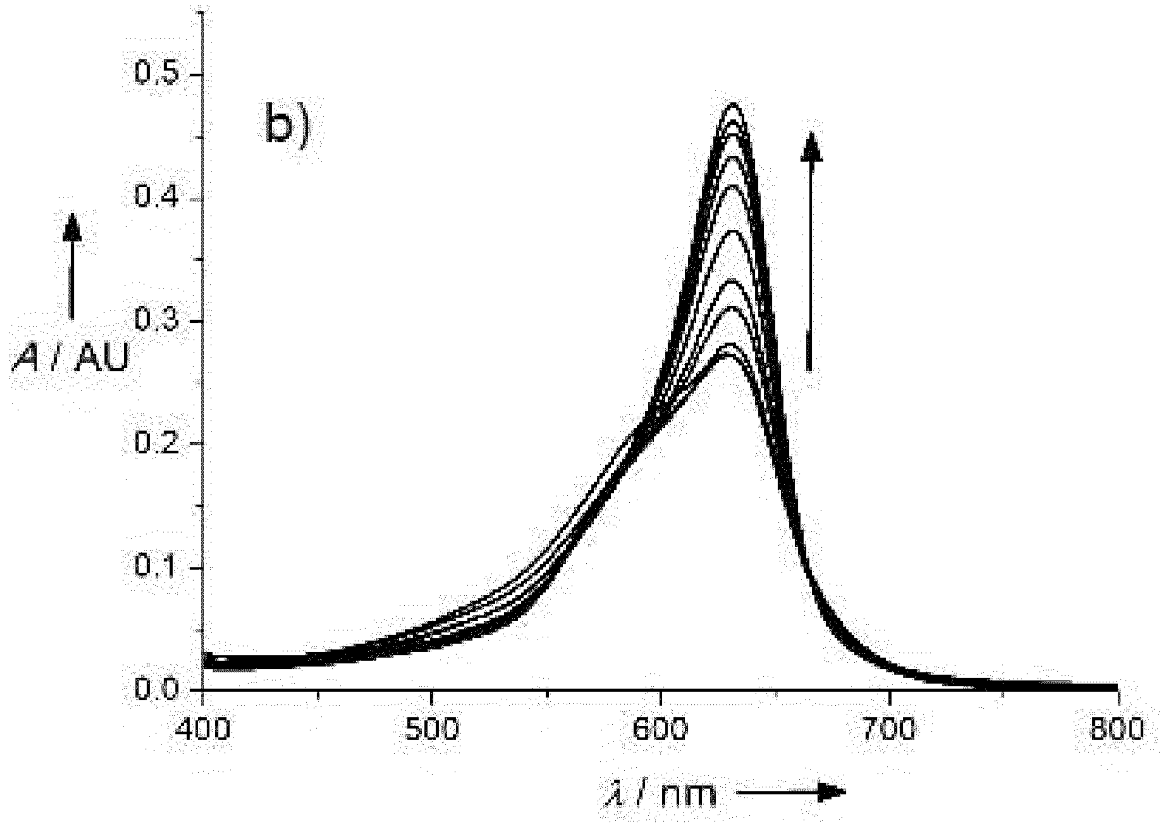
b)
0.0
0.1
0.2
0.3
0.4
0.5
A / AU
400
500
600
700
800
λ / nm

Fig. 22 (cont.)

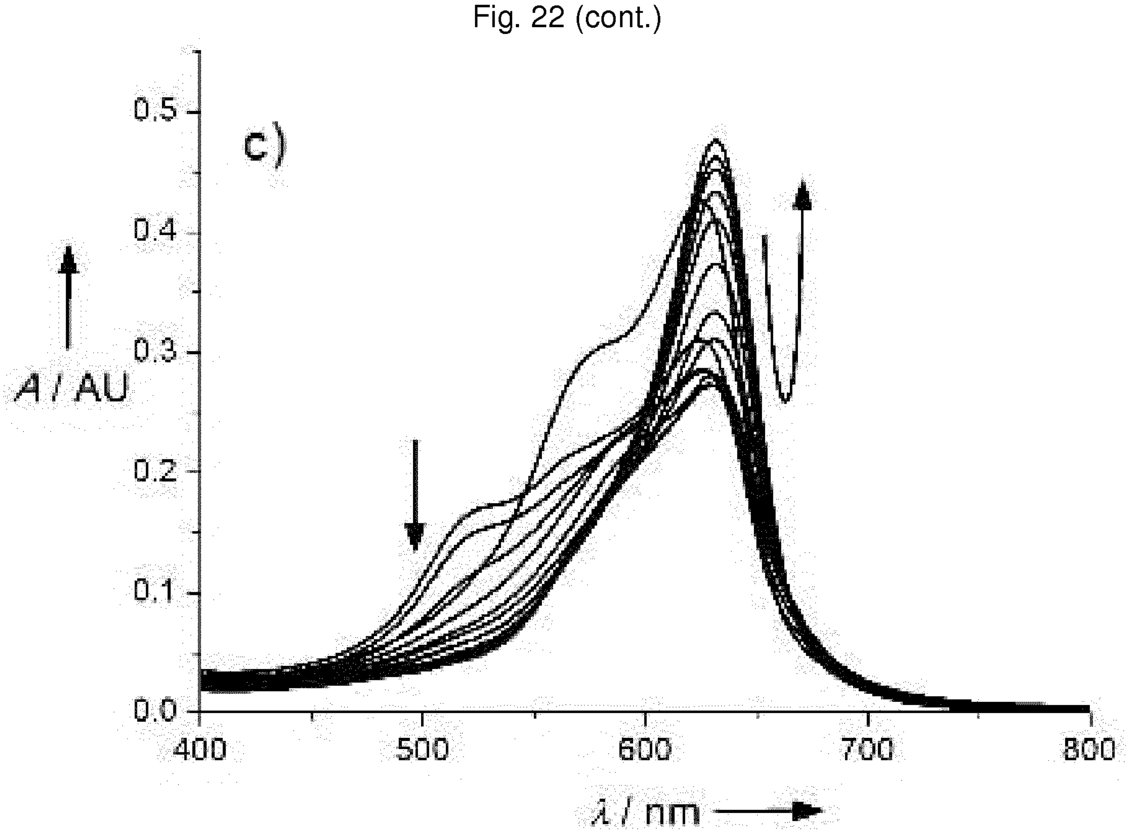


Fig. 23

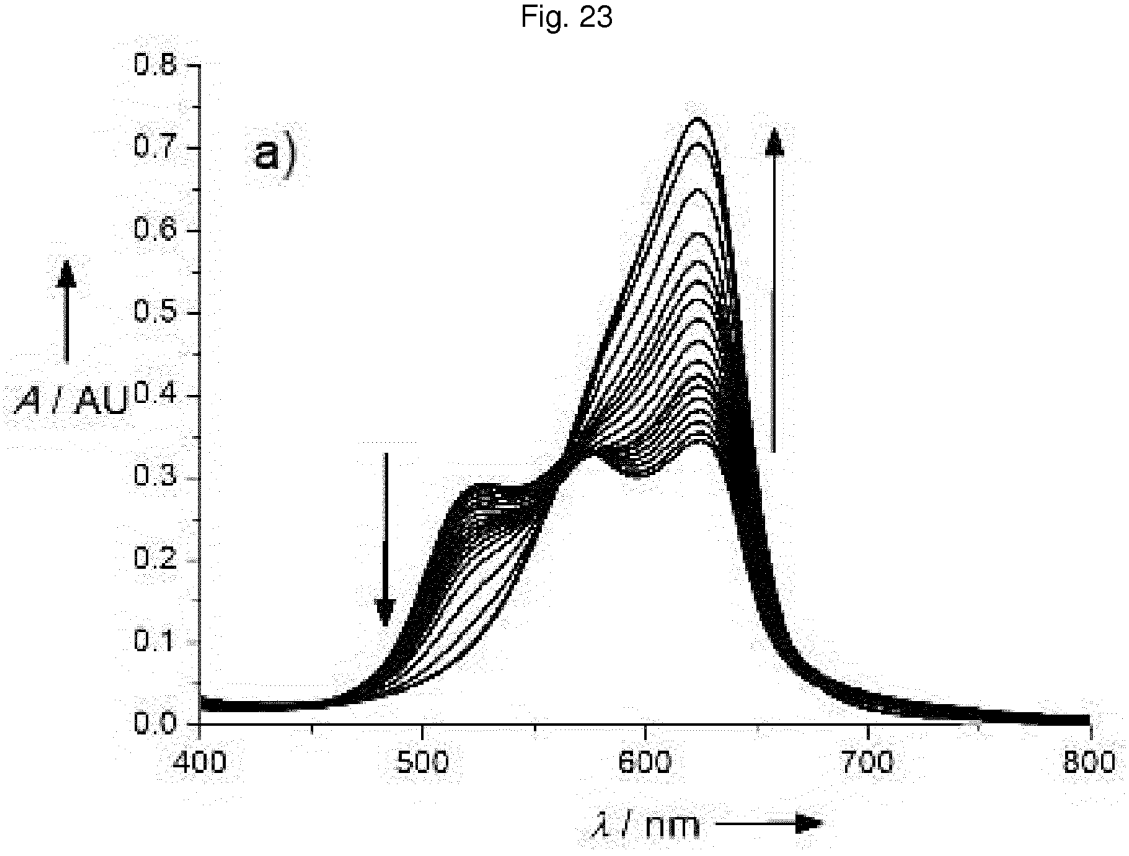

Fig. 23 (cont.)

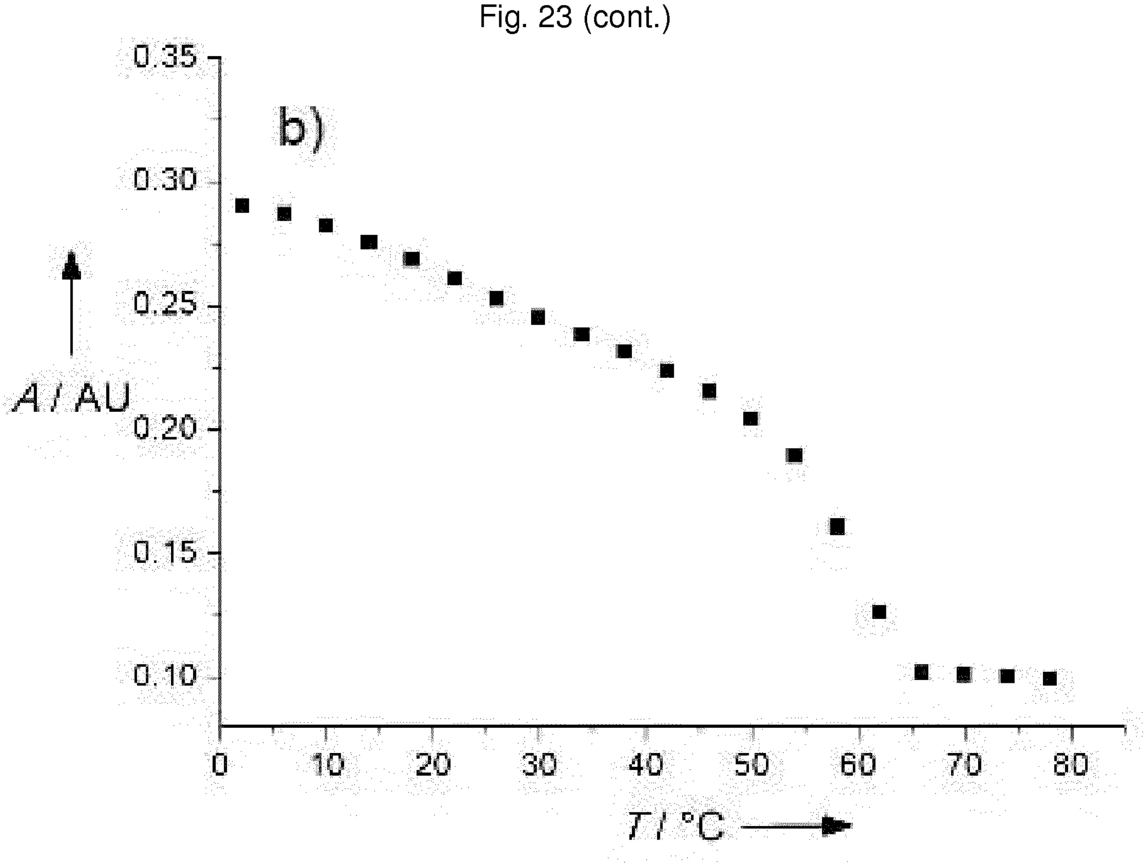
b)
A / AU
0.35
0.30
0.25
0.20
0.15
0.10
0
10
20
30
40
50
60
70
80
T / °C

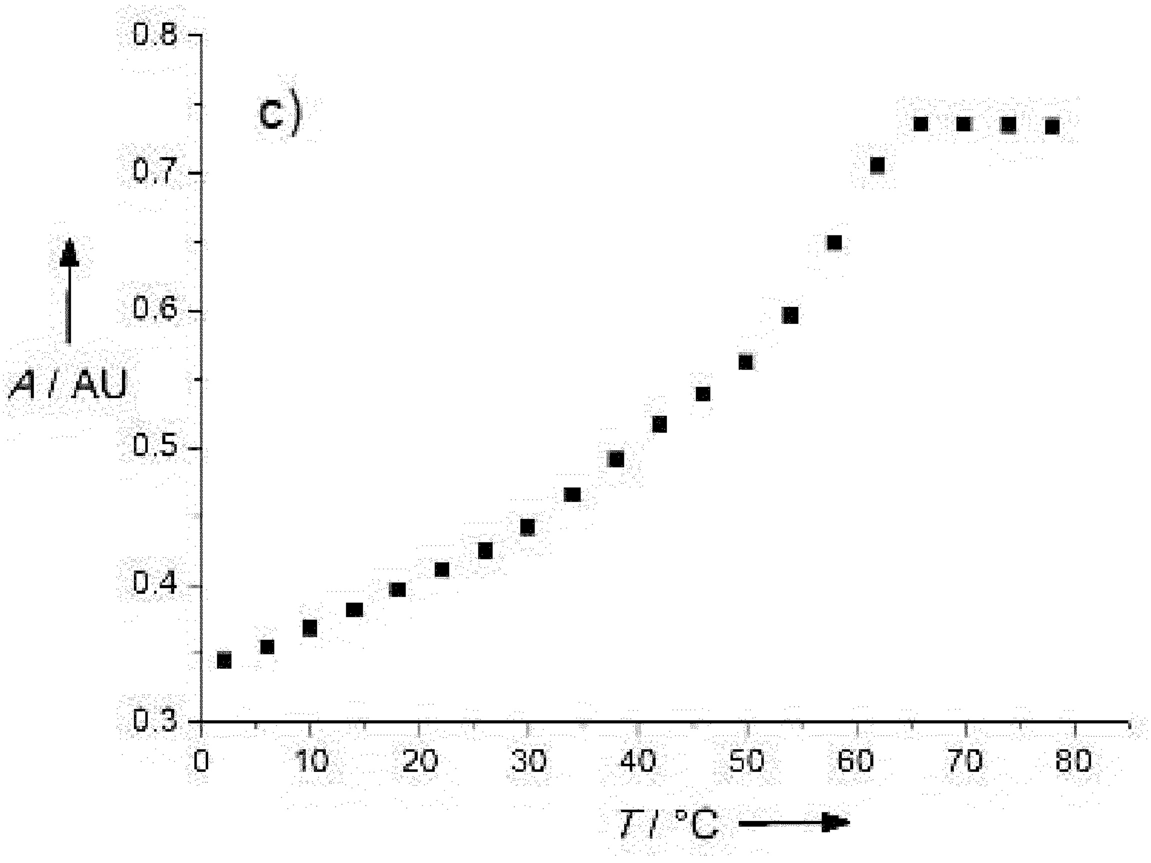
c)
A / AU
0.8
0.7
0.6
0.5
0.4
0.3
0
10
20
30
40
50
60
70
80
T / °C

Fig. 24

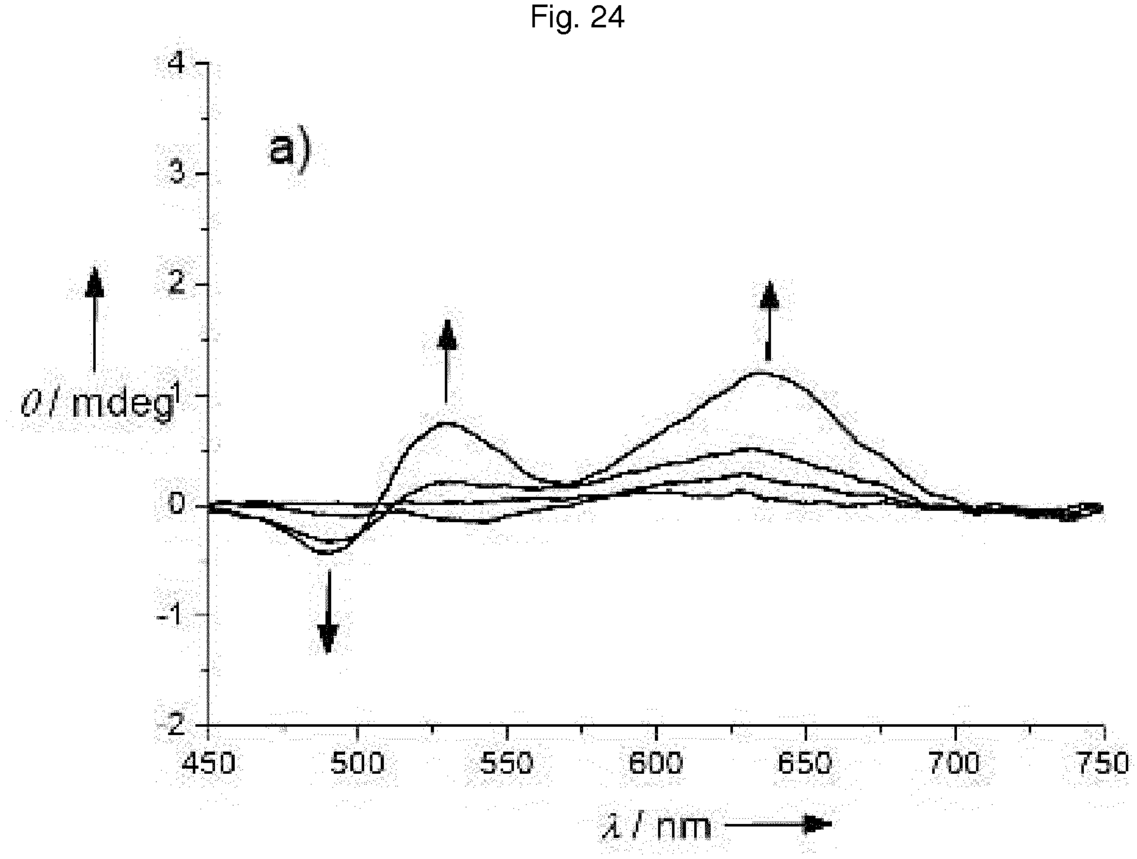
a)
4
3
2
1
0
-1
-2
θ / mdeg
450
500
550
600
650
700
750
λ / nm

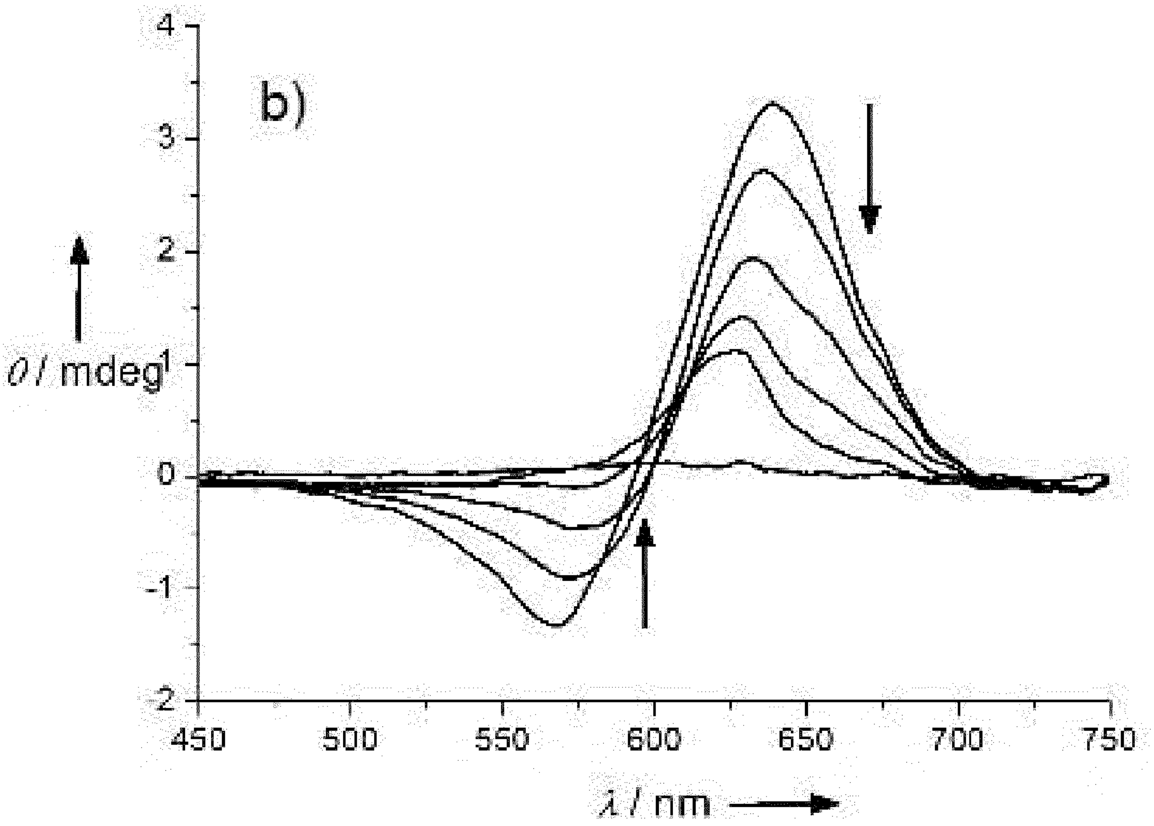
b)
4
3
2
1
0
-1
-2
θ / mdeg
450
500
550
600
650
700
750
λ / nm

Fig. 24 (cont.)

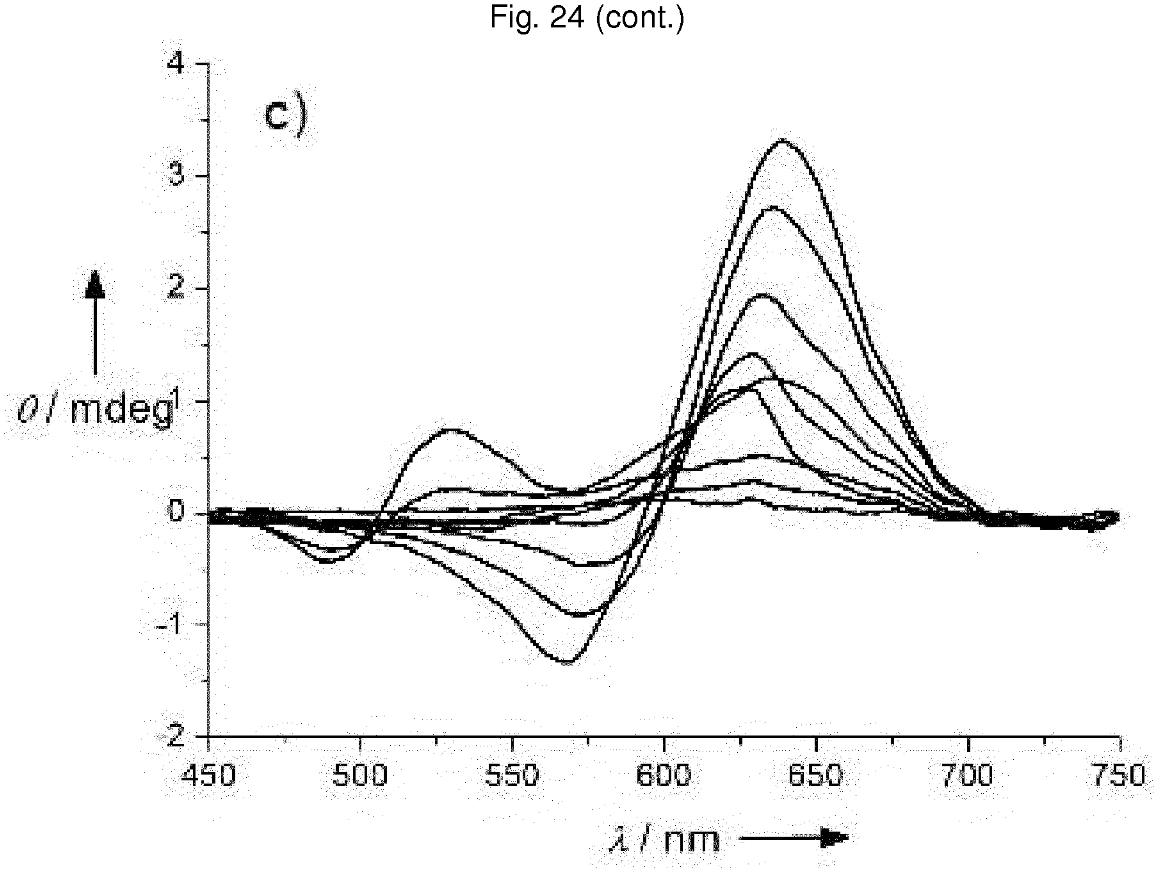

Fig. 25

a)

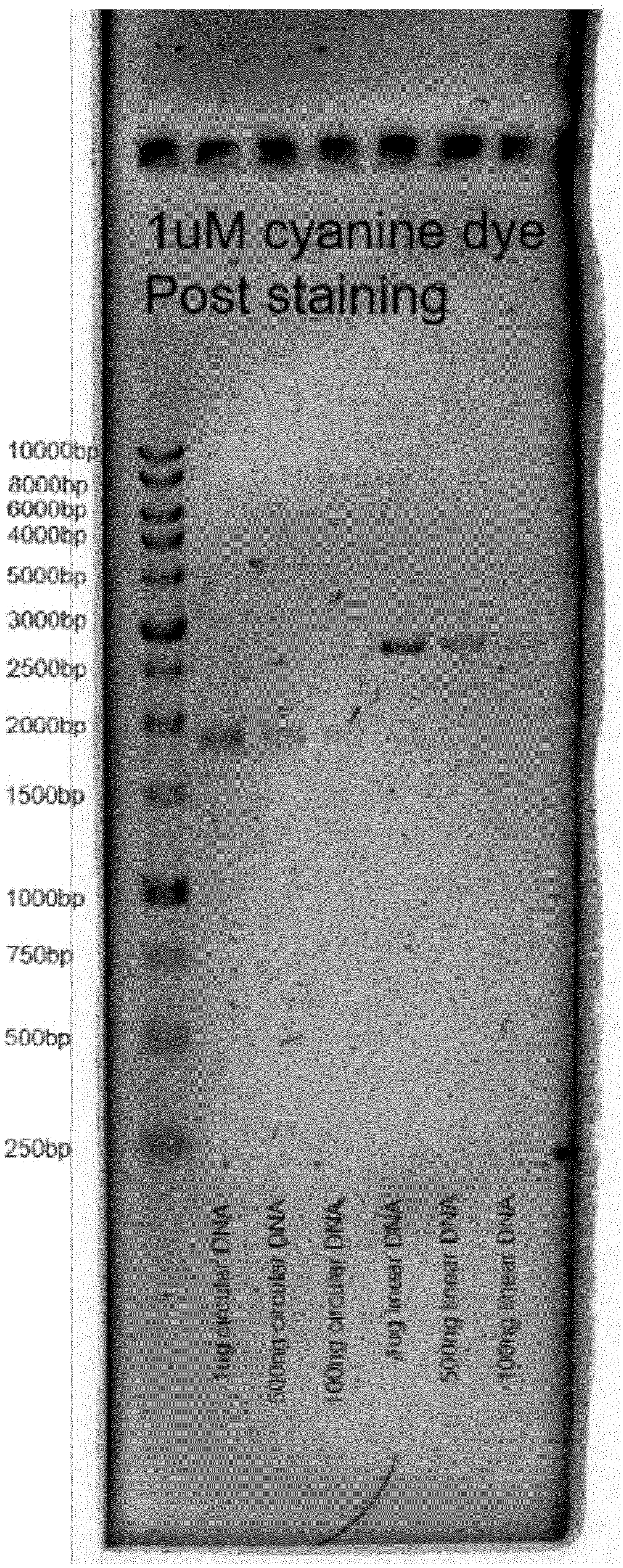
1uM cyanine dye
Post staining
DNA ladder CSL 1kb
10000bp
8000bp
6000bp
4000bp
5000bp
3000bp
2500bp
2000bp
1500bp
1000bp
750bp
500bp
250bp
1ug circular DNA
500ng circular DNA
100ng circular DNA
1ug linear DNA
500ng linear DNA
100ng linear DNA

b)

Fig. 25 (cont.)

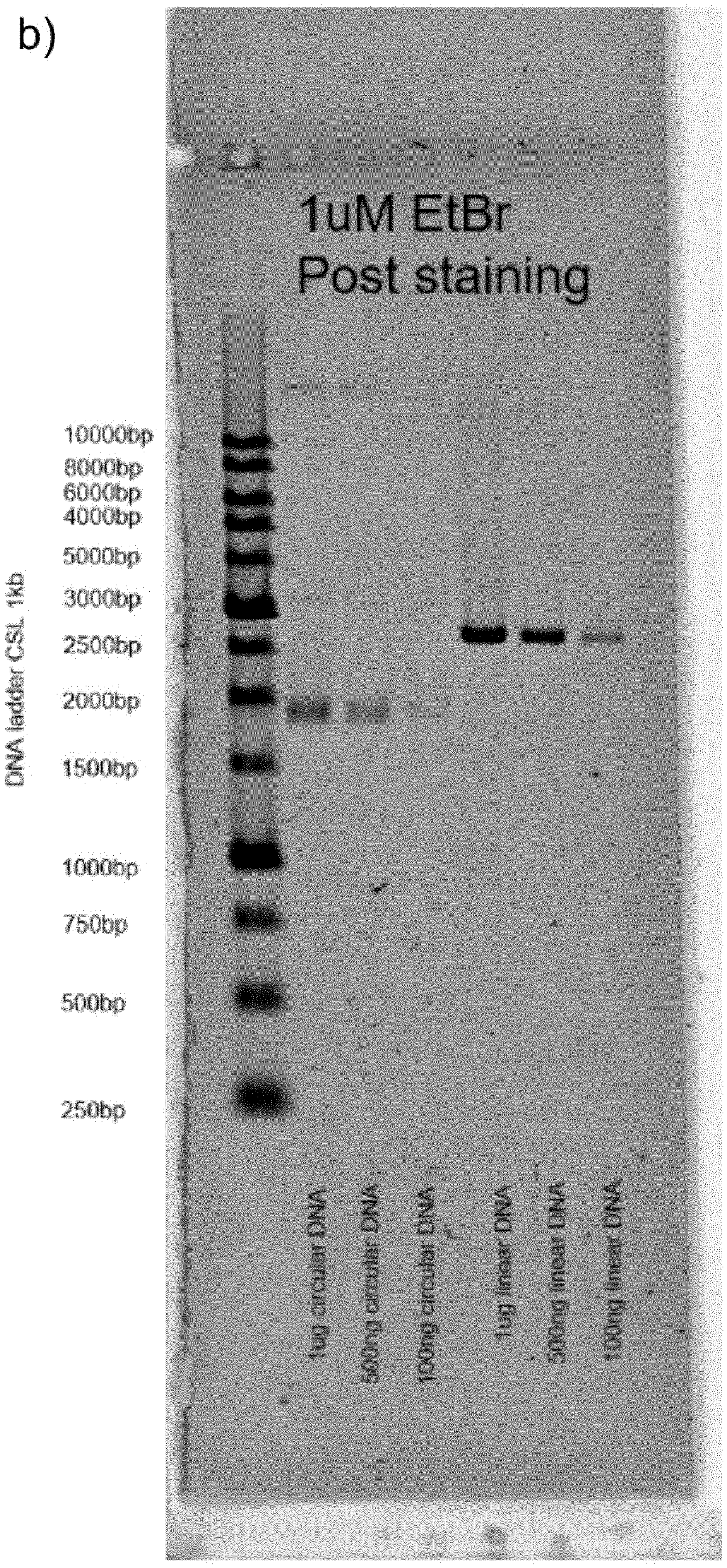
1uM EtBr
Post staining
DNA ladder CSL 1kb
10000bp
8000bp
6000bp
4000bp
5000bp
3000bp
2500bp
2000bp
1500bp
1000bp
750bp
500bp
250bp
1ug circular DNA
500ng circular DNA
100ng circular DNA
1ug linear DNA
500ng linear DNA
100ng linear DNA

Fig. 26

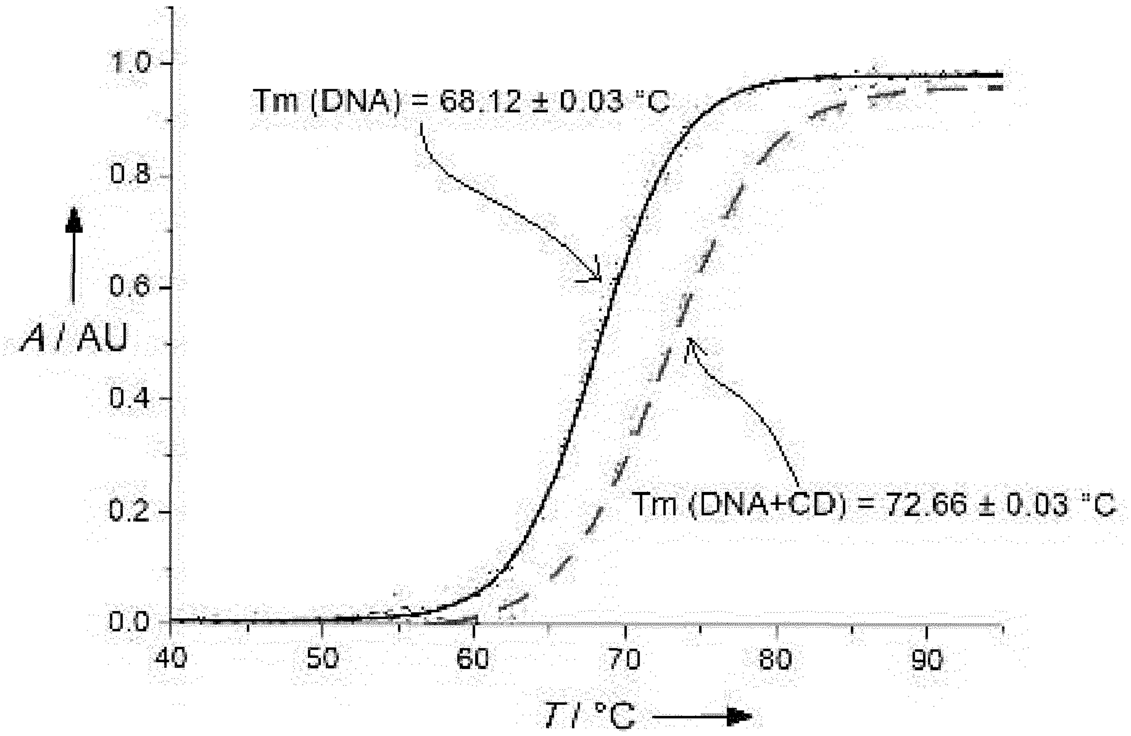


Fig. 27A

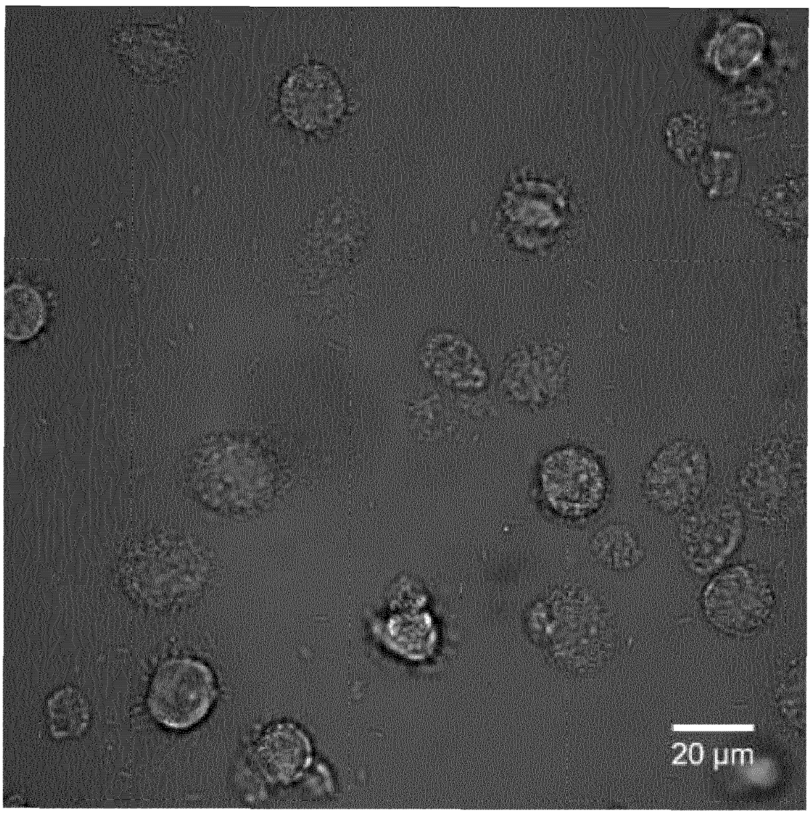

Fig. 27B

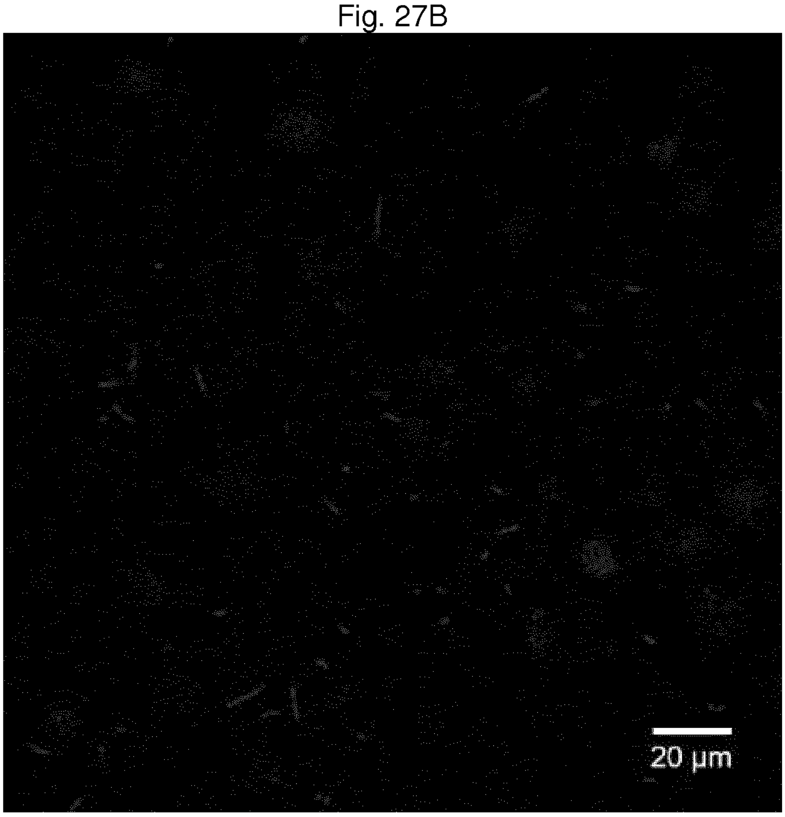


Fig. 27C

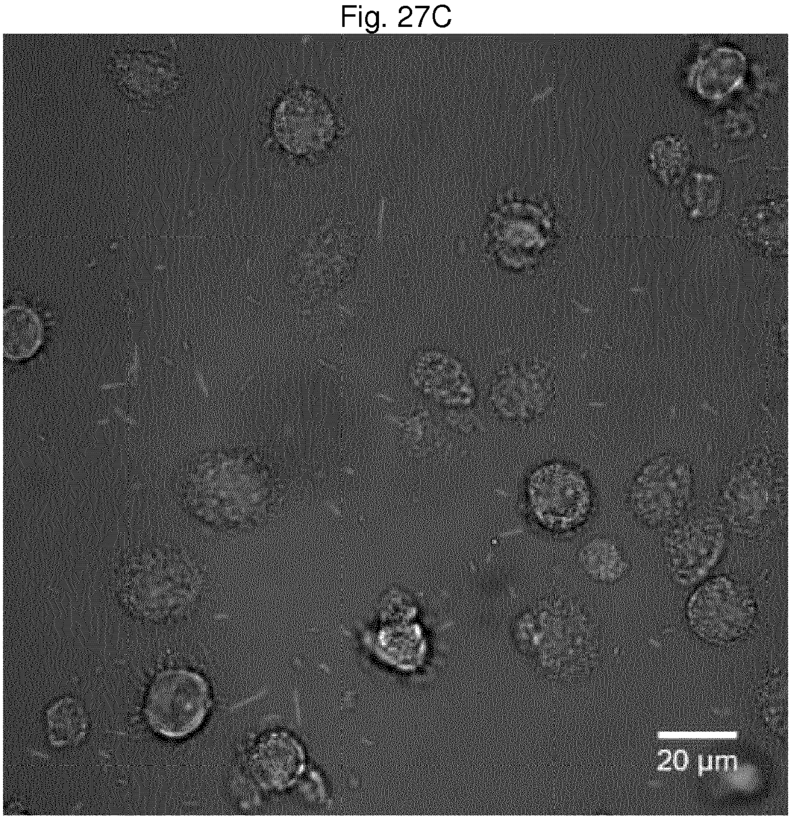

Fig. 28A

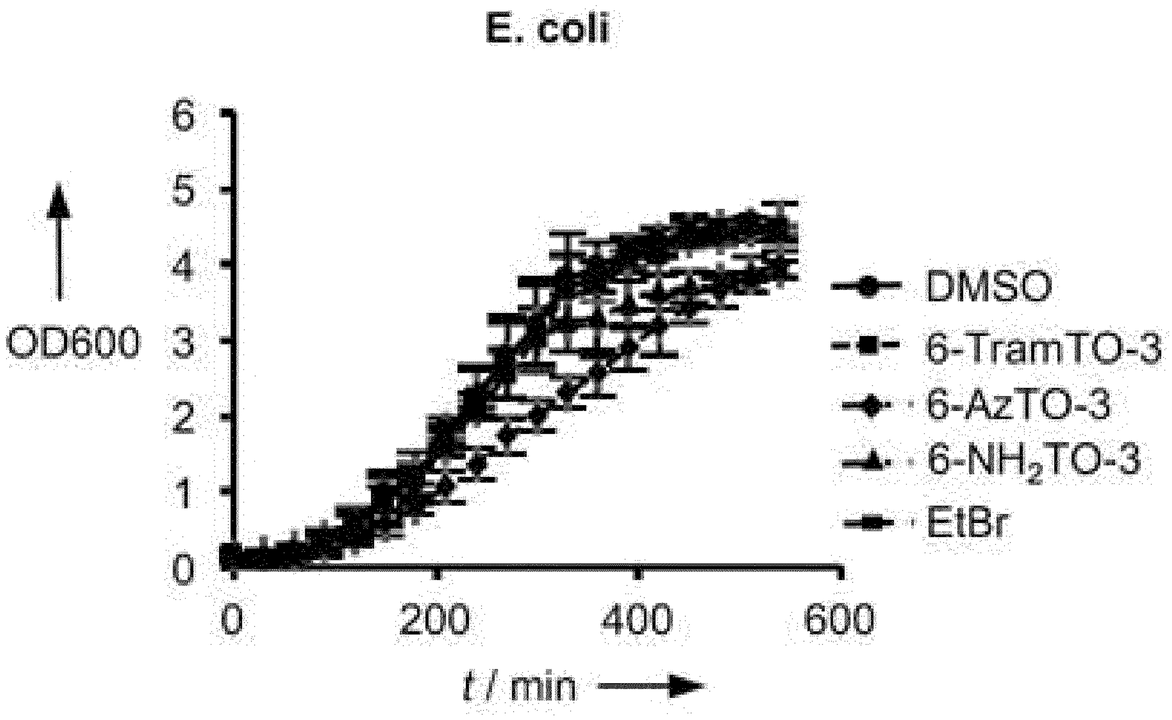


Fig. 28B

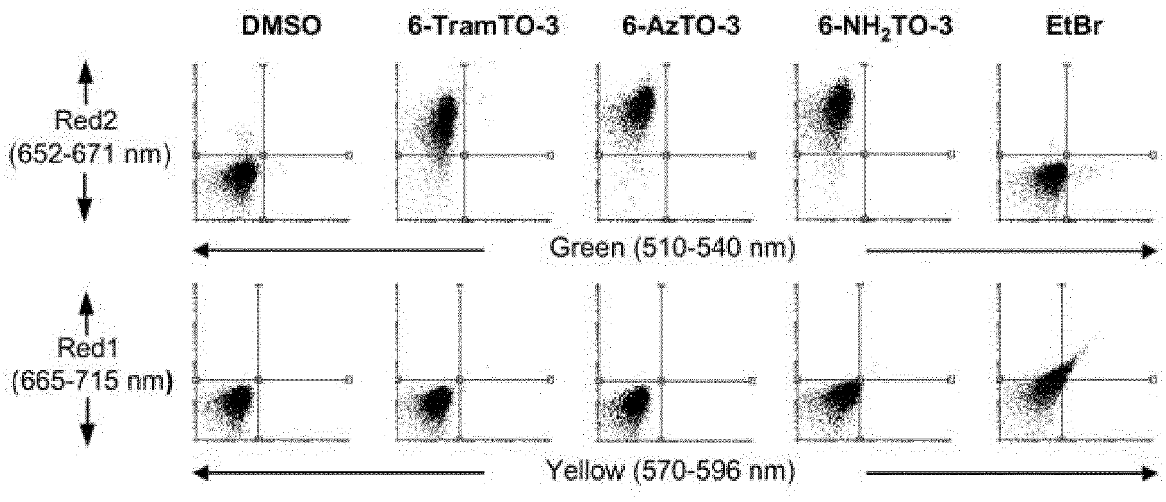

Fig. 29A

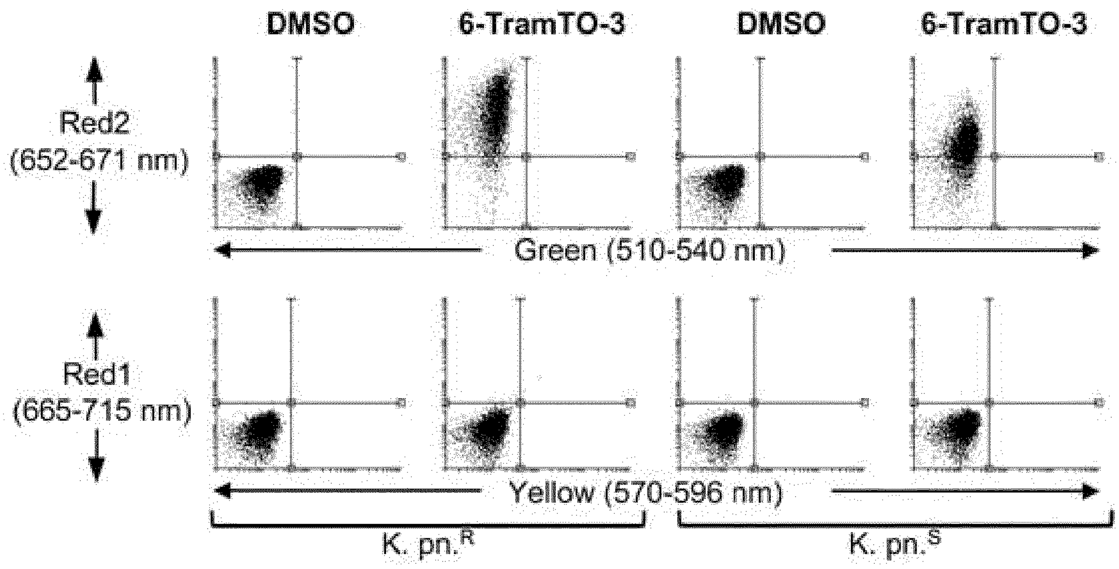


Fig. 29B

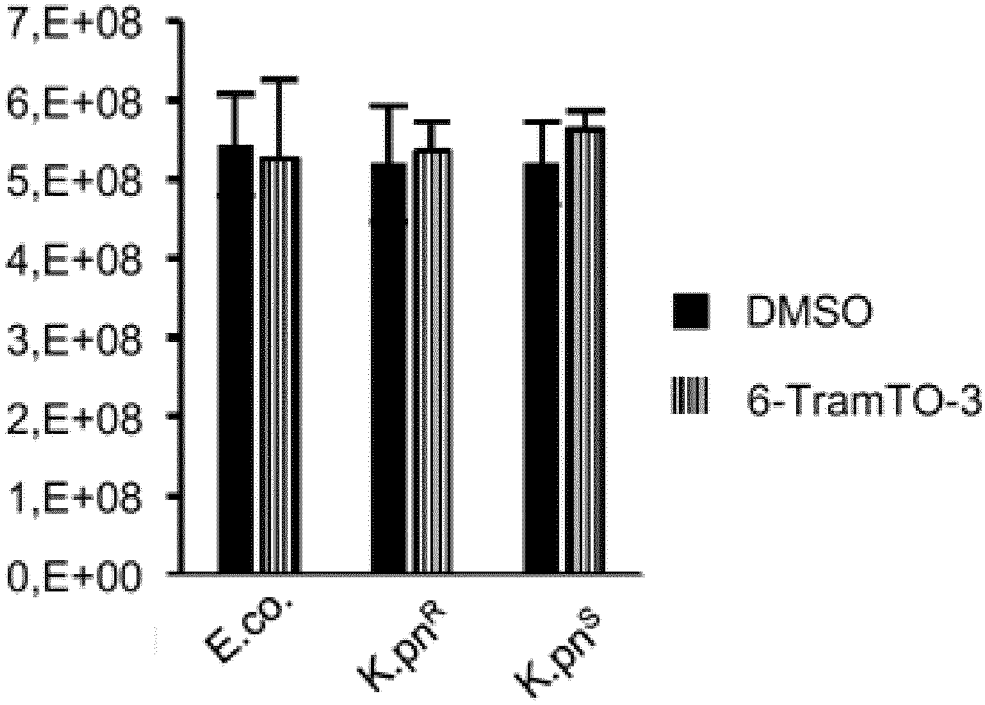

Fig. 29C

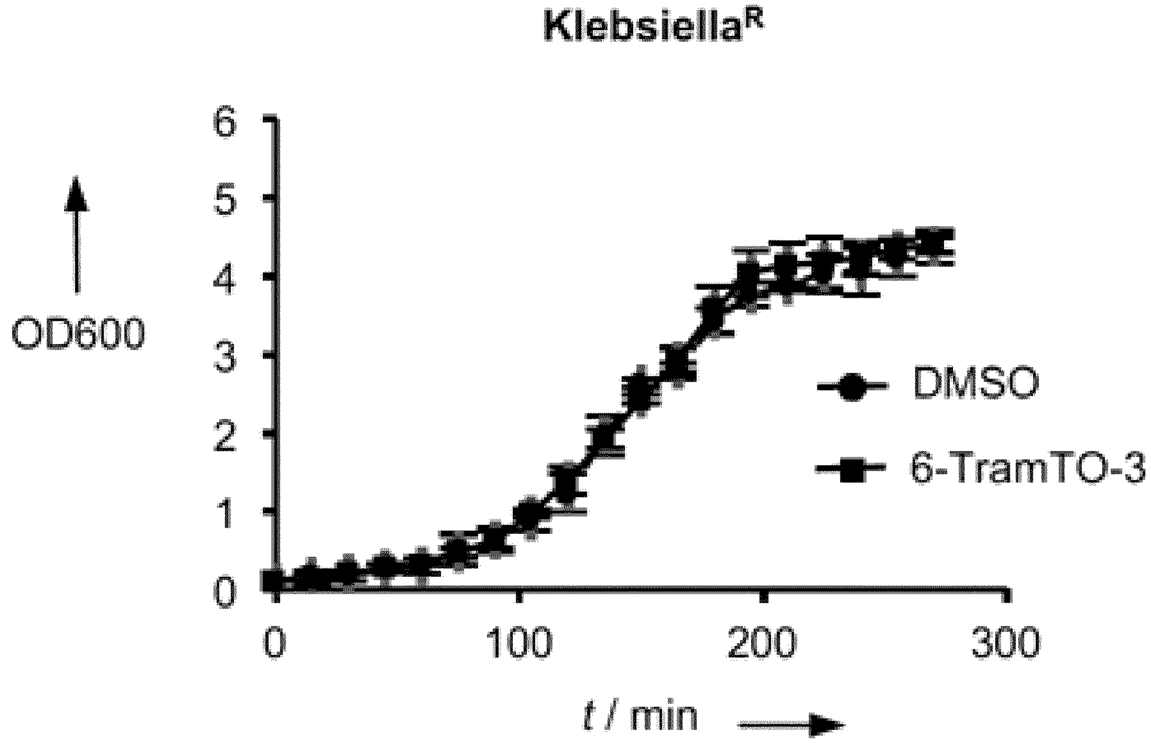
Klebsiella^R
OD600
6
5
4
3
2
1
0
100
200
300
t / min
DMSO
6-TramTO-3

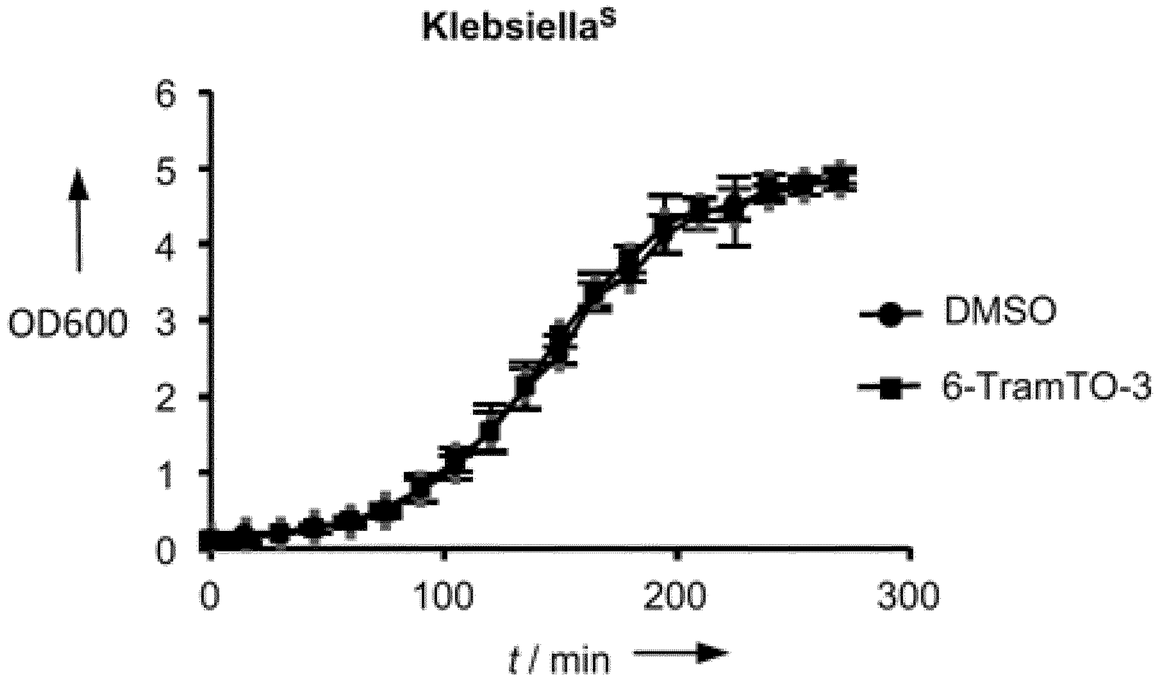
Klebsiella^S
OD600
6
5
4
3
2
1
0
100
200
300
t / min
DMSO
6-TramTO-3

Fig. 30

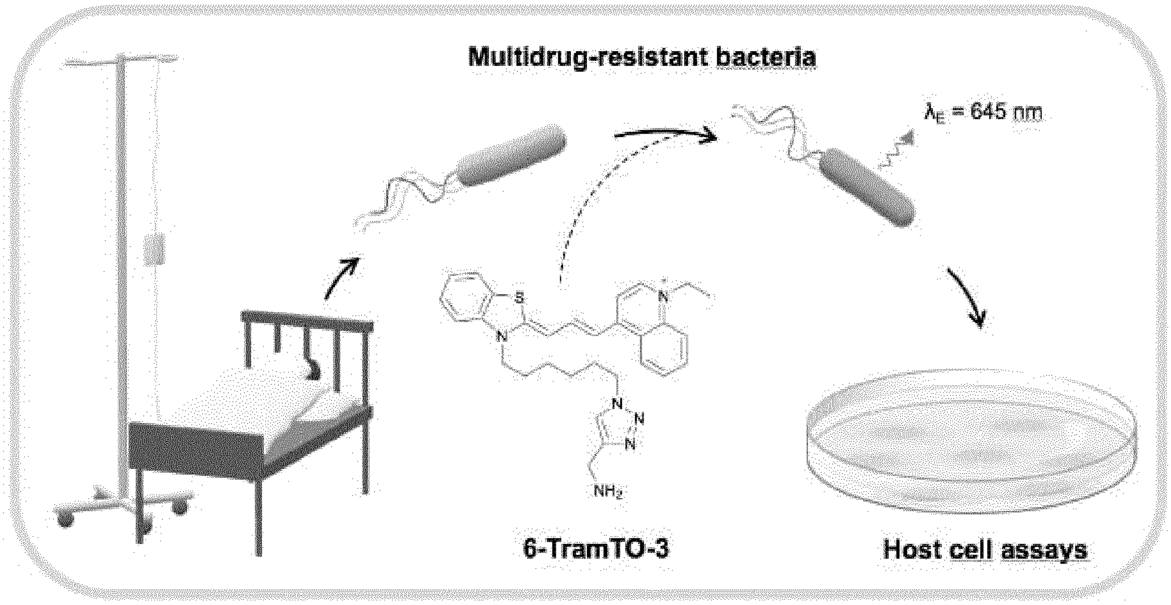
Multidrug-resistant bacteria
λE = 645 nm
6-TramTO-3
Host cell assays

Fig 31

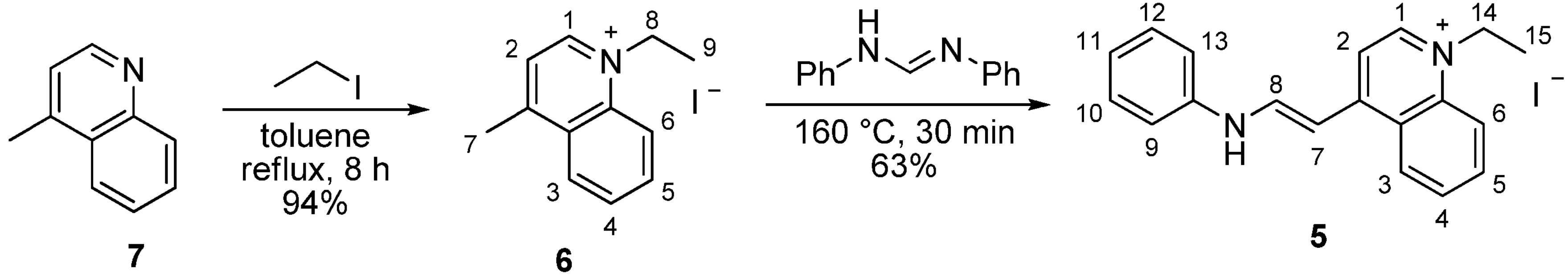


7 6 5

Fig. 32

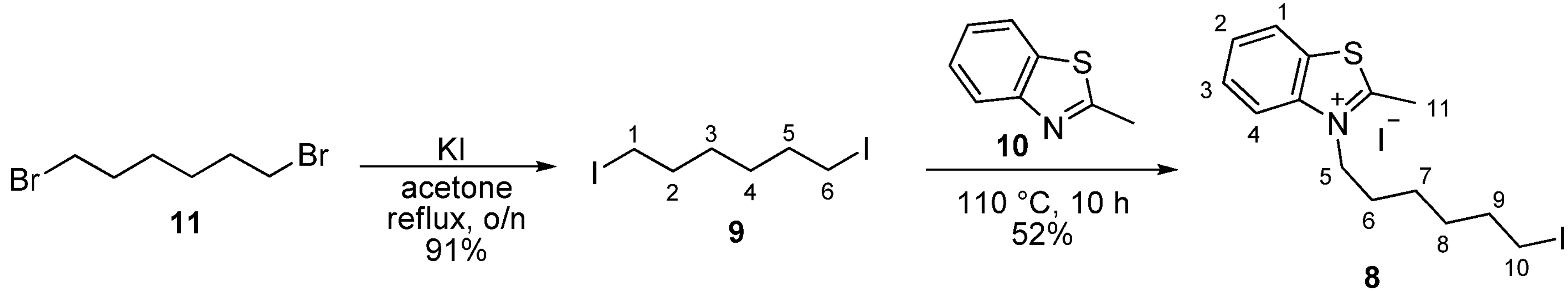


11 9 10 8

Fig. 33

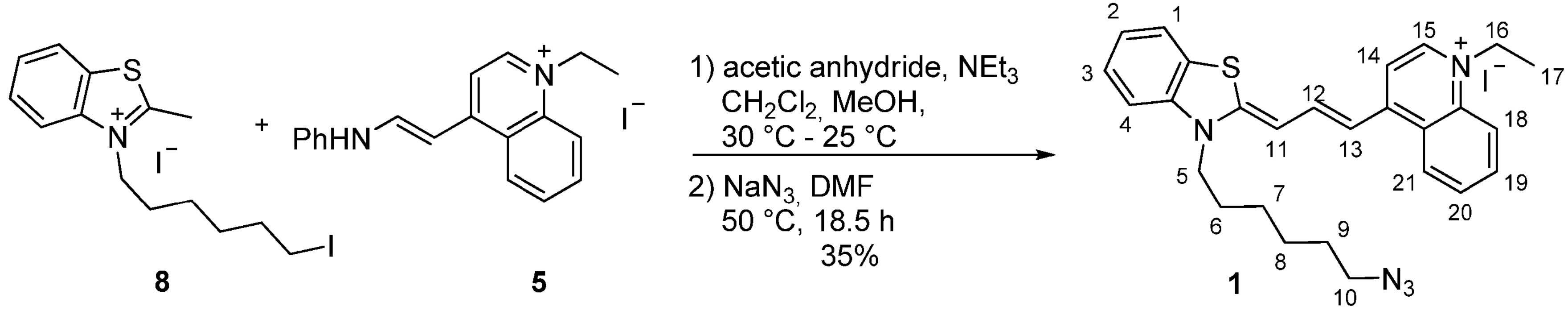


8 5 1

Fig. 34

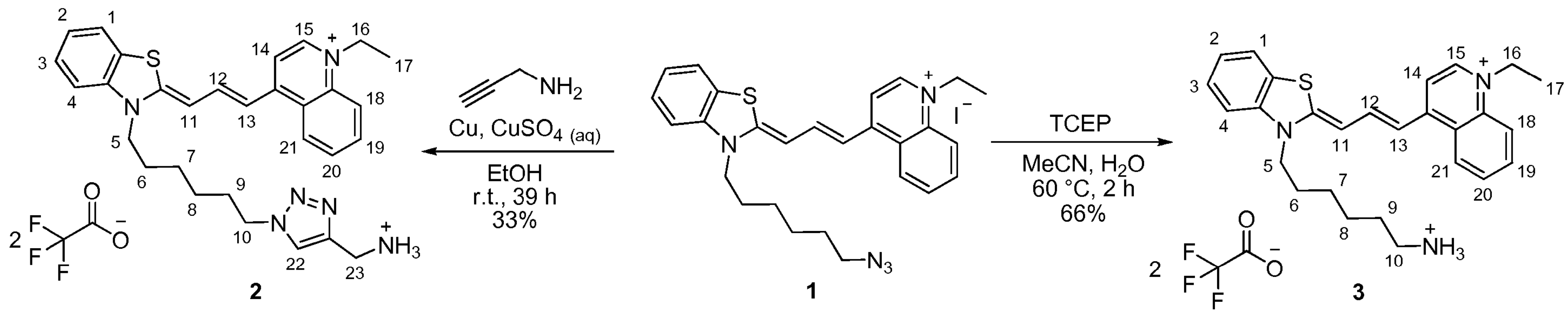


2 1 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2016340717 A1
- US 2011262904 A1
- WO 2013189043 A1
- US 2015185182 A1
- US 2010151509 A1


### Non-patent literature cited in the description


- **JIEQIONG QUI et al.** *Nucleic Acid Research,* 01 July 2016, vol. 44 (17), 1-12